# EUROPEAN PATENT SPECIFICATION

(11) **EP 1 633 387 B1**
(45) Date of publication and mention of the grant of the patent: **22.02.2012**
(21) Application number: 04755620.4
(22) Date of filing: 17.06.2004
(51) Int. Cl.: A61K 38/17, A61K 39/00, A61K 31/7088, A61K 48/00

(54) **COMBINATIONS OF TUMOR-ASSOCIATED ANTIGENS FOR THE TREATMENT OF VARIOUS TYPES OF CANCERS**
KOMBINATIONEN VON TUMOR-ASSOZIIERTEN ANTIGENEN ZUR BEHANDLUNG VON VERSCHIEDENEN KREBSTYPEN
COMBINAISONS D'ANTIGENES ASSOCIES A DES TUMEURS DANS DES COMPOSITIONS POUR DIVERS TYPES DE CANCERS

(30) Priority: 17.06.2003 US 479554 P
(43) Date of publication of application: 15.03.2006
(60) Divisional application: 10180553.9
(73) Proprietor: Mannkind Corporation, Valencia, California 91355 (US)
(72) Inventor: CHIANG, Chih-Sheng, Chatsworth, CA 91311 (US); BOT, Adrian, Valencia, CA 91355 (US); SIMARD, John J. L., Vancouver, British Colombia Z6G1A6 (CA); DIAMOND, David, C., West Hills, CA 91304 (US)
(74) Representative: Maiwald Patentanwalts GmbH
(86) International application number: PCT/US2004/019571
(87) International publication number: WO 2004/112825

(56) References cited:
- WO-A-00/06723
- WO-A-01/85932
- WO-A1-03/075952
- MOINGEON P: "Cancer vaccines" VACCINE, BUTTERWORTH SCIENTIFIC. GUILDFORD, GB, vol. 19, no. 11-12, 8 December 2001 (2001-12-08), pages 1305-1326, XP004313943 ISSN: 0264-410X
- MATSUSHITA MAIKO ET AL: "Preferentially expressed antigen of melanoma (PRAME) in the development of diagnostic and therapeutic methods for hematological malignancies." LEUKEMIA & LYMPHOMA. MAR 2003, vol. 44, no. 3, March 2003 (2003-03), pages 439-444, XP009040231 ISSN: 1042-8194
- CHANG S S ET AL: "Five different anti-prostate-specific membrane antigen (PSMA) antibodies confirm PSMA expression in tumor-associated neovasculature" CANCER RESEARCH, AMERICAN ASSOCIATION FOR CANCER RESEARCH, BALTIMORE, MD, US, vol. 59, no. 13, 1 July 1999 (1999-07-01), pages 3192-3198, XP002313495 ISSN: 0008-5472 cited in the application
- REYNOLDS S R ET AL: "Identification of HLA-A*03, A*11 and B*07-restricted melanoma-associated peptides that are immunogenic in vivo by vaccine-induced immune response (VIIR) analysis" JOURNAL OF IMMUNOLOGICAL METHODS, ELSEVIER SCIENCE PUBLISHERS B.V.,AMSTERDAM, NL, vol. 244, no. 1-2, 20 October 2000 (2000-10-20), pages 59-67, XP004218446 ISSN: 0022-1759
- KIRKIN A F ET AL: "THE IMMUNOGENIC PROPERTIES OF MELANOMA-ASSOCIATED ANTIGENS RECOGNIZED BY CYTOTOXIC T LYMPHOCYTES" EXPERIMENTAL AND CLINICAL IMMUNOGENETICS, S.KARGER, BASEL, CH, vol. 15, April 1998 (1998-04), pages 19-32, XP000925662 ISSN: 0254-9670
- MINCHEFF M ET AL: "Naked DNA and adenoviral immunizations for immunotherapy of prostate cancer: A phase I/II clinical trial" EUROPEAN UROLOGY, S. KARGER AG., BASEL, CH, vol. 38, no. 2, August 2000 (2000-08), pages 208-217, XP002957430 ISSN: 0302-2838

### SEQUENCE LISTING

<110> Chiang, Chih-Sheng
<120> COMBINATIONS OF TUMOR-ASSOCIATED
   ANTIGENS IN COMPOSITIONS FOR VARIOUS TYPES OF CANCERS
<130> MANNK.035A
<140> Not Assigned
   <141>
<150> 60/479,554
   <151> 2003-06-17
<160> 18
<170> FastSEQ for Windows Version 4.0
<210> 1
   <211> 529
   <212> PRT
   <213> Tyrosinase Precursor
<400> 1
<210> 2
   <211> 118
   <212> PRT
   <213> MART-1
<400> 2
   Met Pro Arg Glu Asp Ala His Phe Ile Tyr Gly Tyr Pro Lys Lys Gly
   1               5                   10                  15
   His Gly His Ser Tyr Thr Thr Ala Glu Glu Ala Ala Gly Ile Gly Ile
               20                  25                  30
   Leu Thr Val Ile Leu Gly Val Leu Leu Leu Ile Gly Cys Trp Tyr Cys
           35                  40                  45
   Arg Arg Arg Asn Gly Tyr Arg Ala Leu Met Asp Lys Ser Leu His Val
       50                  55                  60
   Gly Thr Gln Cys Ala Leu Thr Arg Arg Cys Pro Gln Glu Gly Phe Asp
   65                  70                  75                  80
   His Arg Asp Ser Lys Val Ser Leu Gln Glu Lys Asn Cys Glu Pro Val
                   85                  90                  95
   Val Pro Asn Ala Pro Pro Ala Tyr Glu Lys Leu Ser Ala Glu Gln Ser
               100                 105                 110
   Pro Pro Pro Tyr Ser Pro
           115
<210> 3
   <211> 223
   <212> PRT
   <213> Synovial Sarcoma
<400> 3
   Met Asn Gly Asp Asp Ala Phe Ala Arg Arg Pro Thr Val Gly Ala Gln
   1               5                   10                  15
   Ile Pro Glu Lys Ile Gln Lys Ala Phe Asp Asp Ile Ala Lys Tyr Phe
               20                  25                  30
   Ser Lys Glu Glu Trp Glu Lys Met Lys Ala Ser Glu Lys Ile Phe Tyr
           35                  40                  45
   Val Tyr Met Lys Arg Lys Tyr Glu Ala Met Thr Lys Leu Gly Phe Lys
       50                  55                  60
   Ala Thr Leu Pro Pro Phe Met Cys Asn Lys Arg Ala Glu Asp Phe Gln
   65                  70                  75                  80
   Gly Asn Asp Leu Asp Asn Asp Pro Asn Arg Gly Asn Gln Val Glu Arg
                   85                  90                  95
   Pro Gln Met Thr Phe Gly Arg Leu Gln Gly Ile Ser Pro Lys Ile Met
               100                 105                 110
   Pro Lys Lys Pro Ala Glu Glu Gly Asn Asp Ser Glu Glu Val Pro Glu
           115                 120                 125
   Ala Ser Gly Pro Gln Asn Asp Gly Lys Glu Leu Cys Pro Pro Gly Lys
       130                 135                 140
   Pro Thr Thr Ser Glu Lys Ile His Glu Arg Ser Gly Asn Arg Glu Ala
   145                 150                 155                 160
   Gln Glu Lys Glu Glu Arg Arg Gly Thr Ala His Arg Trp Ser Ser Gln
                   165                 170                 175
   Asn Thr His Asn Ile Gly Arg Phe Ser Leu Ser Thr Ser Met Gly Ala
               180                 185                 190
   Val His Gly Thr Pro Lys Thr Ile Thr His Asn Arg Asp Pro Lys Gly
           195                 200                 205
   Gly Asn Met Pro Gly Pro Thr Asp Cys Val Arg Glu Asn Ser Trp
       210                 215                 220
<210> 4
   <211> 750
   <212> PRT
   <213> Folate Hydrolase
<400> 4
   Met Trp Asn Leu Leu His Glu Thr Asp Ser Ala Val Ala Thr Ala Arg
   1               5                   10                  15
   Arg Pro Arg Trp Leu Cys Ala Gly Ala Leu Val Leu Ala Gly Gly Phe
               20                  25                  30
   Phe Leu Leu Gly Phe Leu Phe Gly Trp Phe Ile Lys Ser Ser Asn Glu
           35                  40                  45
   Ala Thr Asn Ile Thr Pro Lys His Asn Met Lys Ala Phe Leu Asp Glu
       50                  55                  60
   Leu Lys Ala Glu Asn Ile Lys Lys Phe Leu Tyr Asn Phe Thr Gln Ile
   65                  70                  75                  80
   Pro His Leu Ala Gly Thr Glu Gln Asn Phe Gln Leu Ala Lys Gln Ile
                   85                  90                  95
   Gln Ser Gln Trp Lys Glu Phe Gly Leu Asp Ser Val Glu Leu Ala His
               100                 105                 110
   Tyr Asp Val Leu Leu Ser Tyr Pro Asn Lys Thr His Pro Asn Tyr Ile
           115                 120                 125
   Ser Ile Ile Asn Glu Asp Gly Asn Glu Ile Phe Asn Thr Ser Leu Phe
       130                 135                 140
   Glu Pro Pro Pro Pro Gly Tyr Glu Asn Val Ser Asp Ile Val Pro Pro
   145                 150                 155                 160
   Phe Ser Ala Phe Ser Pro Gln Gly Met Pro Glu Gly Asp Leu Val Tyr
                   165                 170                 175
   Val Asn Tyr Ala Arg Thr Glu Asp Phe Phe Lys Leu Glu Arg Asp Met
               180                 185                 190
   Lys Ile Asn Cys Ser Gly Lys Ile Val Ile Ala Arg Tyr Gly Lys Val
           195                 200                 205
   Phe Arg Gly Asn Lys Val Lys Asn Ala Gln Leu Ala Gly Ala Lys Gly
       210                 215                 220
   Val Ile Leu Tyr Ser Asp Pro Ala Asp Tyr Phe Ala Pro Gly Val Lys
   225                 230                 235                 240
   Ser Tyr Pro Asp Gly Trp Asn Leu Pro Gly Gly Gly Val Gln Arg Gly
                   245                 250                 255
   Asn Ile Leu Asn Leu Asn Gly Ala Gly Asp Pro Leu Thr Pro Gly Tyr
               260                 265                 270
   Pro Ala Asn Glu Tyr Ala Tyr Arg Arg Gly Ile Ala Glu Ala Val Gly
           275                 280                 285
   Leu Pro Ser Ile Pro Val His Pro Ile Gly Tyr Tyr Asp Ala Gln Lys
       290                 295                 300
   Leu Leu Glu Lys Met Gly Gly Ser Ala Pro Pro Asp Ser Ser Trp Arg
   305                 310                 315                 320
   Gly Ser Leu Lys Val Pro Tyr Asn Val Gly Pro Gly Phe Thr Gly Asn
                   325                 330                 335
   Phe Ser Thr Gln Lys Val Lys Met His Ile His Ser Thr Asn Glu Val
               340                 345                 350
   Thr Arg Ile Tyr Asn Val Ile Gly Thr Leu Arg Gly Ala Val Glu Pro
           355                 360                 365
   Asp Arg Tyr Val Ile Leu Gly Gly His Arg Asp Ser Trp Val Phe Gly
       370                 375                 380
   Gly Ile Asp Pro Gln Ser Gly Ala Ala Val Val His Glu Ile Val Arg
   385                 390                 395                 400
   Ser Phe Gly Thr Leu Lys Lys Glu Gly Trp Arg Pro Arg Arg Thr Ile
                   405                 410                 415
   Leu Phe Ala Ser Trp Asp Ala Glu Glu Phe Gly Leu Leu Gly Ser Thr
               420                 425                 430
   Glu Trp Ala Glu Glu Asn Ser Arg Leu Leu Gln Glu Arg Gly Val Ala
           435                 440                 445
   Tyr Ile Asn Ala Asp Ser Ser Ile Glu Gly Asn Tyr Thr Leu Arg Val
       450                 455                 460
   Asp Cys Thr Pro Leu Met Tyr Ser Leu Val His Asn Leu Thr Lys Glu
   465                 470                 475                 480
   Leu Lys Ser Pro Asp Glu Gly Phe Glu Gly Lys Ser Leu Tyr Glu Ser
                   485                 490                 495
   Trp Thr Lys Lys Ser Pro Ser Pro Glu Phe Ser Gly Met Pro Arg Ile
               500                 505                 510
   Ser Lys Leu Gly Ser Gly Asn Asp Phe Glu Val Phe Phe Gln Arg Leu
           515                 520                 525
   Gly Ile Ala Ser Gly Arg Ala Arg Tyr Thr Lys Asn Trp Glu Thr Asn
       530                 535                 540
   Lys Phe Ser Gly Tyr Pro Leu Tyr His Ser Val Tyr Glu Thr Tyr Glu
   545                 550                 555                 560
   Leu Val Glu Lys Phe Tyr Asp Pro Met Phe Lys Tyr His Leu Thr Val
                   565                 570                 575
   Ala Gln Val Arg Gly Gly Met Val Phe Glu Leu Ala Asn Ser Ile Val
               580                 585                 590
   Leu Pro Phe Asp Cys Arg Asp Tyr Ala Val Val Leu Arg Lys Tyr Ala
           595                 600                 605
   Asp Lys Ile Tyr Ser Ile Ser Met Lys His Pro Gln Glu Met Lys Thr
       610                 615                 620
   Tyr Ser Val Ser Phe Asp Ser Leu Phe Ser Ala Val Lys Asn Phe Thr
   625                 630                 635                 640
   Glu Ile Ala Ser Lys Phe Ser Glu Arg Leu Gln Asp Phe Asp Lys Ser
                   645                 650                 655
   Asn Pro Ile Val Leu Arg Met Met Asn Asp Gln Leu Met Phe Leu Glu
               660                 665                 670
   Arg Ala Phe Ile Asp Pro Leu Gly Leu Pro Asp Arg Pro Phe Tyr Arg
           675                 680                 685
   His Val Ile Tyr Ala Pro Ser Ser His Asn Lys Tyr Ala Gly Glu Ser
       690                 695                 700
   Phe Pro Gly Ile Tyr Asp Ala Leu Phe Asp Ile Glu Ser Lys Val Asp
   705                 710                 715                 720
   Pro Ser Lys Ala Trp Gly Glu Val Lys Arg Gln Ile Tyr Val Ala Ala
                   725                 730                 735
   Phe Thr Val Gln Ala Ala Ala Glu Thr Leu Ser Glu Val Ala
               740                 745                 750
<210> 5
   <211> 309
   <212> PRT
   <213> MAGE-1
<400> 5
   Met Ser Leu Glu Gln Arg Ser Leu His Cys Lys Pro Glu Glu Ala Leu
   1               5                   10                  15
   Glu Ala Gln Gln Glu Ala Leu Gly Leu Val Cys Val Gln Ala Ala Thr
               20                  25                  30
   Ser Ser Ser Ser Pro Leu Val Leu Gly Thr Leu Glu Glu Val Pro Thr
           35                  40                  45
   Ala Gly Ser Thr Asp Pro Pro Gln Ser Pro Gln Gly Ala Ser Ala Phe
       50                  55                  60
   Pro Thr Thr Ile Asn Phe Thr Arg Gln Arg Gln Pro Ser Glu Gly Ser
   65                  70                  75                  80
   Ser Ser Arg Glu Glu Glu Gly Pro Ser Thr Ser Cys Ile Leu Glu Ser
                   85                  90                  95
   Leu Phe Arg Ala Val Ile Thr Lys Lys Val Ala Asp Leu Val Gly Phe
               100                 105                 110
   Leu Leu Leu Lys Tyr Arg Ala Arg Glu Pro Val Thr Lys Ala Glu Met
           115                 120                 125
   Leu Glu Ser Val Ile Lys Asn Tyr Lys His Cys Phe Pro Glu Ile Phe
       130                 135                 140
   Gly Lys Ala Ser Glu Ser Leu Gln Leu Val Phe Gly Ile Asp Val Lys
   145                 150                 155                 160
   Glu Ala Asp Pro Thr Gly His Ser Tyr Val Leu Val Thr Cys Leu Gly
                   165                 170                 175
   Leu Ser Tyr Asp Gly Leu Leu Gly Asp Asn Gln Ile Met Pro Lys Thr
               180                 185                 190
   Gly Phe Leu Ile Ile Val Leu Val Met Ile Ala Met Glu Gly Gly His
           195                 200                 205
   Ala Pro Glu Glu Glu Ile Trp Glu Glu Leu Ser Val Met Glu Val Tyr
       210                 215                 220
   Asp Gly Arg Glu His Ser Ala Tyr Gly Glu Pro Arg Lys Leu Leu Thr
   225                 230                 235                 240
   Gln Asp Leu Val Gln Glu Lys Tyr Leu Glu Tyr Arg Gln Val Pro Asp
                   245                 250                 255
   Ser Asp Pro Ala Arg Tyr Glu Phe Leu Trp Gly Pro Arg Ala Leu Ala
               260                 265                 270
   Glu Thr Ser Tyr Val Lys Val Leu Glu Tyr Val Ile Lys Val Ser Ala
           275                 280                 285
   Arg Val Arg Phe Phe Phe Pro Ser Leu Arg Glu Ala Ala Leu Arg Glu
       290                 295                 300
   Glu Glu Glu Gly Val
   305
<210> 6
   <211> 314
   <212> PRT
   <213> MAGE-3
<400> 6
   Met Pro Leu Glu Gln Arg Ser Gln His Cys Lys Pro Glu Glu Gly Leu
    1               5                  10                  15
   Glu Ala Arg Gly Glu Ala Leu Gly Leu Val Gly Ala Gln Ala Pro Ala
               20                  25                  30
   Thr Glu Glu Gln Glu Ala Ala Ser Ser Ser Ser Thr Leu Val Glu Val
           35                  40                  45
   Thr Leu Gly Glu Val Pro Ala Ala Glu Ser Pro Asp Pro Pro Gln Ser
       50                  55                  60
   Pro Gln Gly Ala Ser Ser Leu Pro Thr Thr Met Asn Tyr Pro Leu Trp
   65                  70                  75                  80
   Ser Gln Ser Tyr Glu Asp Ser Ser Asn Gln Glu Glu Glu Gly Pro Ser
                   85                  90                  95
   Thr Phe Pro Asp Leu Glu Ser Glu Phe Gln Ala Ala Leu Ser Arg Lys
               100                 105                 110
   Val Ala Glu Leu Val His Phe Leu Leu Leu Lys Tyr Arg Ala Arg Glu
           115                 120                 125
   Pro Val Thr Lys Ala Glu Met Leu Gly Ser Val Val Gly Asn Trp Gln
   130 135 140
   Tyr Phe Phe Pro Val Ile Phe Ser Lys Ala Ser Ser Ser Leu Gln Leu
   145 150 155 160
   Val Phe Gly Ile Glu Leu Met Glu Val Asp Pro Ile Gly His Leu Tyr
   165 170 175
   Ile Phe Ala Thr Cys Leu Gly Leu Ser Tyr Asp Gly Leu Leu Gly Asp
   180 185 190
   Asn Gln Ile Met Pro Lys Ala Gly Leu Leu Ile Ile Val Leu Ala Ile
   195 200 205
   Ile Ala Arg Glu Gly Asp Cys Ala Pro Glu Glu Lys Ile Trp Glu Glu
   210 215 220
   Leu Ser Val Leu Glu Val Phe Glu Gly Arg Glu Asp Ser Ile Leu Gly
   225 230 235 240
   Asp Pro Lys Lys Leu Leu Thr Gln His Phe Val Gln Glu Asn Tyr Leu
   245 250 255
   Glu Tyr Arg Gln Val Pro Gly Ser Asp Pro Ala Cys Tyr Glu Phe Leu
   260 265 270
   Trp Gly Pro Arg Ala Leu Val Glu Thr Ser Tyr Val Lys Val Leu His
   275 280 285
   His Met Val Lys Ile Ser Gly Gly Pro His Ile Ser Tyr Pro Pro Leu
   290 295 300
   His Glu Trp Val Leu Arg Glu Gly Glu Glu
   305 310
<210> 7
   <211> 180
   <212> PRT
   <213> Autoimmunogenic Cancer
<400> 7
   Met Gln Ala Glu Gly Arg Gly Thr Gly Gly Ser Thr Gly Asp Ala Asp
   1 5 10 15
   Gly Pro Gly Gly Pro Gly Ile Pro Asp Gly Pro Gly Gly Asn Ala Gly
   20 25 30
   Gly Pro Gly Glu Ala Gly Ala Thr Gly Gly Arg Gly Pro Arg Gly Ala
   35 40 45
   Gly Ala Ala Arg Ala Ser Gly Pro Gly Gly Gly Ala Pro Arg Gly Pro
   50 55 60
   His Gly Gly Ala Ala Ser Gly Leu Asn Gly Cys Cys Arg Cys Gly Ala
   65 70 75 80
   Arg Gly Pro Glu Ser Arg Leu Leu Glu Phe Tyr Leu Ala Met Pro Phe
   85 90 95
   Ala Thr Pro Met Glu Ala Glu Leu Ala Arg Arg Ser Leu Ala Gln Asp
   100 105 110
   Ala Pro Pro Leu Pro Val Pro Gly Val Leu Leu Lys Glu Phe Thr Val
   115 120 125
   Ser Gly Asn Ile Leu Thr Ile Arg Leu Thr Ala Ala Asp His Arg Gln
   130 135 140
   Leu Gln Leu Ser Ile Ser Ser Cys Leu Gln Gln Leu Ser Leu Leu Met
   145 150 155 160
   Trp Ile Thr Gln Cys Phe Leu Pro Val Phe Leu Ala Gln Pro Pro Ser
   165 170 175
   Gly Gln Arg Arg
   180
<210> 8
   <211> 509
   <212> PRT
   <213> Preferentially expressed antigen in melanoma
<400> 8
   Met Glu Arg Arg Arg Leu Trp Gly Ser Ile Gln Ser Arg Tyr Ile Ser
   1               5                   10                  15
   Met Ser Val Trp Thr Ser Pro Arg Arg Leu Val Glu Leu Ala Gly Gln
               20                  25                  30
   Ser Leu Leu Lys Asp Glu Ala Leu Ala Ile Ala Ala Leu Glu Leu Leu
           35                  40                  45
   Pro Arg Glu Leu Phe Pro Pro Leu Phe Met Ala Ala Phe Asp Gly Arg
       50                  55                  60
   His Ser Gln Thr Leu Lys Ala Met Val Gln Ala Trp Pro Phe Thr Cys
   65                  70                  75                  80
   Leu Pro Leu Gly Val Leu Met Lys Gly Gln His Leu His Leu Glu Thr
                   85                  90                  95
   Phe Lys Ala Val Leu Asp Gly Leu Asp Val Leu Leu Ala Gln Glu Val
               100                 105                 110
   Arg Pro Arg Arg Trp Lys Leu Gln Val Leu Asp Leu Arg Lys Asn Ser
           115                 120                 125
   His Gln Asp Phe Trp Thr Val Trp Ser Gly Asn Arg Ala Ser Leu Tyr
       130                 135                 140
   Ser Phe Pro Glu Pro Glu Ala Ala Gln Pro Met Thr Lys Lys Arg Lys
   145                 150                 155                 160
   Val Asp Gly Leu Ser Thr Glu Ala Glu Gln Pro Phe Ile Pro Val Glu
                   165                 170                 175
   Val Leu Val Asp Leu Phe Leu Lys Glu Gly Ala Cys Asp Glu Leu Phe
               180                 185                 190
   Ser Tyr Leu Ile Glu Lys Val Lys Arg Lys Lys Asn Val Leu Arg Leu
           195                 200                 205
   Cys Cys Lys Lys Leu Lys Ile Phe Ala Met Pro Met Gln Asp Ile Lys
       210                 215                 220
   Met Ile Leu Lys Met Val Gln Leu Asp Ser Ile Glu Asp Leu Glu Val
   225                 230                 235                 240
   Thr Cys Thr Trp Lys Leu Pro Thr Leu Ala Lys Phe Ser Pro Tyr Leu
                   245                 250                 255
   Gly Gln Met Ile Asn Leu Arg Arg Leu Leu Leu Ser His Ile His Ala
               260                 265                 270
   Ser Ser Tyr Ile Ser Pro Glu Lys Glu Glu Gln Tyr Ile Ala Gln Phe
           275                 280                 285
   Thr Ser Gln Phe Leu Ser Leu Gln Cys Leu Gln Ala Leu Tyr Val Asp
       290                 295                 300
   Ser Leu Phe Phe Leu Arg Gly Arg Leu Asp Gln Leu Leu Arg His Val
   305                 310                 315                 320
   Met Asn Pro Leu Glu Thr Leu Ser Ile Thr Asn Cys Arg Leu Ser Glu
                   325                 330                 335
   Gly Asp Val Met His Leu Ser Gln Ser Pro Ser Val Ser Gln Leu Ser
               340                 345                 350
   Val Leu Ser Leu Ser Gly Val Met Leu Thr Asp Val Ser Pro Glu Pro
           355                 360                 365
   Leu Gln Ala Leu Leu Glu Arg Ala Ser Ala Thr Leu Gln Asp Leu Val
           370                 375                 380
   Phe Asp Glu Cys Gly Ile Thr Asp Asp Gln Leu Leu Ala Leu Leu Pro
   385                 390                 395                 400
   Ser Leu Ser His Cys Ser Gln Leu Thr Thr Leu Ser Phe Tyr Gly Asn
                   405                 410                 415
   Ser Ile Ser Ile Ser Ala Leu Gln Ser Leu Leu Gln His Leu Ile Gly
               420                 425                 430
   Leu Ser Asn Leu Thr His Val Leu Tyr Pro Val Pro Leu Glu Ser Tyr
           435                 440                 445
   Glu Asp Ile His Gly Thr Leu His Leu Glu Arg Leu Ala Tyr Leu His
       450                 455                 460
   Ala Arg Leu Arg Glu Leu Leu Cys Glu Leu Gly Arg Pro Ser Met Val
   465                 470                 475                 480
   Trp Leu Ser Ala Asn Pro Cys Pro His Cys Gly Asp Arg Thr Phe Tyr
                   485                 490                 495
   Asp Pro Glu Pro Ile Leu Cys Pro Cys Phe Met Pro Asn
               500                 505
<210> 9
   <211> 1255
   <212> PRT
   <213> Receptor protein-tyrosine kinase
<400> 9
<210> 10
   <211> 2384
   <212> DNA
   <213> Homo sapiens
<400> 10
   tattgagttc ttcaaacatt gtagcctctt tatggtctct gagaaataac taccttaaac 60
   ccataatctt taatacttcc taaactttct taataagaga agctctattc ctgacactac 120
   ctctcatttg caaggtcaaa tcatcattag ttttgtagtc tattaactgg gtttgcttag 180
   gtcaggcatt attattacta accttattgt taatattcta accataagaa ttaaactatt 240
   aatggtgaat agagtttttc actttaacat aggcctatcc cactggtggg atacgagcca 300
   attcgaaaga aaagtcagtc atgtgctttt cagaggatga aagcttaaga taaagactaa 360
   aagtgtttga tgctggaggt gggagtggta ttatataggt ctcagccaag acatgtgata 420
   atcactgtag tagtagctgg aaagagaaat ctgtgactcc aattagccag ttcctgcaga 480
   ccttgtgagg actagaggaa gaatgctcct ggctgttttg tactgcctgc tgtggagttt 540
   ccagacctcc gctggccatt tccctagagc ctgtgtctcc tctaagaacc tgatggagaa 600
   ggaatgctgt ccaccgtgga gcggggacag gagtccctgt ggccagcttt caggcagagg 660
   ttcctgtcag aatatccttc tgtccaatgc accacttggg cctcaatttc ccttcacagg 720
   ggtggatgac cgggagtcgt ggccttccgt cttttataat aggacctgcc agtgctctgg 780
   caacttcatg ggattcaact gtggaaactg caagtttggc ttttggggac caaactgcac 840
   agagagacga ctcttggtga gaagaaacat cttcgatttg agtgccccag agaaggacaa 900
   attttttgcc tacctcactt tagcaaagca taccatcagc tcagactatg tcatccccat 960
   agggacctat ggccaaatga aaaatggatc aacacccatg tttaacgaca tcaatattta 1020
   tgacctcttt gtctggatgc attattatgt gtcaatggat gcactgcttg ggggatctga 1080
   aatctggaga gacattgatt ttgcccatga agcaccagct tttctgcctt ggcatagact 1140
   cttcttgttg cggtgggaac aagaaatcca gaagctgaca ggagatgaaa acttcactat 1200
   tccatattgg gactggcggg atgcagaaaa gtgtgacatt tgcacagatg agtacatggg 1260
   aggtcagcac cccacaaatc ctaacttact cagcccagca tcattcttct cctcttggca 1320
   gattgtctgt agccgattgg aggagtacaa cagccatcag tctttatgca atggaacgcc 1380
   cgagggacct ttacggcgta atcctggaaa ccatgacaaa tccagaaccc caaggctccc 1440
   ctcttcagct gatgtagaat tttgcctgag tttgacccaa tatgaatctg gttccatgga 1500
   taaagctgcc aatttcagct ttagaaatac actggaagga tttgctagtc cacttactgg 1560
   gatagcggat gcctctcaaa gcagcatgca caatgccttg cacatctata tgaatggaac 1620
   aatgtcccag gtacagggat ctgccaacga tcctatcttc cttcttcacc atgcatttgt 1680
   tgacagtatt tttgagcagt ggctccgaag gcaccgtcct cttcaagaag tttatccaga 1740
   agccaatgca cccattggac ataaccggga atcctacatg gttcctttta taccactgta 1800
   cagaaatggt gatttcttta tttcatccaa agatctgggc tatgactata gctatctaca 1860
   agattcagac ccagactctt ttcaagacta cattaagtcc tatttggaac aagcgagtcg 1920
   gatctggtca tggctccttg gggcggcgat ggtaggggcc gtcctcactg ccctgctggc 1980
   agggcttgtg agcttgctgt gtcgtcacaa gagaaagcag cttcctgaag aaaagcagcc 2040
   actcctcatg gagaaagagg attaccacag cttgtatcag agccatttat aaaaggctta 2100
   ggcaatagag tagggccaaa aagcctgacc tcactctaac tcaaagtaat gtccaggttc 2160
   ccagagaata tctgctggta tttttctgta aagaccattt gcaaaattgt aacctaatac 2220
   aaagtgtagc cttcttccaa ctcaggtaga acacacctgt ctttgtcttg ctgttttcac 2280
   tcagcccttt taacattttc ccctaagccc atatgtctaa ggaaaggatg ctatttggta 2340
   atgaggaact gttatttgta tgtgaattaa agtgctctta tttt 2384
<210> 11
   <211> 1524
   <212> DNA
   <213> Human melanoma antigen recognized by T-cells (MARA
<400> 11
   agcagacaga ggactctcat taaggaaggt gtcctgtgcc ctgaccctac aagatgccaa 60
   gagaagatgc tcacttcatc tatggttacc ccaagaaggg gcacggccac tcttacacca 120
   cggctgaaga ggccgctggg atcggcatcc tgacagtgat cctgggagtc ttactgctca 180
   tcggctgttg gtattgtaga agacgaaatg gatacagagc cttgatggat aaaagtcttc 240
   atgttggcac tcaatgtgcc ttaacaagaa gatgcccaca agaagggttt gatcatcggg 300
   acagcaaagt gtctcttcaa gagaaaaact gtgaacctgt ggttcccaat gctccacctg 360
   cttatgagaa actctctgca gaacagtcac caccacctta ttcaccttaa gagccagcga 420
   gacacctgag acatgctgaa attatttctc tcacactttt gcttgaattt aatacagaca 480
   tctaatgttc tcctttggaa tggtgtagga aaaatgcaag ccatctctaa taataagtca 540
   gtgttaaaat tttagtaggt ccgctagcag tactaatcat gtgaggaaat gatgagaaat 600
   attaaattgg gaaaactcca tcaataaatg ttgcaatgca tgatactatc tgtgccagag 660
   gtaatgttag taaatccatg gtgttatttt ctgagagaca gaattcaagt gggtattctg 720
   gggccatcca atttctcttt acttgaaatt tggctaataa caaactagtc aggttttcga 780
   accttgaccg acatgaactg tacacagaat tgttccagta ctatggagtg ctcacaaagg 840
   atacttttac aggttaagac aaagggttga ctggcctatt tatctgatca agaacatgtc 900
   agcaatgtct ctttgtgctc taaaattcta ttatactaca ataatatatt gtaaagatcc 960
   tatagctctt tttttttgag atggagtttc gcttttgttg cccaggctgg agtgcaatgg 1020
   cgcgatcttg gctcaccata acctccgcct cccaggttca agcaattctc ctgccttagc 1080
   ctcctgagta gctgggatta caggcgtgcg ccactatgcc tgactaattt tgtagtttta 1140
   gtagagacgg ggtttctcca tgttggtcag gctggtctca aactcctgac ctcaggtgat 1200
   ctgcccgcct cagcctccca aagtgctgga attacaggcg tgagccacca cgcctggctg 1260
   gatcctatat cttaggtaag acatataacg cagtctaatt acatttcact tcaaggctca 1320
   atgctattct aactaatgac aagtattttc tactaaacca gaaattggta gaaggattta 1380
   aataagtaaa agctactatg tactgcctta gtgctgatgc ctgtgtactg ccttaaatgt 1440
   acctatggca atttagctct cttgggttcc caaatccctc tcacaagaat gtgcagaaga 1500
   aatcataaag gatcagagat tctg 1524
<210> 12
   <211> 1466
   <212> DNA
   <213> Homo sapiens synovial sarcoma, X breakpoint 2 (SSA
<400> 12
   gcatgctctg actttctctc tctttcgatt cttccatact cagagtacgc acggtctgat 60
   tttctctttg gattcttcca aaatcagagt cagactgctc ccggtgccat gaacggagac 120
   gacgcctttg caaggagacc cacggttggt gctcaaatac cagagaagat ccaaaaggcc 180
   ttcgatgata ttgccaaata cttctctaag gaagagtggg aaaagatgaa agcctcggag 240
   aaaatcttct atgtgtatat gaagagaaag tatgaggcta tgactaaact aggtttcaag 300
   gccaccctcc cacctttcat gtgtaataaa cgggccgaag acttccaggg gaatgatttg 360
   gataatgacc ctaaccgtgg gaatcaggtt gaacgtcctc agatgacttt cggcaggctc 420
   cagggaatct ccccgaagat catgcccaag aagccagcag aggaaggaaa tgattcggag 480
   gaagtgccag aagcatctgg cccacaaaat gatgggaaag agctgtgccc cccgggaaaa 540
   ccaactacct ctgagaagat tcacgagaga tctggaaata gggaggccca agaaaaggaa 600
   gagagacgcg gaacagctca tcggtggagc agtcagaaca cacacaacat tggtcgattc 660
   agtttgtcaa cttctatggg tgcagttcat ggtaccccca aaacaattac acacaacagg 720
   gacccaaaag gggggaacat gcctggaccc acagactgcg tgagagaaaa cagctggtga 780
   tttatgaaga gatcagcgac cctgaggaag atgacgagta actcccctca gggatacgac 840
   acatgcccat gatgagaagc agaacgtggt gacctttcac gaacatgggc atggctgcgg 900
   acccctcgtc atcaggtgca tagcaagtga aagcaagtgt tcacaacagt gaaaagttga 960
   gcgtcatttt tcttagtgtg ccaagagttc gatgttagcg tttacgttgt attttcttac 1020
   actgtgtcat tctgttagat actaacattt tcattgatga gcaagacata cttaatgcat 1080
   atttggtttt gtgtatccat gcacctacct tagaaaacaa gtattgtcgg ttacctctgc 1140
   atggaacagc attaccctcc tctctcccca gatgtgacta ctgagggcag ttctgagtgt 1200
   ttaatttcag attttttcct ctgcatttac acacacacgc acacaaacca caccacacac 1260
   acacacacac acacacacac acacacacac acacaccaag taccagtata agcatctgcc 1320
   atctgctttt cccattgcca tgcgtcctgg tcaagctccc ctcactctgt ttcctggtca 1380
   gcatgtactc ccctcatccg attcccctgt agcagtcact gacagttaat aaacctttgc 1440
   aaacgttcaa aaaaaaaaaa aaaaaa 1466
<210> 13
   <211> 2653
   <212> DNA
   <213> Homo sapiens folate hydrolase (prostate-specific A
<400> 13
   ctcaaaaggg gccggatttc cttctcctgg aggcagatgt tgcctctctc tctcgctcgg 60
   attggttcag tgcactctag aaacactgct gtggtggaga aactggaccc caggtctgga 120
   gcgaattcca gcctgcaggg ctgataagcg aggcattagt gagattgaga gagactttac 180
   cccgccgtgg tggttggagg gcgcgcagta gagcagcagc acaggcgcgg gtcccgggag 240
   gccggctctg ctcgcgccga gatgtggaat ctccttcacg aaaccgactc ggctgtggcc 300
   accgcgcgcc gcccgcgctg gctgtgcgct ggggcgctgg tgctggcggg tggcttcttt 360
   ctcctcggct tcctcttcgg gtggtttata aaatcctcca atgaagctac taacattact 420
   ccaaagcata atatgaaagc atttttggat gaattgaaag ctgagaacat caagaagttc 480
   ttatataatt ttacacagat accacattta gcaggaacag aacaaaactt tcagcttgca 540
   aagcaaattc aatcccagtg gaaagaattt ggcctggatt ctgttgagct agcacattat 600
   gatgtcctgt tgtcctaccc aaataagact catcccaact acatctcaat aattaatgaa 660
   gatggaaatg agattttcaa cacatcatta tttgaaccac ctcctccagg atatgaaaat 720
   gtttcggata ttgtaccacc tttcagtgct ttctctcctc aaggaatgcc agagggcgat 780
   ctagtgtatg ttaactatgc acgaactgaa gacttcttta aattggaacg ggacatgaaa 840
   atcaattgct ctgggaaaat tgtaattgcc agatatggga aagttttcag aggaaataag 900
   gttaaaaatg cccagctggc aggggccaaa ggagtcattc tctactccga ccctgctgac 960
   tactttgctc ctggggtgaa gtcctatcca gatggttgga atcttcctgg aggtggtgtc 1020
   cagcgtggaa atatcctaaa tctgaatggt gcaggagacc ctctcacacc aggttaccca 1080
   gcaaatgaat atgcttatag gcgtggaatt gcagaggctg ttggtcttcc aagtattcct 1140
   gttcatccaa ttggatacta tgatgcacag aagctcctag aaaaaatggg tggctcagca 1200
   ccaccagata gcagctggag aggaagtctc aaagtgccct acaatgttgg acctggcttt 1260
   actggaaact tttctacaca aaaagtcaag atgcacatcc actctaccaa tgaagtgaca 1320
   agaatttaca atgtgatagg tactctcaga ggagcagtgg aaccagacag atatgtcatt 1380
   ctgggaggtc accgggactc atgggtgttt ggtggtattg accctcagag tggagcagct 1440
   gttgttcatg aaattgtgag gagctttgga acactgaaaa aggaagggtg gagacctaga 1500
   agaacaattt tgtttgcaag ctgggatgca gaagaatttg gtcttcttgg ttctactgag 1560
   tgggcagagg agaattcaag actccttcaa gagcgtggcg tggcttatat taatgctgac 1620
   tcatctatag aaggaaacta cactctgaga gttgattgta caccgctgat gtacagcttg 1680
   gtacacaacc taacaaaaga gctgaaaagc cctgatgaag gctttgaagg caaatctctt 1740
   tatgaaagtt ggactaaaaa aagtccttcc ccagagttca gtggcatgcc caggataagc 1800
   aaattgggat ctggaaatga ttttgaggtg ttcttccaac gacttggaat tgcttcaggc 1860
   agagcacggt atactaaaaa ttgggaaaca aacaaattca gcggctatcc actgtatcac 1920
   agtgtctatg aaacatatga gttggtggaa aagttttatg atccaatgtt taaatatcac 1980
   ctcactgtgg cccaggttcg aggagggatg gtgtttgagc tagccaattc catagtgctc 2040
   ccttttgatt gtcgagatta tgctgtagtt ttaagaaagt atgctgacaa aatctacagt 2100
   atttctatga aacatccaca ggaaatgaag acatacagtg tatcatttga ttcacttttt 2160
   tctgcagtaa agaattttac agaaattgct tccaagttca gtgagagact ccaggacttt 2220
   gacaaaagca acccaatagt attaagaatg atgaatgatc aactcatgtt tctggaaaga 2280
   gcatttattg atccattagg gttaccagac aggccttttt ataggcatgt catctatgct 2340
   ccaagcagcc acaacaagta tgcaggggag tcattcccag gaatttatga tgctctgttt 2400
   gatattgaaa gcaaagtgga cccttccaag gcctggggag aagtgaagag acagatttat 2460
   gttgcagcct tcacagtgca ggcagctgca gagactttga gtgaagtagc ctaagaggat 2520
   tctttagaga atccgtattg aatttgtgtg gtatgtcact cagaaagaat cgtaatgggt 2580
   atattgataa attttaaaat tggtatattt gaaataaagt tgaatattat atataaaaaa 2640
   aaaaaaaaaa aaa 2653
<210> 14
   <211> 2420
   <212> DNA
   <213> Human antigen (MAGE-1) gene, complete cds
<400> 14
   ggatccaggc cctgccagga aaaatataag ggccctgcgt gagaacagag ggggtcatcc 60
   actgcatgag agtggggatg tcacagagtc cagcccaccc tcctggtagc actgagaagc 120
   cagggctgtg cttgcggtct gcaccctgag ggcccgtgga ttcctcttcc tggagctcca 180
   ggaaccaggc agtgaggcct tggtctgaga cagtatcctc aggtcacaga gcagaggatg 240
   cacagggtgt gccagcagtg aatgtttgcc ctgaatgcac accaagggcc ccacctgcca 300
   caggacacat aggactccac agagtctggc ctcacctccc tactgtcagt cctgtagaat 360
   cgacctctgc tggccggctg taccctgagt accctctcac ttcctccttc aggttttcag 420
   gggacaggcc aacccagagg acaggattcc ctggaggcca cagaggagca ccaaggagaa 480
   gatctgtaag taggcctttg ttagagtctc caaggttcag ttctcagctg aggcctctca 540
   cacactccct ctctccccag gcctgtgggt cttcattgcc cagctcctgc ccacactcct 600
   gcctgctgcc ctgacgagag tcatcatgtc tcttgagcag aggagtctgc actgcaagcc 660
   tgaggaagcc cttgaggccc aacaagaggc cctgggcctg gtgtgtgtgc aggctgccac 720
   ctcctcctcc tctcctctgg tcctgggcac cctggaggag gtgcccactg ctgggtcaac 780
   agatcctccc cagagtcctc agggagcctc cgcctttccc actaccatca acttcactcg 840
   acagaggcaa cccagtgagg gttccagcag ccgtgaagag gaggggccaa gcacctcttg 900
   tatcctggag tccttgttcc gagcagtaat cactaagaag gtggctgatt tggttggttt 960
   tctgctcctc aaatatcgag ccagggagcc agtcacaaag gcagaaatgc tggagagtgt 1020
   catcaaaaat tacaagcact gttttcctga gatcttcggc aaagcctctg agtccttgca 1080
   gctggtcttt ggcattgacg tgaaggaagc agaccccacc ggccactcct atgtccttgt 1140
   cacctgccta ggtctctcct atgatggcct gctgggtgat aatcagatca tgcccaagac 1200
   aggcttcctg ataattgtcc tggtcatgat tgcaatggag ggcggccatg ctcctgagga 1260
   ggaaatctgg gaggagctga gtgtgatgga ggtgtatgat gggagggagc acagtgccta 1320
   tggggagccc aggaagctgc tcacccaaga tttggtgcag gaaaagtacc tggagtaccg 1380
   gcaggtgccg gacagtgatc ccgcacgcta tgagttcctg tggggtccaa gggccctcgc 1440
   tgaaaccagc tatgtgaaag tccttgagta tgtgatcaag gtcagtgcaa gagttcgctt 1500
   tttcttccca tccctgcgtg aagcagcttt gagagaggag gaagagggag tctgagcatg 1560
   agttgcagcc aaggccagtg ggagggggac tgggccagtg caccttccag ggccgcgtcc 1620
   agcagcttcc cctgcctcgt gtgacatgag gcccattctt cactctgaag agagcggtca 1680
   gtgttctcag tagtaggttt ctgttctatt gggtgacttg gagatttatc tttgttctct 1740
   tttggaattg ttcaaatgtt tttttttaag ggatggttga atgaacttca gcatccaagt 1800
   ttatgaatga cagcagtcac acagttctgt gtatatagtt taagggtaag agtcttgtgt 1860
   tttattcaga ttgggaaatc cattctattt tgtgaattgg gataataaca gcagtggaat 1920
   aagtacttag aaatgtgaaa aatgagcagt aaaatagatg agataaagaa ctaaagaaat 1980
   taagagatag tcaattcttg ccttatacct cagtctattc tgtaaaattt ttaaagatat 2040
   atgcatacct ggatttcctt ggcttctttg agaatgtaag agaaattaaa tctgaataaa 2100
   gaattcttcc tgttcactgg ctcttttctt ctccatgcac tgagcatctg ctttttggaa 2160
   ggccctgggt tagtagtgga gatgctaagg taagccagac tcatacccac ccatagggtc 2220
   gtagagtcta ggagctgcag tcacgtaatc gaggtggcaa gatgtcctct aaagatgtag 2280
   ggaaaagtga gagaggggtg agggtgtggg gctccgggtg agagtggtgg agtgtcaatg 2340
   ccctgagctg ggcatttttg ggctttggga aactgcagtt ccttctgggg gagctgattg 2400
   taatgatctt gggtggatcc 2420
<210> 15
   <211> 4204
   <212> DNA
   <213> Human MAGE-3 antigen (MAGE-3) gene, complete cds
<400> 15
   acgcaggcag tgatgtcacc cagaccacac cccttccccc aatgccactt cagggggtac 60
   tcagagtcag agacttggtc tgaggggagc agaagcaatc tgcagaggat ggcggtccag 120
   gctcagccag gcatcaactt caggaccctg agggatgacc gaaggccccg cccacccacc 180
   cccaactccc ccgaccccac caggatctac agcctcagga cccccgtccc aatccttacc 240
   ccttgcccca tcaccatctt catgcttacc tccaccccca tccgatcccc atccaggcag 300
   aatccagttc cacccctgcc cggaacccag ggtagtaccg ttgccaggat gtgacgccac 360
   tgacttgcgc attggaggtc agaagaccgc gagattctcg ccctgagcaa cgagcgacgg 420
   cctgacgtcg gcggagggaa gccggcccag gctcggtgag gaggcaaggt aagacgctga 480
   gggaggactg aggcgggcct cacctcagac agagggcctc aaataatcca gtgctgcctc 540
   tgctgccggg cctgggccac cccgcagggg aagacttcca ggctgggtcg ccactacctc 600
   accccgccga cccccgccgc tttagccacg gggaactctg gggacagagc ttaatgtggc 660
   cagggcaggg ctggttagaa gaggtcaggg cccacgctgt ggcaggaatc aaggtcagga 720
   ccccgagagg gaactgaggg cagcctaacc accaccctca ccaccattcc cgtcccccaa 780
   cacccaaccc cacccccatc ccccattccc atccccaccc ccacccctat cctggcagaa 840
   tccgggcttt gcccctggta tcaagtcacg gaagctccgg gaatggcggc caggcacgtg 900
   agtcctgagg ttcacatcta cggctaaggg agggaagggg ttcggtatcg cgagtatggc 960
   cgttgggagg cagcgaaagg gcccaggcct cctggaagac agtggagtcc tgaggggacc 1020
   cagcatgcca ggacaggggg cccactgtac ccctgtctca aaccgaggca ccttttcatt 1080
   cggctacggg aatcctaggg atgcagaccc acttcagcag ggggttgggg cccagccctg 1140
   cgaggagtca tggggaggaa gaagagggag gactgagggg accttggagt ccagatcagt 1200
   ggcaaccttg ggctggggga tgctgggcac agtggccaaa tgtgctctgt gctcattgcg 1260
   ccttcagggt gaccagagag ttgagggctg tggtctgaag agtgggactt caggtcagca 1320
   gagggaggaa tcccaggatc tgcagggccc aaggtgtacc cccaaggggc ccctatgtgg 1380
   tggacagatg cagtggtcct aggatctgcc aagcatccag gtgaagagac tgagggagga 1440
   ttgagggtac ccctgggaca gaatgcggac tgggggcccc ataaaaatct gccctgctcc 1500
   tgctgttacc tcagagagcc tgggcagggc tgtcagctga ggtccctcca ttatcctagg 1560
   atcactgatg tcagggaagg ggaagccttg gtctgagggg gctgcactca gggcagtaga 1620
   gggaggctct cagaccctac taggagtgga ggtgaggacc aagcagtctc ctcacccagg 1680
   gtacatggac ttcaataaat ttggacatct ctcgttgtcc tttccgggag gacctgggaa 1740
   tgtatggcca gatgtgggtc ccctcatgtt tttctgtacc atatcaggta tgtgagttct 1800
   tgacatgaga gattctcagg ccagcagaag ggagggatta ggccctataa ggagaaaggt 1860
   gagggccctg agtgagcaca gaggggatcc tccaccccag tagagtgggg acctcacaga 1920
   gtctggccaa ccctcctgac agttctggga atccgtggct gcgtttgctg tctgcacatt 1980
   gggggcccgt ggattcctct cccaggaatc aggagctcca ggaacaaggc agtgaggact 2040
   tggtctgagg cagtgtcctc aggtcacaga gtagaggggg ctcagatagt gccaacggtg 2100
   aaggtttgcc ttggattcaa accaagggcc ccacctgccc cagaacacat ggactccaga 2160
   gcgcctggcc tcaccctcaa tactttcagt cctgcagcct cagcatgcgc tggccggatg 2220
   taccctgagg tgccctctca cttcctcctt caggttctga ggggacaggc tgacctggag 2280
   gaccagaggc ccccggagga gcactgaagg agaagatctg taagtaagcc tttgttagag 2340
   cctccaaggt tccattcagt actcagctga ggtctctcac atgctccctc tctccccagg 2400
   ccagtgggtc tccattgccc agctcctgcc cacactcccg cctgttgccc tgaccagagt 2460
   catcatgcct cttgagcaga ggagtcagca ctgcaagcct gaagaaggcc ttgaggcccg 2520
   aggagaggcc ctgggcctgg tgggtgcgca ggctcctgct actgaggagc aggaggctgc 2580
   ctcctcctct tctactctag ttgaagtcac cctgggggag gtgcctgctg ccgagtcacc 2640
   agatcctccc cagagtcctc agggagcctc cagcctcccc actaccatga actaccctct 2700
   ctggagccaa tcctatgagg actccagcaa ccaagaagag gaggggccaa gcaccttccc 2760
   tgacctggag tccgagttcc aagcagcact cagtaggaag gtggccgagt tggttcattt 2820
   tctgctcctc aagtatcgag ccagggagcc ggtcacaaag gcagaaatgc tggggagtgt 2880
   cgtcggaaat tggcagtatt tctttcctgt gatcttcagc aaagcttcca gttccttgca 2940
   gctggtcttt ggcatcgagc tgatggaagt ggaccccatc ggccacttgt acatctttgc 3000
   cacctgcctg ggcctctcct acgatggcct gctgggtgac aatcagatca tgcccaaggc 3060
   aggcctcctg ataatcgtcc tggccataat cgcaagagag ggcgactgtg cccctgagga 3120
   gaaaatctgg gaggagctga gtgtgttaga ggtgtttgag gggagggaag acagtatctt 3180
   gggggatccc aagaagctgc tcacccaaca tttcgtgcag gaaaactacc tggagtaccg 3240
   gcaggtcccc ggcagtgatc ctgcatgtta tgaattcctg tggggtccaa gggccctcgt 3300
   tgaaaccagc tatgtgaaag tcctgcacca tatggtaaag atcagtggag gacctcacat 3360
   ttcctaccca cccctgcatg agtgggtttt gagagagggg gaagagtgag tctgagcacg 3420
   agttgcagcc agggccagtg ggagggggtc tgggccagtg caccttccgg ggccgcatcc 3480
   cttagtttcc actgcctcct gtgacgtgag gcccattctt cactctttga agcgagcagt 3540
   cagcattctt agtagtgggt ttctgttctg ttggatgact ttgagattat tctttgtttc 3600
   ctgttggagt tgttcaaatg ttccttttaa cggatggttg aatgagcgtc agcatccagg 3660
   tttatgaatg acagtagtca cacatagtgc tgtttatata gtttaggagt aagagtcttg 3720
   ttttttactc aaattgggaa atccattcca ttttgtgaat tgtgacataa taatagcagt 3780
   ggtaaaagta tttgcttaaa attgtgagcg aattagcaat aacatacatg agataactca 3840
   agaaatcaaa agatagttga ttcttgcctt gtacctcaat ctattctgta aaattaaaca 3900
   aatatgcaaa ccaggatttc cttgacttct ttgagaatgc aagcgaaatt aaatctgaat 3960
   aaataattct tcctcttcac tggctcgttt cttttccgtt cactcagcat ctgctctgtg 4020
   ggaggccctg ggttagtagt ggggatgcta aggtaagcca gactcacgcc tacccatagg 4080
   gctgtagagc ctaggacctg cagtcatata attaaggtgg tgagaagtcc tgtaagatgt 4140
   agaggaaatg taagagaggg gtgagggtgt ggcgctccgg gtgagagtag tggagtgtca 4200
   gtgc                                                              4204
<210> 16
   <211> 752
   <212> DNA
   <213> Human autoimmunogenic cancer/testis antigen NY-ESs
<400> 16
   atcctcgtgg gccctgacct tctctctgag agccgggcag aggctccgga gccatgcagg 60
   ccgaaggccg gggcacaggg ggttcgacgg gcgatgctga tggcccagga ggccctggca 120
   ttcctgatgg cccagggggc aatgctggcg gcccaggaga ggcgggtgcc acgggcggca 180
   gaggtccccg gggcgcaggg gcagcaaggg cctcggggcc gggaggaggc gccccgcggg 240
   gtccgcatgg cggcgcggct tcagggctga atggatgctg cagatgcggg gccagggggc 300
   cggagagccg cctgcttgag ttctacctcg ccatgccttt cgcgacaccc atggaagcag 360
   agctggcccg caggagcctg gcccaggatg ccccaccgct tcccgtgcca ggggtgcttc 420
   tgaaggagtt cactgtgtcc ggcaacatac tgactatccg actgactgct gcagaccacc 480
   gccaactgca gctctccatc agctcctgtc tccagcagct ttccctgttg atgtggatca 540
   cgcagtgctt tctgcccgtg tttttggctc agcctccctc agggcagagg cgctaagccc 600
   agcctggcgc cccttcctag gtcatgcctc ctcccctagg gaatggtccc agcacgagtg 660
   gccagttcat tgtgggggcc tgattgtttg tcgctggagg aggacggctt acatgtttgt 720
   ttctgtagaa aataaaactg agctacgaaa aa                               752
<210> 17
   <211> 2148
   <212> DNA
   <213> Homo sapiens preferentially expressed antigen in A
<400> 17
   gcttcagggt acagctcccc cgcagccaga agccgggcct gcagcgcctc agcaccgctc 60
   cgggacaccc cacccgcttc ccaggcgtga cctgtcaaca gcaacttcgc ggtgtggtga 120
   actctctgag gaaaaaccat tttgattatt actctcagac gtgcgtggca acaagtgact 180
   gagacctaga aatccaagcg ttggaggtcc tgaggccagc ctaagtcgct tcaaaatgga 240
   acgaaggcgt ttgtggggtt ccattcagag ccgatacatc agcatgagtg tgtggacaag 300
   cccacggaga cttgtggagc tggcagggca gagcctgctg aaggatgagg ccctggccat 360
   tgccgccctg gagttgctgc ccagggagct cttcccgcca ctcttcatgg cagcctttga 420
   cgggagacac agccagaccc tgaaggcaat ggtgcaggcc tggcccttca cctgcctccc 480
   tctgggagtg ctgatgaagg gacaacatct tcacctggag accttcaaag ctgtgcttga 540
   tggacttgat gtgctccttg cccaggaggt tcgccccagg aggtggaaac ttcaagtgct 600
   ggatttacgg aagaactctc atcaggactt ctggactgta tggtctggaa acagggccag 660
   tctgtactca tttccagagc cagaagcagc tcagcccatg acaaagaagc gaaaagtaga 720
   tggtttgagc acagaggcag agcagccctt cattccagta gaggtgctcg tagacctgtt 780
   cctcaaggaa ggtgcctgtg atgaattgtt ctcctacctc attgagaaag tgaagcgaaa 840
   gaaaaatgta ctacgcctgt gctgtaagaa gctgaagatt tttgcaatgc ccatgcagga 900
   tatcaagatg atcctgaaaa tggtgcagct ggactctatt gaagatttgg aagtgacttg 960
   tacctggaag ctacccacct tggcgaaatt ttctccttac ctgggccaga tgattaatct 1020
   gcgtagactc ctcctctccc acatccatgc atcttcctac atttccccgg agaaggaaga 1080
   gcagtatatc gcccagttca cctctcagtt cctcagtctg cagtgcctgc aggctctcta 1140
   tgtggactct ttatttttcc ttagaggccg cctggatcag ttgctcaggc acgtgatgaa 1200
   ccccttggaa accctctcaa taactaactg ccggctttcg gaaggggatg tgatgcatct 1260
   gtcccagagt cccagcgtca gtcagctaag tgtcctgagt ctaagtgggg tcatgctgac 1320
   cgatgtaagt cccgagcccc tccaagctct gctggagaga gcctctgcca ccctccagga 1380
   cctggtcttt gatgagtgtg ggatcacgga tgatcagctc cttgccctcc tgccttccct 1440
   gagccactgc tcccagctta caaccttaag cttctacggg aattccatct ccatatctgc 1500
   cttgcagagt ctcctgcagc acctcatcgg gctgagcaat ctgacccacg tgctgtatcc 1560
   tgtccccctg gagagttatg aggacatcca tggtaccctc cacctggaga ggcttgccta 1620
   tctgcatgcc aggctcaggg agttgctgtg tgagttgggg cggcccagca tggtctggct 1680
   tagtgccaac ccctgtcctc actgtgggga cagaaccttc tatgacccgg agcccatcct 1740
   gtgcccctgt ttcatgccta actagctggg tgcacatatc aaatgcttca ttctgcatac 1800
   ttggacacta aagccaggat gtgcatgcat cttgaagcaa caaagcagcc acagtttcag 1860
   acaaatgttc agtgtgagtg aggaaaacat gttcagtgag gaaaaaacat tcagacaaat 1920
   gttcagtgag gaaaaaaagg ggaagttggg gataggcaga tgttgacttg aggagttaat 1980
   gtgatctttg gggagataca tcttatagag ttagaaatag aatctgaatt tctaaaggga 2040
   gattctggct tgggaagtac atgtaggagt taatccctgt gtagactgtt gtaaagaaac 2100
   tgttgaaaat aaagagaagc aatgtgaagc aaaaaaaaaa aaaaaaaa          2148
<210> 18
   <211> 4530
   <212> DNA
   <213> Human tyrosine kinase-type receptor (HER2) mRNA, s
<400> 18
   aattctcgag ctcgtcgacc ggtcgacgag ctcgagggtc gacgagctcg agggcgcgcg 60
   cccggccccc acccctcgca gcaccccgcg ccccgcgccc tcccagccgg gtccagccgg 120
   agccatgggg ccggagccgc agtgagcacc atggagctgg cggccttgtg ccgctggggg 180
   ctcctcctcg ccctcttgcc ccccggagcc gcgagcaccc aagtgtgcac cggcacagac 240
   atgaagctgc ggctccctgc cagtcccgag acccacctgg acatgctccg ccacctctac 300
   cagggctgcc aggtggtgca gggaaacctg gaactcacct acctgcccac caatgccagc 360
   ctgtccttcc tgcaggatat ccaggaggtg cagggctacg tgctcatcgc tcacaaccaa 420
   gtgaggcagg tcccactgca gaggctgcgg attgtgcgag gcacccagct ctttgaggac 480
   aactatgccc tggccgtgct agacaatgga gacccgctga acaataccac ccctgtcaca 540
   ggggcctccc caggaggcct gcgggagctg cagcttcgaa gcctcacaga gatcttgaaa 600
   ggaggggtct tgatccagcg gaacccccag ctctgctacc aggacacgat tttgtggaag 660
   gacatcttcc acaagaacaa ccagctggct ctcacactga tagacaccaa ccgctctcgg 720
   gcctgccacc cctgttctcc gatgtgtaag ggctcccgct gctggggaga gagttctgag 780
   gattgtcaga gcctgacgcg cactgtctgt gccggtggct gtgcccgctg caaggggcca 840
   ctgcccactg actgctgcca tgagcagtgt gctgccggct gcacgggccc caagcactct 900
   gactgcctgg cctgcctcca cttcaaccac agtggcatct gtgagctgca ctgcccagcc 960
   ctggtcacct acaacacaga cacgtttgag tccatgccca atcccgaggg ccggtataca 1020
   ttcggcgcca gctgtgtgac tgcctgtccc tacaactacc tttctacgga cgtgggatcc 1080
   tgcaccctcg tctgccccct gcacaaccaa gaggtgacag cagaggatgg aacacagcgg 1140
   tgtgagaagt gcagcaagcc ctgtgcccga gtgtgctatg gtctgggcat ggagcacttg 1200
   cgagaggtga gggcagttac cagtgccaat atccaggagt ttgctggctg caagaagatc 1260
   tttgggagcc tggcatttct gccggagagc tttgatgggg acccagcctc caacactgcc 1320
   ccgctccagc cagagcagct ccaagtgttt gagactctgg aagagatcac aggttaccta 1380
   tacatctcag catggccgga cagcctgcct gacctcagcg tcttccagaa cctgcaagta 1440
   atccggggac gaattctgca caatggcgcc tactcgctga ccctgcaagg gctgggcatc 1500
   agctggctgg ggctgcgctc actgagggaa ctgggcagtg gactggccct catccaccat 1560
   aacacccacc tctgcttcgt gcacacggtg ccctgggacc agctctttcg gaacccgcac 1620
   caagctctgc tccacactgc caaccggcca gaggacgagt gtgtgggcga gggcctggcc 1680
   tgccaccagc tgtgcgcccg agggcactgc tggggtccag ggcccaccca gtgtgtcaac 1740
   tgcagccagt tccttcgggg ccaggagtgc gtggaggaat gccgagtact gcaggggctc 1800
   cccagggagt atgtgaatgc caggcactgt ttgccgtgcc accctgagtg tcagccccag 1860
   aatggctcag tgacctgttt tggaccggag gctgaccagt gtgtggcctg tgcccactat 1920
   aaggaccctc ccttctgcgt ggcccgctgc cccagcggtg tgaaacctga cctctcctac 1980
   atgcccatct ggaagtttcc agatgaggag ggcgcatgcc agccttgccc catcaactgc 2040
   acccactcct gtgtggacct ggatgacaag ggctgccccg ccgagcagag agccagccct 2100
   ctgacgtcca tcgtctctgc ggtggttggc attctgctgg tcgtggtctt gggggtggtc 2160
   tttgggatcc tcatcaagcg acggcagcag aagatccgga agtacacgat gcggagactg 2220
   ctgcaggaaa cggagctggt ggagccgctg acacctagcg gagcgatgcc caaccaggcg 2280
   cagatgcgga tcctgaaaga gacggagctg aggaaggtga aggtgcttgg atctggcgct 2340
   tttggcacag tctacaaggg catctggatc cctgatgggg agaatgtgaa aattccagtg 2400
   gccatcaaag tgttgaggga aaacacatcc cccaaagcca acaaagaaat cttagacgaa 2460
   gcatacgtga tggctggtgt gggctcccca tatgtctccc gccttctggg catctgcctg 2520
   acatccacgg tgcagctggt gacacagctt atgccctatg gctgcctctt agaccatgtc 2580
   cgggaaaacc gcggacgcct gggctcccag gacctgctga actggtgtat gcagattgcc 2640
   aaggggatga gctacctgga ggatgtgcgg ctcgtacaca gggacttggc cgctcggaac 2700
   gtgctggtca agagtcccaa ccatgtcaaa attacagact tcgggctggc tcggctgctg 2760
   gacattgacg agacagagta ccatgcagat gggggcaagg tgcccatcaa gtggatggcg 2820
   ctggagtcca ttctccgccg gcggttcacc caccagagtg atgtgtggag ttatggtgtg 2880
   actgtgtggg agctgatgac ttttggggcc aaaccttacg atgggatccc agcccgggag 2940
   atccctgacc tgctggaaaa gggggagcgg ctgccccagc cccccatctg caccattgat 3000
   gtctacatga tcatggtcaa atgttggatg attgactctg aatgtcggcc aagattccgg 3060
   gagttggtgt ctgaattctc ccgcatggcc agggaccccc agcgctttgt ggtcatccag 3120
   aatgaggact tgggcccagc cagtcccttg gacagcacct tctaccgctc actgctggag 3180
   gacgatgaca tgggggacct ggtggatgct gaggagtatc tggtacccca gcagggcttc 3240
   ttctgtccag accctgcccc gggcgctggg ggcatggtcc accacaggca ccgcagctca 3300
   tctaccagga gtggcggtgg ggacctgaca ctagggctgg agccctctga agaggaggcc 3360
   cccaggtctc cactggcacc ctccgaaggg gctggctccg atgtatttga tggtgacctg 3420
   ggaatggggg cagccaaggg gctgcaaagc ctccccacac atgaccccag ccctctacag 3480
   cggtacagtg aggaccccac agtacccctg ccctctgaga ctgatggcta cgttgccccc 3540
   ctgacctgca gcccccagcc tgaatatgtg aaccagccag atgttcggcc ccagccccct 3600
   tcgccccgag agggccctct gcctgctgcc cgacctgctg gtgccactct ggaaagggcc 3660
   aagactctct ccccagggaa gaatggggtc gtcaaagacg tttttgcctt tgggggtgcc 3720
   gtggagaacc ccgagtactt gacaccccag ggaggagctg cccctcagcc ccaccctcct 3780
   cctgccttca gcccagcctt cgacaacctc tattactggg accaggaccc accagagcgg 3840
   ggggctccac ccagcacctt caaagggaca cctacggcag agaacccaga gtacctgggt 3900
   ctggacgtgc cagtgtgaac cagaaggcca agtccgcaga agccctgatg tgtcctcagg 3960
   gagcagggaa ggcctgactt ctgctggcat caagaggtgg gagggccctc cgaccacttc 4020
   caggggaacc tgccatgcca ggaacctgtc ctaaggaacc ttccttcctg cttgagttcc 4080
   cagatggctg gaaggggtcc agcctcgttg gaagaggaac agcactgggg agtctttgtg 4140
   gattctgagg ccctgcccaa tgagactcta gggtccagtg gatgccacag cccagcttgg 4200
   ccctttcctt ccagatcctg ggtactgaaa gccttaggga agctggcctg agaggggaag 4260
   cggccctaag ggagtgtcta agaacaaaag cgacccattc agagactgtc cctgaaacct 4320
   agtactgccc cccatgagga aggaacagca atggtgtcag tatccaggct ttgtacagag 4380
   tgcttttctg tttagttttt actttttttg ttttgttttt ttaaagacga aataaagacc 4440
   caggggagaa tgggtgttgt atggggaggc aagtgtgggg ggtccttctc cacacccact 4500
   ttgtccattt gcaaatatat tttggaaaac                                  4530

## Description

### Background of the Invention

### Field of the Invention

Disclosed herein are methods and compositions for inducing an immune response against various combinations of tumor-associated antigens, which can promote effective immunologic intervention in pathogenic processes.

### Description of the Related Art

The American Cancer Society has estimated that over one million people get cancer each year, and that approximately one out of every two American men and one out of every three American women will have some type of cancer at some point during their lifetime.

Cancer generally develops when cells in a part of the body begin to grow out of control. Although there are many kinds of cancer, they usually start because of out-of-control growth of abnormal cells.

Normal body cells grow, divide, and die in an orderly fashion. Cancer cells are different in that they continue to grow and divide. Instead of dying, they outlive normal cells and continue to form new abnormal cells.

Usual treatment options for cancer include surgery, radiation therapy, and chemotherapy. A fourth branch of treatment is developing, which is referred to as immunotherapy. Immunotherapies attempt to help the immune system recognize cancer cells, and/or to strengthen a response against cancer cells in order to destroy the cancer. Immunotherapies include active and passive immunotherapies. Active immuotherapies attempt to stimulate the body's own immune system to fight the disease. Passive immunotherapies generally do not rely on the body to attack the disease; instead, they use immune system components (such as antibodies) created outside of the body.

Despite the various types of treatments, a continuing need exists for additional treatment options.

### Summary of the Invention

Embodiments of the invention disclosed herein are directed to the use of effective combinations of TuAAs for the immunotherapy of patients with various types of cancer. Both immunogenic compositions for inducing an immune response to these combinations of antigens and methods for their use are disclosed.

Some embodiments relate to methods of treating neoplastic diseases. The methods can include the step of immunizing a patient against, for example, PRAME and at least one other tumor associated antigen. To immunize a patient against an antigen such as, for example, PRAME, means to administer to the patient some portion of the antigen, or some other immunogenic agent, that is capable of inducing a specific immune response directed to the antigen. Accordingly, in some embodiments, immunizing against PRAME can include administering a complete and intact PRAME antigen to the patient, while in other embodiments it can include administering one or more epitopes, one or more epitope clusters, one or more fragments, and the like, of PRAME, and/or administering, for example, a nucleic acid encoding any of the foregoing epitope(s), cluster(s), fragment(s), and the like. Although PRAME is used in an exemplary way for convenience in this discussion, the principle is applicable to any antigen against which a patient may be immunized.

One embodiment relates to methods of treating ovarian or colorectal cancer. The methods can include the step of immunizing a patient against, for example, PRAME and at least one other tumor associated antigen. In some embodiments the tumor associated antigen can include, for example, SSX-2, NY-ESO-1, PSMA, and the like. Preferably, the methods can include immunizing against PRAME, NY-ESO-1 and SSX-2, for example. More preferably, the methods can include immunization against PRAME, NY-ESO-1, SSX-2, and PSMA, for example. The method can further include the step of immunizing against at least one antigen associated with tumor neovasculature. The antigen associated with tumor neovasculature can be, for example, PSMA, VEGFR2 and Tie-2. In a preferred embodiment, the methods can induce a cytolytic T cell response.

In one embodiment, a method of inducing a cytolytic T-cell response in the treatment of ovarian or colorectal cancer is disclosed. The method can include, for example, the step of immunizing a pateint against PRAME and at least one of the tumor-associated antigens. In some embodiments, the tumor-associated antigen can be, for example, SSX-2, NY-ESO-1, PSMA, and the like. For example, the method can include immunization against PRAME, NY-ESO-1, and SSX-2. The method can further include immunization against PSMA. The method can further include the step of immunizing against at least one antigen associated with tumor neovasculature. The antigen associated with tumor neovasculature can be, for example, PSMA, VEGFR2, and Tie-2.

The present disclosure also relates to methods of treating pancreatic cancer. The methods can include the step of immunizing against, for example, PSMA and at least one other tumor-associated antigen. The tumor-associated antigen can be, for example, SSX-2 and NY-ESO-1. Preferably, the methods can include immunizing against PSMA, NY-BSO-1, and SSX-2. The method can further include the step of immunizing against at least one antigen associated with tumor neovasculature. The antigen associated with tumor neovasculature can be, for example, VEGFR2 and Tie-2. In a preferred embodiment, the methods can induce a cytolytic T cell response.

Further, the present disclosure relates to a method of inducing a cytolytic T-cell response in the treatment of pancreatic cancer. The method can include, for example, the step of immunizing a patient against PSMA and at least one of the tumor-associated antigens. In some embodiments, the tumor-associated antigen can be, for example, SSX-2, NY-LSO-1, and the like. For example, the method can include immunization against PSMA, NY-ESO-1, and SSX-2. The method can further include the step of immunizing against at least one antigen associated with tumor neovasculature. The antigen associated with tumor neovasculature can be, for example, VEGFR2 and Tie-2.

The present disclosure also relates to methods of treating non-small cell lung cancer. The methods can include the step of immunizing a patient against, for example, PSMA and at least one other tumor associated antigen. For example, the tumor-associated antigen can be MAOE-3, SSX-2, NY-ESO-1, and the like. Preferably, the methods can include immunizing against PSMA, NY-ESO-1 and SSX-2. More preferably, the methods can include immunization against PSMA, NY-ESO-1, SSX-2, and MAGE-3, for example. The method can further include the step of immunizing against at least one antigen associated with tumor neovasculature. The antigen associated with tumor neovasculature can be, for example, VEGFR2 and Tie-2. In a preferred embodiment, the methods can induce a cytolytic T cell response.

The present disclosure relates to a method of inducing a cytolytic T-cell response in the treatment of non-small cell lung cancer. The method can include, for example, the step of immunizing a patient against PSMA and at least one of tumor-associated antigen. The tumor-associated antigen can be, for example, MAGE-3, SSX-2, NY-ESO-1, and the like. For example, the method can include immunization against PSMA, NY-ESO-1, and SSX-2. The method can further include immunization against PSMA, NY-ESO-1, SSX-2, and MAGE-3. The method can further include the step of immunizing against at least one antigen associated with tumor neovasculature. The antigen associated with tumor neovasculature can be, for example, VEGFR2 and Tie-2.

The present disclosure further relates to methods of treating renal cell carcinoma. The methods can include the step of immunizing a patient against, for example, PSMA and at least one other tumor associated antigen. The tumor-associated antigen can be, for example, PRAMB, SSX-2, and the like. Preferably, the methods can include immunizing against PSMA, PRAME, and SSX-2, for example. The method can further include the step of immunizing against at least one antigen associated with tumor neovasculature. The antigen associated with tumor neovasculature can be, for example, VEGFR2 and Tie-2. In a preferred embodiment, the methods can induce a cytolytic T cell response.

The present disclosure also relates to a method of inducing a cytolytic T-cell response in the treatment of renal cell carcinoma. The method can include, for example, the step of immunizing a patient against PSMA and at least one other tumor-associated antigen. In some embodiments, the tumor-associated antigen can be PRAME, SSX-2, and the like. For example, the method can include immunization against PSMA, PRAME, and SSX-2. The method can further include the step of immunizing against at least one antigen associated with tumor neovasculature. The antigen associated with tumor neovasculature can be, for example, VEGFR2 and Tie-2.

The present disclosure also relates to methods of treating melanoma. The methods can include the step of immunizing a patient against, for example, at least one tumor-associated antigen selected from each of two groups. The first group can include, for example, tyrosinase, Melan-A, and the like. The second group can include, for example, SSX-2, NY-ESO-1, and the like. Preferably, the method can include immunizing against Melan-A, SSX-2. and NY-ESO. More preferably, the method can include immunization against Melan-A, SSX-2, and tyrosinase, for example. The method can further include the step of immunizing against at least one antigen associated with tumor neovasculature. The antigen associated with tumor neovasculature can be, for example, PSMA, VEGFR2, and Tie-2. In a preferred embodiment, the methods can induce a cytolytic T cell response.

The present disclosure also relates to a method of inducing a cytolytic T-cell response in the treatment of melanoma. The method can include, for example, the step of immunizing a patient against at least one tumor-associated antigen selected from each of two groups. The first group can include, for example, tyrosinase, Melan-A, and the like. The second group can include, for example, SSX-2, NY-ESO-1, and the like. For example, the method can include immunization against Melan-A, SSX-2, and NY-ESO. Alternatively, the method can include, for example, immunization against Melan-A, SSX-2, and tyrosinase. The method can further include the step of immunizing against at least one antigen associated with tumor neovasculature. The antigen associated with tumor neovasculature can be, for example, PSMA, VBGFR2, and Tie-2.

Some embodiments relate to the use of a composition comprising PRAME and at least one other tumor associated antigen, in the preparation of a medicament for the treatment of treating ovarian or colorectal cancer. In some embodiments the tumor associated antigen include SSX-2 NY-ESO-1, PSMA. Preferably, the tumor-associated antigens can be NY-ESO-1 and SSX-2. More preferably, the tumor-associated antigens can be NY-ESO-1, SSX-2, and PSMA. The method can further include the step of immunizing against at least one antigen associated with tumor neovasculature. The antigen associated with tumor neovasculature can be PSMA, VEGFR2, and Tie-2. In a preferred embodiment, the medicament can induce a cytolytic T cell response.

The present disclosure also relates to the use of a composition comprising, for example, PSMA and at least one other tumor-associated antigen in the preparation of medicament for the treatment of pancreatic cancer. The tumor-associated antigen can be, for example, SSX-2 and NY-ESO-1. Preferably, the medicament comprises PSMA, NY-ESO-1, and SSX-2. The method can further include the step of immunizing against at least one antigen associated with tumor neovasculature. The antigen associated with tumor neovasculature can be, for example, VEGFR2 and Tie-2. In a preferred embodiment, the medicament can induce a cytolytic T cell response.

Further, the present disclosure also relates to the use of a composition comprising PSMA and at least one other tumor associated antigen in the preparation of a medicament for the treatment of non-small cell lung cancer. In some embodiments, the tumor-associated antigen can be MAGE-3, SSX-2, NY-ESO-1, and the like. Preferably, the composition comprises PSMA, NY-ESO-1 and SSX-2. More preferably, the composition comprises PSMA, NY-ESO-1, SSX-2, and MAGE-3, for example. The method can further include the step of immunizing against at least one antigen associated with tumor neovasculature. The antigen associated with tumor neovasculature can be, for example, VEGFR2 and Tie-2. In a preferred embodiment, the medicament can induce a cytolytic T cell response.

The present disclosure also relates to the use of a composition comprising PSMA and at least one other tumor associated antigen in the preparation of a medicament for the treatment of renal cell carcinoma. The tumor-associated antigen can be, for example, PRAME, SSX-2, and the like. Preferably, the composition comprises PSMA, PRAME, and SSX-2, for example. The method can further include the step of immunizing against at least one antigen associated with tumor neovasculature. The antigen associated with tumor neovasculature can be, for example, VEGFR2 and Tie-2. In a preferred embodiment, the medicament can induce a cytolytic T cell response.

The present disclosure also relates to the use of a composition comprising at least one tumor-associated antigen selected from each of two groups in the preparation of a medicament for the treatment of melanoma. The first group can include, for example, tyrosinase, Melan-A, and the like. The second group can include, for example, SSX-2, NY-ESO-1, and the like. Preferably, the composition comprises Melan-A, SSX-2, and NY-ESO. More preferably, the composition comprises Melan-A, SSX-2, and tyrosinase, for example. The method can further include the step of immunizing against at least one antigen associated with tumor neovasculature. The antigen associated with tumor neovasculature can be, for example, PSMA, VEGFR2, and Tie-2. In a preferred embodiment, the medicament can induce a cytolytic T cell response.

Other embodiments relate to immunogenic compositions for use in the treatment of or for inducing a T cell response against ovarian or colorectal cancer. The composition include, individually or in combination, 1) whole antigens, 2) fragments of antigens, 3) epitope clusters derived from antigens, 4) epitopes derived from antigens, 5) nucleic acids encoding any of 1 to 4; wherein the antigens include PRAME and a tumor-associated antigen, including, for example, SSX-2, NY-ESO-1, PSMA. The compositions can further include at least one antigen associated with tumor neovasculature. The antigen associated with tumor neovasculature can be PSMA, VEGFR2 and Tie-2.

The present disclosure further relates to immunogenic compositions for the treatment of or for inducing a T cell response against pancreatic cancer. The compositions can include, for example, individually or in combination, 1) whole antigens, 2) fragments of antigens, 3) epitope clusters derived from antigens, 4) epitopes derived from antigens, or 5) nucleic acids encoding any of 1 to 4; wherein the antigens can include PSMA and at least one tumor-associated antigen. In a preferred embodiment, the tumor-associated antigen can be seleced from SSX-2 and NY-ESO-1, for example. The compositions can further include at least one antigen' associated with tumor neovasculature. The antigen associated with tumor neovasculature can be, for example, VEGFR2 and Tie-2.

The present disclosure also relates to immunogenic compositions for the treatment of or for inducing a T cell response against non-small cell lung cancer. The compositions can include, for example, individually or in combination, 1) whole antigens, 2) fragments of antigens, 3) epitope clusters derived from antigens, 4) epitopes derived from antigens, or 5) nucleic acids encoding any of 1 to 4; wherein the antigens can include PSMA and at least one tumor-associated antigen, including, for example, MAGE-3, SSX-2 and NY-ESO-1. The compositions can further include at least one antigen associated with tumor neovasculature. The antigen associated with tumor neovasculature can be, for example, VEGFR2 and Tie-2.

Also the present disclosure relates to immunogenic compositions for the treatment of or for inducing a T cell response against renal cell carcinoma. The compositions can include, for example, individually or in combination, 1) whole antigens, 2) fragments of antigens, 3) epitope clusters derived from antigens, 4) epitopes derived from antigens, or 5) nucleic acids encoding any of 1 to 4; wherein the antigens can include PSMA and at least one tumor-associated antigen. In a preferred embodiment the tumor associated antigen can be PRAME or SSX-2. The compositions can further include at least one antigen associated with tumor neovasculature. The antigen associated with tumor neovasculature can be, for example, VEGFR2 and Tie-2.

The present disclosure further relates to an immunogenic composition for the treatment of or for inducing a T cell response against melanoma. The composition can include, for example, individually or in combination, 1) whole antigens, 2) fragments of antigens, 3) epitope clusters derived from antigens, 4) epitopes derived from antigens, or 5) nucleic acids encoding any of 1 to 4; wherein the antigens can be selected from each of two groups; wherein the first group includes, for example, tyrosinase, and melan-A; and wherein the second group includes SSX-2 and NY-ESO-1, and the like. The compositions can further include at least one antigen associated with tumor neovasculature. The antigen associated with tumor neovasculature can be, for example, PSMA, VEGFR2 and Tie-2.

Further embodiments relate to compositions and methods for inducing a CTL response using combinations of tumor-associated antigens, including fragments of tumor-associated antigens, clusters and epitopes. The compositons can include nucleic acid constructs, for example, a single construct that encodes all of the desired antigens. In other embodiments a single construct encodes a single antigen, while in other embodiments one construct can have a combination of epitopes with similar immunogenicity and another construct can have epitopes with similar immunogenicity.

Still other embodiments relate to methods of designing and preparing immunogenic compositions, which methods can include the steps of determining the presence of one or more antigens on a tumor type, and obtaining the one or more antigens for inclusion in a composition that induces CTL.

### Brief Description of the Drawings

Figure 1 shows the schedule of immunization with two plasmid (pCBP expressing SSX2 41-49 and pSEM expressing Melan A).

Figure 2 is a bar graph that shows CTL activity obtained using the protocol in Figure 1.

Figure 3 shows the schedule of immunization of an entrain-and-amplify immunization protocol using plasmids and peptides representing two epitopes.

Figure 4 is a table showing *in vivo* clearance of epitope-pulsed cells in mice immunized according to the protocol of Figure 3.

Figure 5 shows the preferred immunization protocols for inducing multivalent responses.

### Detailed Description of the Preferred Embodiment

The frequency of expression of many tumor-associated antigens (TuAAs) in various types of cancers is known. However, the frequency of appearance of some antigens, and especially certain combinations of TuAAs in various types of cancers has not been reported. Accurate measurement of the presence of TuAAs in tumor tissues aids in determining which TuAAs will be useful for the treatment of a particular type of cancer.

Many attempts to develop active immunotherapies for cancer have utilized a single antigen. This can be problematic for two distinct reasons. Firstly, the expression of any particular TuAA in cancer can be mosaic with the antigen expression ranging from high in some cells within a tumor mass to completely absent in others. Moreover, the TuAA may be expressed in some lesions but not others. By directing an immune response against more than a single antigen, if properly selected, the number of tumor cells that can be recognized can be maximized. Secondly, it has been observed in some cases that tumors lose expression of a TuAA following immunization, giving rise to a resistant population. If the immune response is directed against more than one TuAA it becomes much more difficult for a resistant tumor to arise because it must then simultaneously lose expression of each of the antigens in order to escape. Thus, in treating cancer with immunotherapy, it can be advantageous to use a combination of TuAAs both due to more complete coverage of the population of tumor cells, and because there will be less chance of tumor escape through antigen loss of expression of the TuAAs, provided the tumor is positive for at least two of the TuAAs used.

### Definitions:

Unless otherwise clear from the context of the use of a term herein, the following listed terms shall generally have the indicated meanings for purposes of this description.

PROFESSIONAL ANTIGEN-PRESENTING CELL (pAPC) - a cell that possesses T cell costimulatory molecules and is able to induce a T cell response. Well characterized pAPCs include dendritic cells, B cells, and macrophages.

PERIPHERAL CELL - a cell that is not a pAPC.

HOUSEKEEPING PROTEASOME - a proteasome normally active in peripheral cells, and generally not present or not strongly active in pAPCs.

IMMUNOPROTEASOME - a proteasome normally active in pAPCs; the immunoproteasome is also active in some peripheral cells in infected tissues or following exposure to interferon.

EPITOPE - a molecule or substance capable of stimulating an immune response. In preferred embodiments, epitopes according to this definition include but are not necessarily limited to a polypeptide and a nucleic acid encoding a polypeptide, wherein the polypeptide is capable of stimulating an immune response. In other preferred embodiments, epitopes according to this definition include but are not necessarily limited to peptide presented on the surface of cells, the peptides being non-covalently bound to the binding cleft of class I MHC, such that they can interact with T cell receptors (TCR). Epitopes presented by class I MHC may be in immature or mature form. "Mature" refers to an MHC epitope in distinction to any precursor ("immature") that may include or consist essentially of a housekeeping epitope, but also includes other sequences in a primary translation product that are removed by processing, including without limitation, alone or in any combination, proteasomal digestion, N-terminal trimming, or the action of exogenous enzymatic activities. Thus, a mature epitope may be provided embedded in a somewhat longer polypeptide, the immunological potential of which is due, at least in part, to the embedded epitope; likewise, the mature epitope can be provided in its ultimate form that can bind in the MHC binding cleft to be recognized by TCR.

MHC EPITOPE - a polypeptide having a known or predicted binding affinity for a mammalian class I or class II major histocompatibility complex (MHC) molecule.

HOUSEKEEPING EPITOPE - In a preferred embodiment, a housekeeping epitope is defined as a polypeptide fragment that is an MHC epitope, and that is displayed on a cell in which housekeeping proteasomes are predominantly active. In another preferred embodiment, a housekeeping epitope is defined as a polypeptide containing a housekeeping epitope according to the foregoing definition, that is flanked by one to several additional amino acids. In another preferred embodiment, a housekeeping epitope is defined as a nucleic acid that encodes a housekeeping epitope according to the foregoing definitions. Exemplary housekeeping epitopes are provided in U.S. Application Nos. 10/117,937, filed on April 4, 2002 (Pub. No. 20030220239 A1), and 10/657,022, and in PCT Application No. PCT/US2003/027706 (Pub. No. WO04022709A2), filed 9/5/2003; and U.S. Provisional Application Nos. 60/282,211, filed on April 6, 2001; 60/337,017, filed on November 7, 2001; 60/363210 filed 3/7/02; and 60/409,123, filed on September 5, 2002. Each of the listed applications is entitled EPITOPE SEQUENCES.

IMMUNE EPITOPE - In a preferred embodiment, an immune epitope is defined as a polypeptide fragment that is an MHC epitope, and that is displayed on a cell in which immunoproteasomes are predominantly active. In another preferred embodiment, an immune epitope is defined as a polypeptide containing an immune epitope according to the foregoing definition, that is flanked by one to several additional amino acids. In another preferred embodiment, an immune epitope is defined as a polypeptide including an epitope cluster sequence, having at least two polypeptide sequences having a known or predicted affinity for a class I MHC. In yet another preferred embodiment, an immune epitope is defined as a nucleic acid that encodes an immune epitope according to any of the foregoing definitions.

TARGET CELL - In a preferred embodiment, a target cells is a cell associated with a pathogenic condition that can be acted upon by the components of the immune system, for example, a cell infected with a virus or other intracellular parasite, or a neoplastic cell. In another embodiment, a target cell is a cell to be targeted by the vaccines and methods of the invention. Examples of target cells according to this definition include but are not necessarily limited to: a neoplastic cell and a cell harboring an intracellular parasite, such as, for example, a virus, a bacterium, or a protozoan. Target cells can also include cells that are targeted by CTL as a part of an assay to determine or confirm proper epitope liberation and processing by a cell expressing immunoproteasome, to determine T cell specificity or immunogenicity for a desired epitope. Such cells can be transformed to express the liberation sequence, or the cells can simply be pulsed with peptide/epitope.

TARGET-ASSOCIATED ANTIGEN (TAA) - a protein or polypeptide present in a target cell.

TUMOR-ASSOCIATED ANTIGENS (TuAA) - a TAA, wherein the target cell is a neoplastic cell.

HLA EPITOPE - a polypeptide having a known or predicted binding affinity for a human class I or class II HLA complex molecule.

ANTIBODY - a natural immunoglobulin (Ig), poly- or monoclonal, or any molecule composed in whole or in part of an Ig binding domain, whether derived biochemically, or by use of recombinant DNA, or by any other means. Examples include *inter alia*, F(ab), single chain Fv, and Ig variable region-phage coat protein fusions.

SUBSTANTIAL SIMILARITY - this term is used to refer to sequences that differ from a reference sequence in an inconsequential way as judged by examination of the sequence. Nucleic acid sequences encoding the same amino acid sequence are substantially similar despite differences in degenerate positions or minor differences in length or composition of any non-coding regions. Amino acid sequences differing only by conservative substitution or minor length variations are substantially similar. Additionally, amino acid sequences comprising housekeeping epitopes that differ in the number of N-terminal flanking residues, or immune epitopes and epitope clusters that differ in the number of flanking residues at either terminus, are substantially similar. Nucleic acids that encode substantially similar amino acid sequences are themselves also substantially similar.

FUNCTIONAL SIMILARITY - this term is used to refer to sequences that differ from a reference sequence in an inconsequential way as judged by examination of a biological or biochemical property, although the sequences may not be substantially similar. For example, two nucleic acids can be useful as hybridization probes for the same sequence but encode differing amino acid sequences. Two peptides that induce cross-reactive CTL responses are functionally similar even if they differ by non-conservative amino acid substitutions (and thus may not be within the substantial similarity definition). Pairs of antibodies, or TCRs, that recognize the same epitope can be functionally similar to each other despite whatever structural differences exist. Testing for functional similarity of immunogenicity can be conducted by immunizing with the "altered" antigen and testing the ability of an elicited response, including but not limited to an antibody response, a CTL response, cytokine production, and the like, to recognize the target antigen. Accordingly, two sequences may be designed to differ in certain respects while retaining the same function. Such designed sequence variants of disclosed or claimed sequences are among the embodiments of the present invention.

EXPRESSION CASSETTE - a polynucleotide sequence encoding a polypeptide, operably linked to a promoter and other transcription and translation control elements, including but not limited to enhancers, termination codons, internal ribosome entry sites, and polyadenylation sites. The cassette can also include sequences that facilitate moving it from one host molecule to another.

EMBEDDED EPITOPE - in some embodiments, an embedded epitope is an epitope that is wholly contained within a longer polypeptide; in other embodiments, the term also can include an epitope in which only the N-terminus or the C-terminus is embedded such that the epitope is not wholly in an interior position with respect to the longer polypeptide.

MATURE EPITOPE - a peptide with no additional sequence beyond that present when the epitope is bound in the MHC peptide-binding cleft.

EPITOPE CLUSTER - a polypeptide, or a nucleic acid sequence encoding it, that is a segment of a protein sequence, including a native protein sequence, comprising two or more known or predicted epitopes with binding affinity for a shared MHC restriction element In preferred embodiments, the density of epitopes within the cluster is greater than the density of all known or predicted epitopes with binding affinity for the shared MHC restriction element within the complete protein sequence. Epitope clusters are disclosed and more fully defined in U.S. Patent Application No. 09/561,571 entitled EPITOPE CLUSTERS.

LIBERATION SEQUENCE - a designed or engineered sequence comprising or encoding a housekeeping epitope embedded in a larger sequence that provides a context allowing the housekeeping epitope to be liberated by processing activities including, for example, immunoproteasome activity, N terminal trimming, and/or other processes or activities, alone or in any combination.

CTLp - CTL precursors are T cells that can be induced to exhibit cytolytic activity. Secondary *in vitro* lytic activity, by which CTLp are generally observed, can arise from any combination of naive, effector, and memory CTL *in vivo*.

MEMORY T CELL - A T cell, regardless of its location in the body, that has been previously activated by antigen, but is in a quiescent physiologic state requiring re-exposure to antigen in order to gain effector function. Phenotypically they are generally CD62L⁻ CD44^{hi} CD107α⁻ IGN-γ⁻ LTβ⁻ TNF-α⁻ and is in G0 of the cell cycle.

EFFECTOR T CELL - A T cell that, upon encountering antigen, readily exhibits effector function. Effector T cells are generally capable of exiting the lymphatic system and entering the immunological periphery. Phenotypically they are generally CD62L⁻ CD44^{hi} CD107α⁺ IGN-γ⁺ LTβ⁺ TNF-α⁺ and actively cycling.

EFFECTOR FUNCTION - Generally, T cell activation generally, including acquisition of cytolytic activity and/or cytokine secretion.

INDUCING a T cell response - Includes in many embodiments the process of generating a T cell response from naive, or in some contexts, quiescent cells; activating T cells.

AMPLIFYING a T cell response - Includes in many embodiments the process or increasing the number of cells, the number of activated cells, the level of activity, rate of proliferation, or similar parameter ofT cells involved in a specific response.

ENTRAINMENT - Includes in many embodiments an induction that confers particular stability on the immune profile of the induced lineage of T cells.

TOLL-LIKE RECEPTOR (TLR) - Toll-like receptors (TLRs) are a family of pattern recognition receptors that are activated by specific components of microbes and certain host molecules. As part of the innate immune system, they contribute to the first line of defense against many pathogens, but also play a role in adaptive immunity.

TOLL-LIKE RECEPTOR (TLR) LIGAND - Any molecule capable of binding and activating a toll-like receptor. Examples include, without limitation: poly IC A synthetic, double-stranded RNA know for inducing interferon. The polymer is made of one strand each of polyinosinic acid and polycytidylic acid, double-stranded RNA, unmethylated CpG oligodeoxyribonucleotide or other immunostimulatory sequences (ISSs), lipopolysacharide (LPS), β-glucans, and imidazoquinolines, as well as derivatives and analogues thereof.

IMMUNOPOTENTIATING ADJUVANTS - Adjuvants that activate pAPC or T cells including, for example: TLR ligands, endocytic-Pattern Recognition Receptor (PRR) ligands, quillaja saponins, tucaresol, cytokines, and the like. Some preferred adjuvants are disclosed in Marciani, D.J. Drug Discovery Today 8:934-943, 2003.

IMMUNOSTIMULATORY SEQUENCE (ISS) - Generally an oligodeoxyribonucleotide containing an unmethlylated CpG sequence. The CpG may also be embedded in bacterially produced DNA, particularly plasmids. Further embodiments include various analogues; among preferred embodiments are molecules with one or more phosphorothioate bonds or non-physiologic bases.

VACCINE - In preferred embodiments a vaccine can be an immunogenic composition providing or aiding in prevention of disease. In other embodiments, a vaccine is a composition that can provide or aid in a cure of a disease. In others, a vaccine composition can provide or aid in amelioration of a disease. Further embodiments of a vaccine immunogenic composition can be used as therapeutic and/or prophylactic agents.

IMMUNIZATION - a process to induce partial or complete protection against a disease. Alternatively, a process to induce or amplify an immune system response to an antigen. In the second definition it can connote a protective immune response, particularly proinflammatory or active immunity, but can also include a regulatory response. Thus in some embodiments immunization is distinguished from tolerization (a process by which the immune system avoids producing proinflammatory or active immunity) while in other embodiments this term includes tolerization.

ENCODE - an open-ended term such that a nucleic acid encoding a particular amino acid sequence can consist of codons specifying that (poly)peptide, but can also comprise additional sequences either translatable, or for the control of transcription, translation, or replication, or to facilitate manipulation of some host nucleic acid construct.

COVERAGE - the fraction or proportion of tumor cells expressing a particular TuAA or at least one TuAA from a set of selected TuAAs.

REDUNDANCY - the degree to which a population of tumor cells, or some subset of them, express more than one of a selected set of TuAAs.

### Tumor Associated Antigens

Examples of TuAAs useful in embodiments disclosed herein include tyrosinase (SEQ. ID NO. 1), melan-A, (SEQ. ID NO. 2), SSX-2, (SEQ. ID NO.3), PSMA (prostate-specific membrane antigen) (SEQ. ID NO. 4), MAGE-1, (SEQ. ID NO. 5), MAGE-3 (SEQ. ID NO. 6), NY-ESO-1, (SEQ. ID NO. 7), PRAME, (SEQ. ID NO.8), and Her2/Neu (SEQ. ID NO. 9). The natural coding sequences for these nine proteins, or any segments within them, can be determined from their cDNA or complete coding (cds) sequences, SEQ. ID NOS. 10-18, respectively.

**Table 1. SEQ. ID NOS.**

| **SEQ. ID NO.** | **IDENTITY** | **ACCESSION NUMBER**** |
|---|---|---|
| 1 | Tyrosinase protein | P14679 |
| 2 | Melan-A protein | Q16655 |
| 3 | SSX-2 protein | NP_003138 |
| 4 | PSMA protein | NP_004467 |
| 5 | MAGE-1 protein | P43355 |
| 6 | MAGE-3 protein | P43357 |
| 7 | NY-ESO-1 protein | P78358 |
| 8 | PRAME protein | NP 006106 |
| 9 | Her2/Neu protein | P04626 |
| 10 | Tyrosinase cDNA | NM_000372 |
| 11 | Melan-A cDNA | U06452 |
| 12 | SSX-2 cDNA | NM_003147 |
| 13 | PSMA cDNA | NM_004476 |
| 14 | MAGE-1 cds | M77481 |
| 15 | MAGE-3 cds | U03735 |
| 16 | NY-ESO-1 cDNA | U87459 |
| 17 | PRAME cDNA | NM_006115 |
| 18 | Her2/Neu cDNA | M11730 |

| | | |
|---|---|---|
| **All accession numbers used here and throughout can be accessed through the NCBI databases, for example, through the Entrez seek and retrieval system on the world wide web. | | |

Tyrosinase is a melanin biosynthetic enzyme that is considered one of the most specific markers ofmelanocytic differentiation. Tyrosinase is expressed in few cell types, primarily in melanocytes, and high levels are often found in melanomas. The usefulness of tyrosinase as a TuAA is taught in U.S. Patent 5,747,271 entitled "METHOD FOR IDENTIFYING INDIVIDUALS SUFFERING FROM A CELLULAR ABNORMALITY SOME OF WHOSE ABNORMAL CELLS PRESENT COMPLEXES OF HLA-A2/TYROSINASE DERIVED PEPTIDES, AND METHODS FOR TREATING SAID INDIVIDUALS".

GP100, also known as PMe117, also is a melanin biosynthetic protein expressed at high levels in melanomas. GP100 as a TuAA is disclosed in U.S. Patent 5,844,075 entitled "MELANOMA ANTIGENS AND THEIR USE IN DIAGNOSTIC AND THERAPEUTIC METHODS,".

Melan-A, also called MART-1 (Melanoma Antigen Recognized by T cells), is another melanin biosynthetic protein expressed at high levels in melanomas. The usefulness of Melan-A/MART-1 as a TuAA is taught in U.S. Patent Nos. 5,874,560 and 5,994,523 both entitiled "MELANOMA ANTIGENS AND THEIR USE IN DIAGNOSTIC AND THERAPEUTIC METHODS," as well as U.S. Patent No. 5,620,886, entitled "ISOLATED NUCLEIC ACID SEQUENCE CODING FOR A TUMOR REJECTION ANTIGEN PRECURSOR PROCESSED TO AT LEAST ONE TUMOR REJECTION ANTIGEN PRESENTED BY HLA-A2".

SSX-2, also know as Hom-Mel-40, is a member of a family of highly conserved cancer-testis antigens (Gure, A.O. et al. Int. J. Cancer 72:965-971, 1997). Its identification as a TuAA is taught in U.S. Patent 6,025,191 entitled "ISOLATED NUCLEIC ACID MOLECULES WHICH ENCODE A MELANOMA SPECIFIC ANTIGEN AND USES THEREOF,". Cancer-testis antigens are found in a variety of tumors, but are generally absent from normal adult tissues except testis. Expression of different members of the SSX family has been found in various tumor cell lines. Due to the high degree of sequence identity among SSX family members, similar epitopes from more than one member of the family will be generated and able to bind to an MHC molecule, so that some vaccines directed against one member of this family can cross-react and be effective against other members of this family.

MAGE-1 (melanoma-associated antigen-1), MAGE-2 (melanoma-associated antigen-2), and MAGE-3 (melanoma-associated antigen-3) are members of another family of cancer-testis antigens originally discovered in melanoma but found in a variety of tumors. The identification of MAGE proteins as TuAAs is taught in U.S. Patent 5,342,774 entitled NUCLEOTIDE SEQUENCE ENCODING THE TUMOR REJECTION ANTIGEN PRECURSOR, MAGE-1, and in numerous subsequent patents. Currently there are 17 entries for (human) MAGE in the SWISS Protein database. There is extensive similarity among these proteins, such that in many cases, an epitope from one can induce a cross-reactive response to other members of the family. A few members of the MAGE family have not been observed in tumors, most notably MAGE-H1 and MAGE-D1, which are expressed in testes and brain, and bone marrow stromal cells, respectively. The possibility of cross-reactivity on normal tissue is ameliorated by the fact that they are among the least similar to the other MAGE proteins.

GAGE-1 is a member of the GAGE family of cancer testis antigens (Van den Eynde, B., et al., J. Exp. Med. 182: 689-698, 1995; U.S Patent Nos. 5,610,013; 5648226; 5,858,689; 6,013,481; and 6,069,001). The PubGene database currently lists 12 distinct accessible members, some of which are synonymously known as PAGE or XAGE. GAGE-1 through GAGE-8 have a very high degree of sequence identity, so most epitopes can be shared among multiple members of the family.

BAGE is a cancer-testis antigen commonly expressed in melanoma, particularly metastatic melanoma, as well as in carcinomas of the lung, breast, bladder, and squamous cells of the head and neck. Its usefulness as a TuAA is taught in U.S. Patent Nos. 5,683,88 entiltled "TUMOR REJECTION ANTIGENS WHICH CORRESPOND TO AMINO ACID SEQUENCES IN TUMOR REJECTION ANTIGEN PRECURSOR BAGE, AND USES THEREOF' and 5,571,711 entitled "ISOLATED NUCLEIC ACID MOLECULES CODING FOR BAGE TUMOR REJECTION ANTIGEN PRECURSOR," each.

NY-ESO-1, also known as CTAG-1 (Cancer-Testis Antigen-1) and CAG-3 (Cancer Antigen-3), is a cancer-testis antigen found in a wide variety of tumors. NY-ESO-1 as a TuAA is disclosed in U.S. Patent 5,804,381 entitled ISOLATED NUCLEIC ACID MOLECULE ENCODING AN ESOPHAGEAL CANCER ASSOCIATED ANTIGEN, THE ANTIGEN ITSELF, AND USES THEREOF. A paralogous locus encoding antigens with extensive sequence identity, LAGE-1a/s and LAGE-1b/L, has been disclosed in publicly available assemblies of the human genome, and has been concluded to arise through alternate splicing. Additionally, CT-2 (or CTAG-2, Cancer-Testis Antigen-2) appears to be either an allele, a mutant, or a sequencing discrepancy of LAGE-1b/L. Due to the extensive sequence identity, many epitopes from NY-ESO-1 can also induce immunity to tumors expressing these other antigens. NY-ESO-1 and LAGE are virtually identical through amino acid 70. From amino acid 71 through 134 the longest run of identity between the two proteins is 6 residues, but potentially cross-reactive sequences are present. From amino acid 135 through 180, NY-ESO and LAGE-1a/s are identical except for a single residue, but LAGE-1b/L is unrelated due to the alternate splice. The CAMEL and LAGE-2 antigens appear to derive from the LAGE-1 mRNA, but from alternate reading frames, thus giving rise to unrelated protein sequences. More recently, GenBank Accession AF277315.5, Homo sapiens chromosome X clone RP5-865E18, RP5-1087L19, complete sequence, reports three independent loci in this region which are labeled as LAGE1 (corresponding to STAG-2 in the genome assemblies), LAGE2-A and LAGE2-B (both corresponding to CTAG-1 in the genome assemblies).

PRAME, also know as MAPE, DAGE, and OIP4, was originally observed as a melanoma antigen. Subsequently, it has been recognized as a cancer-testis (CT) antigen, but unlike many CT antigens, such as, MAGE, GAGE and BAGE, PRAME is expressed in acute myeloid leukemias. PRAME is a member of the MAPE family, which consists largely of hypothetical proteins with which it shares limited sequence similarity. The usefulness of PRAME as a TuAA is taught in U.S. Patent 5,830,753 entitled "ISOLATED NUCLEIC ACID MOLECULES CODING FOR TUMOR REJECTION ANTIGEN PRECURSOR DAGE AND USES THEREOF,".

PSMA (prostate-specific membranes antigen), a TuAA described in U.S. Patent 5,538,866 entitled "PROSTATE-SPECIFIC MEMBRANES ANTIGENS", is expressed by normal prostate epithelium and, at a higher level, in prostatic cancer. It has also been found in the neovasculature of non-prostatic tumors. PSMA can thus form the basis for vaccines directed to both prostate cancer and to the neovasculature of other tumors. This later concept is more fully described in a provisional U.S. Patent Application No. 60/274,063 entitled "ANTI-NEOVASCULAR VACCINES FOR CANCER," fled March 7, 2001, and U.S. Application No. 10/094,699 (Pub. No. 20030046714 A1), filed on March 7, 2002, entitled "ANTI-NEOVASCULAR PREPARATIONS FOR CANCER,". Briefly, as tumors grow they recruit ingrowth of new blood vessels. This is understood to be necessary to sustain growth as the centers of unvascularized tumors are generally necrotic and angiogenesis inhibitors have been reported to cause tumor regression. Such new blood vessels, or neovasculature, express antigens not found in established vessels, and thus can be specifically targeted. By inducing CTL against neovascular antigens the vessels can be disrupted, interrupting the flow of nutrients to, and removal of wastes from, tumors, leading to regression.

Alternate splicing of the PSMA mRNA leads to a protein with an apparent start at Met₅₈, thereby deleting the putative membrane anchor region of PSMA as described in U.S. Patent 5,935,818 entitled "ISOLATED NUCLEIC ACID MOLECULE ENCODING ALTERNATIVELY SPLICED PROSTATE-SPECIFIC MEMBRANES ANTIGEN AND USES THEREOF,". A protein termed PSMA-like protein, Genbank accession number AF261715, is nearly identical to amino acids 309-750 of PSMA, but has a different expression profile. Thus the most preferred epitopes are those with an N-terminus located from amino acid 58 to 308.

PSA, prostate specific antigen, is a peptidase of the kallikrein family and a differentiation antigen of the prostate. Expression in breast tissue has also been reported. Alternate names include gamma-seminoprotein, kallikrein 3, seminogelase, seminin, and P-30 antigen. PSA has a high degree of sequence identity with the various alternate splicing products prostatic/glandular kallikrein-1 and -2, as well as kalikrein 4, which is also expressed in prostate and breast tissue. Other kallikreins generally share less sequence identity and have different expression profiles. Nonetheless, cross-reactivity that might be provoked by any particular epitope, along with the likelihood that that epitope would be liberated by processing in non-target tissues (most generally by the housekeeping proteasome), should be considered in designing a vaccine.

PSCA, prostate stem cell antigen, and also known as SCAH-2, is a differentiation antigen preferentially expressed in prostate epithelial cells, and overexpresssed in prostate cancers. Lower level expression is seen in some normal tissues including neuroendocrine cells of the digestive tract and collecting ducts of the kidney. PSCA is described in U.S. Patent 5,856,136 entitled "HUMAN STEM CELL ANTIGENS,".

Synaptonemal complex protein 1 (SCP-1), also known as HOM-TES-14, is a meiosis-associated protein and also a cancer-testis antigen (Tureci, O., et al. Proc. Natl. Acad. Sci. USA 95:5211-5216, 1998). As a cancer antigen its expression is not cell-cycle regulated and it is found frequently in gliomas, breast, renal cell, and ovarian carcinomas. It has some similarity to myosins, but with few enough identities that cross-reactive epitopes are not an immediate prospect.

The ED-B domain of fibronectin is also a potential target. Fibronectin is subject to developmentally regulated alternative splicing, with the ED-B domain being encoded by a single exon that is used primarily in oncofetal tissues (Matsuura, H. and S. Hakomori Proc. Natl. Acad. Sci. USA 82:6517-6521, 1985; Camemolla, B. et al. J. Cell Biol. 108:1139-1148, 1989; Loridon-Rosa, B. et al. Cancer Res.50:1608-1612, 1990; Nicolo, G. et al. Cell Differ. Dev. 32:401-408, 1990; Borsi, L. et al. Exp. Cell Res. 199:98-105, 1992; Oyama, F. et al. Cancer Res. 53:2005-2011, 1993; Mandel, U. et al. APMIS 102:695-702, 1994; Farnoud, M.R. et al. Int. J. Cancer 61:27-34, 1995; Pujuguet, P. et al. Am. J. Pathol. 148:579-592, 1996; Gabler, U. et al. Heart 75:358-362, 1996;Chevalier, X Br. J. Rheumatol. 35:407-415, 1996; Midulla, M. Cancer Res. 60:164-169, 2000).

The ED-B domain is also expressed in fibronectin of the neovasculature (Kaczmarek, J. et al. Int. J. Cancer 59:11-16, 1994; Castellani, P. et al. Int. J. Cancer 59:612-618, 1994; Neri, D. et al. Nat. Biotech. 15:1271-1275, 1997; Karelina, T.V. and A.Z. Eisen Cancer Detect. Prev. 22:438-444, 1998; Tarli, L. et al. Blood 94:192-198, 1999; Castellani, P. et al. Acta Neurochir. (Wien) 142:277-282, 2000). As an oncofetal domain, the ED-B domain is commonly found in the fibronectin expressed by neoplastic cells in addition to being expressed by the neovasculature. Thus, CTL-inducing vaccines targeting the ED-B domain can exhibit two mechanisms of action: direct lysis of tumor cells, and disruption of the tumor's blood supply through destruction of the tumor-associated neovasculature. As CTL activity can decay rapidly after withdrawal of vaccine, interference with normal angiogenesis can be minimal. The design and testing of vaccines targeted to neovasculature is described in Provisional U.S. Patent Application No. 60/274,063 entitled "ANTI-NEOVASCULATURE VACCINES FOR CANCER," filed on March 7, 2001, and in U.S. Patent Application No. 10/094,699, (Pub. No. 20030046714 A1), entitled "ANTI-NEOVASCULATURE PREPARATIONS FOR CANCER," filed on March 7, 2002. A tumor cell line is disclosed in Provisional U.S. Application No. 60/363,131, filed on March 7, 2002, entitled "HLA-TRANSGENIC MURINE TUMOR CELL LINE,".

Carcinoembryonic antigen (CEA) is a paradigmatic oncofetal protein first described in 1965 (Gold and Freedman, J. Exp. Med. 121: 439-462, 1965. Fuller references can be found in the Online Medelian Inheritance in Man; record * 114890. It has officially been renamed carcinoembryonic antigen-related cell adhesion molecule 5 (CEACAM5). Its expression is most strongly associated with adenocarcinomas of the epithelial lining of the digestive tract and in fetal colon. CEA is a member of the immunoglobulin supergene family and the defining member of the CEA subfamily.

Survivin, also known as Baculoviral IAP Repeat-Containing Protein 5 (BIRC5), is another protein with an oncofetal pattern of expression. It is a member of the inhibitor of apoptosis protein (IAP) gene family. It is widely over-expressed in cancers (Ambrosini, G. et al., Nat. Med. 3:917-921, 1997; Velculiscu V.E. et al., Nat. Genet. 23:387-388, 1999) and its function as an inhibitor of apoptosis is believed to contribute to the malignant phenotype.

HER2/NEU is an oncogene related to the epidermal growth factor receptor (van de Vijver, et al., New Eng. J. Med. 319:1239-1245, 1988), and apparently identical to the c-ERBB2 oncogene (Di Fiore, et al., Science 237: 178-182, 1987). The over-expression of ERBB2 has been implicated in the neoplastic transformation of prostate cancer. As with HER2, it is amplified and over-expressed in 25-30% of breast cancers among other tumors where expression level is correlated with the aggressiveness of the tumor (Slamon, et al., New Eng. J. Med. 344:783-792, 2001). A more detailed description is available in the Online Medelian Inheritance in Man; record * 164870.

Further examples of tumor-associated antigens include MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, ß-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, and the like.

Additional tumor-associated antigens are described in Chen, YT, "Identification of human tumor antigens by serological expression cloning: an online review on SEREX" Cancer Immun. 2004 [updated 2004 Mar 10; cited 2004 Apr 1] *at* world wide web cancerimmunotherapy.org/SEREX/; and Renkvist, N. et al., "A listing of tumor antigens recognized by T cells," Cancer Immunology Immunotherapy, 50:3-15 (2001).

Table 2, adapted from Scanlan et al., "The cancer/testis genes: Review, standardization, and commentary," Cancer Immunity 4:1 (January 23, 2004), provides a listing of CT Antigens. Table 3 provides the frequency of mRNA expression in various tumor types for the CT antigens in Table 2. Scanlan et al., "The cancer/testis genes: Review, standardization, and commentary," Cancer Immunity 4:1 (January 23, 2004).

**Table 2**

| Listing of CT genes | | |
|---|---|---|
| **CT Identifier** | **Transcript/Transcript family** | **Family Members/CT Identifier (Synonyms)** |
| CT1 | MAGEA | MAGEA1/CT1.1, MAGEA2/CT1.2, |
| | | MAGEA3/CT1.3, MAGEA4/CT1.4, |
| | | MAGEA5/CT1.5, MAGEA6/CT1.6, |
| | | MAGEA7/CT1.7, MAGEA8/CT1.8, MAGEA9/CT.9, |
| | | MAGEA10/CT1.10, MAGEA11/CT1.11, |
| | | MAGEA12/CT1.12 |
| CT2 | BAGE | BAGE/CT2.1, BAGE2/CT2.2, BAGE3/CT2.3, |
| | | BAGE4/CT2.4, BAGE5/CT2.5 |
| CT3 | MAGEB | MAGEB1/CT3.1, MAGEB2/CT3.2, MAGEB5/CT3.3, |
| | | MAGEB6/CT3.4 |
| CT4 | GAGE1 | GAGE1/CT4.1, GAGE2/CT4.2, GAGE3/CT4.3, |
| | | GAGE4/CT4.4, GAGE5/CT4.5, GAGE6/CT4.6, |
| | | GAGE7/CT4.7, GAGE8/CT4.8 |
| CT5 | SSX | SSX1/CT5.1, SSX2/CT5.2a, SSX2/CT5.2b, |
| | | SSX3/CT5.3, SSX4/CT5.4 |
| CT6 | NY-ESO-1 | NY-ESO-1/CT6.1, LAGE-1a/CT6.2a, LAGE- |
| | | 1b/CT6.2b |
| CT7 | MAGEC1 | MAGEC1/CT7.1,MAGEC3/CT7.2 |
| CT8 | SYCP1 | SYCP1/CT8 |
| CT9 | BRDT | BRDT/CT9 |
| CT10 | MAGEE1 | MAGEE1/CT10 |
| CT11 | CTp11/SPANX | SPANXA1/CT11.1, SPANXB1/CT11.2, |
| | | SPANXC/CT11.3, SPANXD/CT11.4 |
| CT12 | XAGE-1/GAGED | XAGE-1a/CT12.1a, XAGE-1b/CT12.1b, XAGE- |
| | | 1c/CT12.1c, XAGE-1d/CT12.1d, XAGE-2/CT12.2, |
| | | XAGE-3a/CT12.3a, XAGE-3b/CT12.3b, XAGE- |
| | | 4/CT12.4 |
| CT13 | HAGE | HAGE/CT13 |
| CT14 | SAGE | SAGE/CT14 |
| CT15 | ADAM2 | ADAM2/CT15 |
| CT16 | PAGE-5 | PAGE-5/CT16.1, CT16.2 |
| CT17 | LIP1 | LIP1/CT17 |
| CT18 | NA88 | NA88/CT12 |
| CT19 | IL13RA1 | IL13RA1/CT19 |
| CT20 | TSP50 | TSP50/CT20 |
| CT21 | CTAGE-1 | CTAGE-1/CT21.1, CTAGE-2/CT21.2 |
| CT22 | SPA17 | SPA17/CT22 |
| CT23 | OY-TES-1 | OY-TES-1/CT23 |
| CT24 | CSAGE | CSAGE/CT24.1, TRAG3/CT24.2 |
| CT25 | MMA1/DSCR8 | MMA-1a/CT25.1a, MMA-1b/CT25.1b |
| CT26 | CAGE | CAGE/CT26 |
| CT27 | BORIS | BORIS/CT27 |
| CT28 | HOM-TES-85 | HOM-TES-85/CT28 |
| CT29 | AF15q14/D40 | D40/CT29 |
| CT30 | E2F-like/HCA661 | HCA661/CT30 |
| CT31 | PLU-1 | PLU-1/CT31 |
| CT32 | LDHC | LDHC/CT32 |
| CT33 | MORC | MORC/CT33 |
| CT34 | SGY-1 | SGY-1/CT34 |
| CT35 | SPO11 | SPO11/CT35 |
| CT36 | TPX1 | TPX-1/CT36 |
| CT37 | NY-SAR-35 | NY-SAR-35/CT37 |
| CT38 | FTHL17 | FTHL17/CT38 |
| CT39 | NXF2 | NXF2/CT39 |
| CT40 | TAF7L | TAF7L/CT40 |
| CT41 | TDRD1 | TDRD1/CT41.1,NY-CO-45/CT41.2 |
| CT42 | TEX15 | TEX15/CT42 |
| CT43 | FATE | FATE/CT43 |
| CT44 | TPTE | TPTE/CT44 |
| --- | PRAME | (MAPE, DAGE) |

**Table 3.**

| **CT Family (Member)** | **Frequency (%) of Expression in Tumor Type** | | | | | | | | | | | | | | | | **Ref** |
|---|---|---|---|---|---|---|---|---|---|---|---|---|---|---|---|---|---|
| | **Blad** | **Brn** | **Brst** | **Col.** | **Eso** | **Gas** | **H/N** | **Liver** | **Leuk/Lymph** | **Lung (NSCLC)** | **Mel** | **Ov** | **Pancr** | **Pros** | **Renal** | **Sarc** | |
| MAGEA1/CT1.1 | 22 | - | 18 | 2 | 53 | 29 | 28 | 80 | 0 | 49 | 48 | 28 | - | 15 | 0 | 14 | 44 |
| BAGE1/CT2.1 | 15 | - | 10 | 0 | - | - | 8 | - | 0 | 4 | 26 | 15 | - | 0 | 0 | 6 | 44 |
| MAGEB1/CT3.1 | 0 | 0 | 17 | 0 | - | 0 | 0 | - | 0 | 14 | 22 | - | - | 0 | 0 | 9 | 45 |
| GAGE/CT4.1 | 12 | - | 9 | 0 | - | - | 19 | 38^{b} | 1 | 19 | 28 | 31 | - | 10 | 0 | 25 | 44 |
| SSX2/CT5.2 | 44 | 6 | 7 | 12 | - | - | 35 | 9^{b} | 36 | 16 | 35 | - | - | 40 | 5 | 50 | 46 |
| NY-ESO-1/CT6.1 | 80 | 0 | 30 | 0 | - | 0 | | 29 | 0 | 17 | 34 | 25 | 0 | 25 | 9 | 0 | 8 |
| MAGEC1/CT7.1 | 44 | - | 30 | 10 | - | - | 36 | - | - | 33 | 70 | - | - | - | - | 60 | 20 |
| SYCP1/CT8 | - | 47 | 20 | 0 | - | 7 | - | 28^{b} | 0 | 7 | 14 | 0 | - | 0 | 8 | 0 | 2 |
| BRDT/CT9 | 0 | - | 0 | 0 | 8 | - | 8 | - | - | 25 | 0 | - | - | - | 0 | - | 16 |
| MAGEE1/CT10 | 44 | - | 38 | 0 | - | - | 36 | - | - | 24 | 50 | - | - | - | - | 0 | 12 |
| SPANXC/CT11.3 | 9 | - | 25 | 22 | 0 | - | - | - | - | 33 | 70 | - | 0 | - | - | - | 14 |
| XAGE-1a/CT12.1a | - | - | - | - | - | - | - | - | - | - | 8 | - | - | - | - | 22 | 47 |
| HAGE/CT13 | 24 | 37 | 5 | 31 | 27 | - | - | 20 | 9 | 32 | 17 | - | - | 22 | 6 | 20 | 13 |
| SAGE/CT14 | 12 | 0 | 5 | 0 | 20 | - | 17 | - | 4 | 22 | 4 | - | - | 0 | 5 | 5 | 13 |
| ADAM2/CT15 | - | - | 0 | 0 | - | - | - | - | - | 0 | 0 | 0 | - | - | 12 | - | 17 |
| PAGE-5/CT16 | - | - | 5 | 11 | - | - | - | - | - | 39 | 22 | 0 | - | - | 44 | - | 17 |
| UP1/CT17 | - | - | 5 | 0 | - | - | - | - | - | 0 | 0 | 0 | - | - | 25 | - | 17 |
| NA88/CT18 | - | - | - | - | - | - | - | - | - | - | 11 | - | - | - | - | - | 48 |
| TSP50/CT20 | - | - | 28 | - | - | - | - | - | - | - | - | - | - | - | - | - | 49 |
| CTAGE-1/CT21.1 | - | - | - | - | - | - | - | - | 35 | - | | - | - | - | - | - | 50 |
| SPA17/CT22 | - | - | - | - | - | - | - | - | 26 | - | - | - | - | - | - | - | 51 |
| OYTES1/CT23 | 28 | - | 40 | 15 | - | 0 | - | 40 | - | 20 | - | - | - | - | 0 | - | 52 |
| MMA1a/CT25.1a | - | - | 0 | 0 | 0 | - | - | - | - | 40 | 26 | - | 0 | - | - | 18 | 15 |
| CAGE/CT26 | - | - | - | - | - | 89 | - | - | - | 100 | - | - | - | - | - | - | 53 |
| HOMTES85/CT28 | - | 35 | 0 | 10 | - | | - | 19 | - | 28 | 36 | 32 | - | 0 | - | - | 54 |
| D40/CTZ9 | - | 20 | - | 13 | - | 0 | - | - | - | 41 | - | 36 | 27 | - | - | - | 55 |
| HCA661/CT30 | 0 | - | - | - | - | 0 | 0 | 29 | - | - | 20 | - | - | - | - | - | 56 |
| PLU-1/CT31 | - | - | 86 | - | - | - | - | - | - | - | - | - | - | - | - | - | 27 |
| LDHC/CT32 | - | - | 35 | 15 | - | - | - | - | - | 47 | 44 | 42 | - | 37 | 57 | - | 18 |
| MORC/CT33 | - | - | 0 | 0 | - | - | - | - | - | 18 | 18 | 14 | - | 0 | 0 | - | 18 |
| SGY-1/CT34 | - | - | 20 | 0 | - | - | - | - | - | 12 | 25 | 57 | - | 12 | 0 | - | 18 |
| SPO11/CT35 | - | - | 0 | 0 | - | - | - | - | - | 0 | 6 | 0 | - | 0 | 0 | - | 18 |
| TPX1/CT36 | - | - | 15 | 0 | - | - | - | - | - | - | 6 | 14 | - | 37 | 14 | - | 18 |
| NYSAR35/CT37 | 42 | - | 23 | 0 | 8 | - | - | - | - | 17 | 6 | 8 | - | - | 0 | 8 | 57 |
| FTHL17/CT38 | 22 | - | 14 | 0 | 0 | - | 10 | - | 0 | 25 | 0 | - | - | 0 | 0 | 0 | 58 |
| NXF2/CT39 | 19 | - | 0 | 11 | 12 | - | 5 | - | 0 | 15 | 55 | - | - | 14 | 0 | 27 | 58 |
| TAF7L/CT40 | 10 | - | 0 | 0 | 0 | - | 10 | - | 0 | 9 | 21 | - | - | 0 | 0 | 12 | 58 |
| TDRD1/CT41.1 | 28 | - | 37 | 0 | 10 | - | 22 | - | 5 | 5 | 0 | - | - | 38 | 0 | 0 | 58 |
| TEX15/CT42 | 21 | - | 0 | 0 | 20 | - | 11 | - | 0 | 21 | 27 | - | - | 12 | 33 | 28 | 58 |
| FATE/CT43 | - | - | - | 21 | - | 7 | - | 66 | - | 0 | - | - | - | - | - | - | 19 |
| TPTE/CT44 | - | - | - | 0 | - | 0 | - | 39 | - | 36 | - | - | - | - | - | - | 19 |

| | | | | | | | | | | | | | | | | | |
|---|---|---|---|---|---|---|---|---|---|---|---|---|---|---|---|---|---|
| ^{a}Abbreviations: Blad, bladder; Brn, brain; Brst, breast; Col, colon; Gas, gastric; H/N, head and neck; Leuk, leukemia; Lymph, lymphoma, NSCLC, non-small cell lung carcinoma; Mel, melanoma; Ov, ovarian; Pancr, pancreatic; Pros, prostate; Sarc, sarcoma; Ref, reference. ^{b}Reference 59. | | | | | | | | | | | | | | | | | |

Additional antigens associated with tumor neovasculature are VEGFR2 (vascular endothelial growth factor receptor 2) described in U.S. Patent No. 6,342,221; and Tie-2, an endothelium specific receptor tyrosine kinase which is described in W09943801.

One of skill in the art will appreciate that any other antigen or protein ' associated with vascular cells can be a target for the immunogenic compositions, including those that are presently known and those yet to be identified.

### Compositions

Immunogenic compositions, including, for example, vaccines, can be prepared using whole antigen or an epitopic peptide. Peptide immunogens can be readily prepared using standard peptide synthesis means known in the art, for example. Immunogens can be prepared commercially by one of numerous companies that do chemical synthesis. An example of such a company is American Peptides, Inc., where the distributor is CLINALFA AG (Laufelfingen, Switzerland). The antigens or immunogens can be prepared in accordance with GMP standards and purity can be assessed by analytical HPLC. The product can be characterized by amino-acid analysis and tested for sterility and the absence of pyrogens.

An antigen may be delivered to an animal's system either directly or indirectly. For example, a polypeptide may be delivered directly as the polypeptide, or it may be delivered indirectly, for example, using a DNA construct or vector, or a recombinant virus that codes for the desired antigen. Any vector driving expression in a professional antigen presenting cell can be suitable for this purpose. In indirect delivery, the antigen is expressed in the cell, then presented by the MHC Class I on the surface of the cell to stimulate a CTL response. Expression of a secreted form of the antigen can be useful to induce an antibody response recognizing antigens that are membrane proteins.

In a preferred embodiment, an encoded antigen can be delivered in the form of a naked plasmid expression vector. Particularly useful constructs are disclosed in U.S. Patent Application No. 09/561,572, entitled "EXPRESSION VECTORS ENCODING EPITOPES OF TARGET-ASSOCIATED ANTIGENS;" U.S. Patent Application No. 10/292,413 (Pub. No. 20030228634 A1), entitled "EXPRESSION VECTORS ENCODING EPITOPES OF TARGET-ASSOCIATED) ANTIGENS AND METHODS FOR THEIR DESIGN;" U.S. Patent Application No. 10/225,568 (Pub No. 2003-0138808); PCT Application No. PCT/US2003/026231 (Pub. No. WO 2004/018666); U.S. Patent No. 6,709,844, entitled AVOIDANCE OF UNDESIRABLE REPLICATION INTERMEDIATES IN PLASMIND PROPAGATION, and in U.S. Patent Application No. 10/026,066 (Pub. No. 20030215425 A1), entitled EPITOPE SYNCHRONIZATION IN ANTIGEN PRESENTING CELLS. The feasibility of and general procedures related to the use of naked DNA for immunization are described in U.S. Patent No. 5,589,466, entitled "INDUCTION OF A PROTECTIVE IMMUNE RESPONSE IN A MAMMAL BY INJECTING A DNA SEQUENCE" and in U.S. Patent No. 5,679,647, entitled "METHODS AND DEVICES FOR IMMUNIZING A HOST AGAINST TUMOR-ASSOCIATED ANTIGENS THROUGH ADMINISTRATIONS OF NAKED POLYNUCLEOTIDES WHICH ENCODE TUMOR-ASSOCIATED ANTIGENIC PEPTIDES,". The former teaches only intramuscular or intradermal injection while the latter teaches only administration to skin or mucosa.

In a preferred embodiment, the antigen can be administered directly to the lymphatic system. Intranodal administration for the generation of CTL is taught in U.S. Patent Application Nos. 09/380,534 and 09/776,232 (Pub. No.20020007173 A1), and in PCT Application No. PCTUS98/14289 (Pub. No. WO9902183A2) each entitled "A METIIOD OF INDUCING A CTL RESPONSE,". Single bolus injection intra lymph node (i.ln.) required only 0.1% of the dose required in order to obtain a similar level of CTL response by intramuscular (i.m.) injection. Therefore a protective response can be established against systemic viral infection with a single bolus delivered i.ln., but not with a dose nearing the practical limit delivered i.m. Repeated bolus injections i.m. failed to establish a protective response against a peripheral virus infection or transplanted tumor, whereas lower doses administered i.ln. were completely effective. Particularly useful intranodal immunization protocols are taught in Provisional U.S. Patent Application No. 60/479,393, entitled "METHODS TO CONTROL MAGNITUDE AND QUALITY THE MHC CLASS I-RESTRICTED IMMUNE RESPONSE".

A class of epitopes that can be advantageous in anti-cancer immunogenic compositions are housekeeping epitopes. These are produced through the action of the housekeeping (or standard) proteasome. Housekeeping epitopes can be liberated from the translation product of expression vectors through proteolytic processing by the immunoproteasome of professional antigen presenting cells (pAPC). In one embodiment of the invention, sequences flanking the housekeeping epitope(s) can be altered to promote cleavage by the immunoproteasome at the desired location(s). Housekeeping epitopes, their uses, and identification are described in U.S. Patent Application Nos. 09/560,465 filed on April 28, 2000, and U.S. Patent Application No. 10/026,066 (Pub. No. 20030215425 A1), filed on December 7, 2001, entitled "EPITOPE SYNCHRONIZATION IN ANTIGEN PRESENTING CELLS," and U.S. Patent Application No. 09/561,074, filed on April 28, 2000, entitled "METHOD OF EPITOPE DISCOVERY," .

Examples of housekeeping epitopes are disclosed in Provisional U.S. Patent Applications Nos. 60/282,211, filed on April 6, 2001; 60/337,017, filed on November 7, 2001; 60/363210 filed March 7, 2002; and 60/409,123, filed on September 5, 2002; U.S. Patent Application No. 10/117,937 (Publication No. 20030220239A1), filed on April 4, 2002; and U.S.'Patent Application No. 10/657,022, and PCT Application No. PCT/US2003/027706 (Pub. No. WO04022709A2) both entitled EPITOPE SEQUENCES.

In other embodiments of the invention, the housekeeping epitope(s) can be flanked by arbitrary sequences or by sequences incorporating residues known to be favored in immunoproteasome cleavage sites. As used herein the term "arbitrary sequences" refers to sequences chosen without reference to the native sequence context of the epitope, their ability to promote processing, or immunological function. In further embodiments of the invention multiple epitopes can be arrayed head-to-tail. These arrays can be made up entirely of housekeeping epitopes. Likewise, the arrays can include alternating housekeeping and immune epitopes. Alternatively, the arrays can include housekeeping epitopes flanked by immune epitopes, whether complete or distally truncated. Further, the arrays can be of any other similar arrangement There is no restriction on placing a housekeeping epitope at the terminal positions of the array. The vectors can additionally contain authentic protein coding sequences or segments thereof containing epitope clusters as a source of immune epitopes. The term "authentic" refers to natural protein sequences.

Epitope clusters and their uses are described in U.S. Patent Application Nos. 09/561,571, entitled "EPITOPE CLUSTERS," filed on April 28, 2000; 10/005,905, entitled "EPITOPE SYNCHRONIZATION IN ANTIGEN PRESENTING CELLS," filed on November 7, 2001; and 10/026,066, filed on December 7, 2001, also entitled "EPITOPE SYNCHRONIZATION IN ANTIGEN PRESENTING CELLS,".

In another embodiment of the invention an encoded antigen can be delivered in the form of a viral vector. A wide array of viruses with modified genomes adapted to express interposed reading frames but often no, or at least a reduced number of, viral proteins are know in the art, including without limitation, retroviruses including lentiviruses, adenoviruses, parvoviruses including adeno-associated virus, herpesviruses, and poxviruses including vaccinia virus. Such viral vectors facilitate delivery of the nucleic acid component into the cell allowing for expression. A subset of these vectors, such as retroviruses and parvoviruses, promote integration of their nucleic acid component into the host genome, whereas others do not.

Bacteria can also serve as vectors, that is they can be used to deliver a nucleic acid molecule capable of causing expression of an antigen. For example, a strain of *Listeria monocytogenes* has been devised that effects its own lysis upon entering the cytosol of macrophages (its normal target), thereby releasing plasmid from which antigen is subsequently expressed (Dietrich, G. et al., Biotechnology 16:181-185, 1998). *Shigela flexneri* and *Escherichia coli* have been similarly used (Sizemore, D.R et al., Science 270:299-302, 1995, and Courvalin, P. et al., Life Sci. 318:1207-1212, 1995, respectively,).

The use of microbial vectors for nucleic acid delivery can be complicated by the immune reactions the vectors themselves provoke. When prolonged or repeated administration is required, antibody elicited by the earlier treatment can prevent useful quantities of the vector from ever reaching its intended host However, by direct administration into a lymph node, for example, the combination of proximity to host cells and the much reduced effective dose makes it possible to administer a dose capable of evading or overwhelming an existing antibody titer.

The word vector has been used, here and elsewhere, in reference to several modalities and variously modified (e.g., expression vector, viral vector, delivery vector, etc.). The underlying principle is that a nucleic acid capable of causing expression of an antigen, rather than the antigen itself ultimately arrives in an APC. Unless modified, explicitly or by local context, the term vector as used herein is intended to encompass all such possibilities.

The techniques discussed above are distinct from the approach of modifying the microbial genome, including extra-chromosomal DNA, such that the antigen is produced as a component of the microbe, which is then itself administered as the immunogen. Examples of microbes used in the genomic modification approach include viruses, bacteria, fungi, and protazoa. In embodiments of the invention described herein, the compositions, including the vaccines, can include the already synthesized antigen or a nucleic acid capable of causing an APC to express the antigen *in vivo*. In alternative embodiments, combinations of these two techniques are used. For example, one embodiment contemplates the use of a virus vector as discussed above that also incorporates a target epitope into a capsid or envelope protein.

Antigens may be used alone or may be delivered in combination with other antigens or with other compounds such as cytokines. Cytokines that are known to enhance immune stimulation of CTL responses, include, for example, GM-CSF, IL- 12, IL-2, TNF, IFN, IL-18, IL-3, IL-4, IL-8, IL-9, IL-13, IL-10, IL-14, IL-15, G-SCF, IFN alpha, IFN beta, IFN gamma, TGF alpha, TGF beta, and the like. Cytokines are known in the art and are readily available in the literature or commercially. Many animal and human tumors have been shown to produce cytokines, such as IL-4, IL-10, TGF-B, that are potent modulators of the immune response and that protect tumors from immune-mediated destruction. The production of IL-4, IL-10 or TGF-B by tumors may achieve this protective effect by suppressing the induction of cellular immunity, including the elaboration of CTL responses. Alternatively, cytokines that support CTL responses can be exogenously added to help in the balance between induction of anti-tumor cell mediated and non-tumor-destructive humoral responses. Several such exogenous cytokines show utility in experimental mouse vaccination models which are known to enhance CTL responses, including GM-CSF, IFN and IL-2. An example of an effective exogenous cytokine that may be used is GM-CSF. GM-CSF is reported to enhance the expression of the so called "co-stimulatory" molecules, such as B7-1 or B7-2 on antigen presenting cells (APC). These co-stimulatory molecules are important players in the variety of interactions that occur during stimulation of CTL by APC. Moreover, GM-CSF is known to induce activation of APCs and to facilitate growth and differentiation of APCs, thereby making these APCs important CTL stimulating cells available both in greater numbers and potency.

### Delivery of the Antigen

While not wanting to be bound by any particular theory, it is thought that T cells do not have a functional memory that is long-lived. Antibody-mediated B-cell memory, on the other hand, appears to have a long-lived effector memory. Thus, delivering an antigen that induces a CTL response is most preferably done over time to keep the patient's immune system appropriately stimulated to attack the target cells. In one approach the presence of antigen is maintained virtually continuously within the lymphatic system to maintain effector CTL function as disclosed in U.S. Patent Application No. 09/776,232 (Pub. No.20020007173 A1), entitled "A METHOD OF INDUCING A CTL RESPONSE". In another approach T cell memory is repeatedly induced, and re-amplified and reactivated as described in Provisional U.S. Patent Application No. 60/479,393, entitled "METHODS TO CONTROL MAGNITUDE AND QUALITY THE MHC CLASS I-RESTRICTED IMMUNE RESPONSE". While it has been suggested that antigens and adjuvants can be prepared as biodegradable microspheres or liposomes, none of these preparations have thus far provided a CTL response that is useful for attacking cancer cells or pathogens on a long term basis. Preferably, delivery of the antigen is sustained over the desired period of time at a level sufficient to maintain the antigen level to obtain the desired response. In one embodiment, a reservoir having fluid antigen composition can be used to deliver the antigen such that it reaches the animal's lymphatic system. While much of the following discussion focuses on the use of infusion to deliver the antigen it is also possible to use bolus injections directly into the lymphatic system, the number and frequency of which will depend on the persistence of antigen conferred by the particular form and formulation of antigen used.

Ultimately antigen finds its way into the lymphatic system in order to most efficiently stimulate CTL. Delivery of antigen can involve infusion into various compartments of the body, including but not limited to subcutaneous, intravenous, intraperitoneal and intralymphatic, the latter being preferred. While each of these points of infusion results in antigen uptake into the lymphatic system, the relative amounts of antigen needed to induce a beneficial CTL. response varies according to the site of infusion. In general, direct infusion of antigen into the lymph system is deemed to be the most efficient means of inducing a CTL response, however, any delivery route may be used. Pump systems are capable of delivering material quantities of antigen in a range that is suitable for inducing a CTL response through delivery to all compartments of the body. CTL stimulation following delivery of antigen via the various routes will vary depending on the properties of different antigens, including factors that influence antigen behavior in the body and its rate of equilibration to (or longevity in) the lymph, such as antigen stability in the body fluid, solubility of antigen in body fluid, binding affinity for HLA and potency as a stimulator of CTL.

In a preferred embodiment, introduction of the antigen is done as directly as possible to the lymphatic system to avoid the destruction of the antigen by metabolism in the body. When introduction of a fluid antigen composition occurs subcutaneously, larger quantities of antigen are needed to assure enough antigen reaches the lymphatic system. Such subcutaneous injection is contemplated by the invention disclosed herein, depending on factors such as cost, stability of the antigen, how quickly the antigen gets to the lymph system, how well it equilibrates with the lymph, and other factors that the attending doctor or specialist will recognize. Subcutaneous delivery generally can require 100 to 1000 times more antigen than direct delivery to the lymph system. It is preferable, therefore, that the antigen composition is introduced through a device for local administration to the lymphatic system, *e.g.*, the spleen, a lymph node, or a lymph vessel. The device for local administration can be positioned outside the patient or implanted into the patient. In either case, the device can have a reservoir to hold the fluid antigen-containing composition, a pump to transfer the composition, and a transmission channel leading from the reservoir to be directed to the preferred region of administration in the patient's body. In either case it is preferably portable.

For the device positioned outside the patient's body (the external device), there are numerous devices used for delivering insulin to diabetic patients that are useful in delivering antigen according to the embodiments described herein. Generally these devices can be comprised of a reservoir for holding the antigen composition (instead of insulin), a programmable pump to pump the composition out of the reservoir, a transmission channel or line for transmitting the composition, and a means to introduce the composition into the animal's body to ultimately reach the lymphatic system.

Preferably, the reservoir for the antigen composition should be large enough for delivery of the desired amount of antigen over time and easily refillable or replaceable without requiring the user to reinsert the means for introducing the antigen composition to the lymph system.

In preparing the antigen compositions of embodiments of the invention disclosed herein, a composition (preferably aqueous) can be prepared to be compatible with the lymph system and physiologically acceptable to the animal being treated. Relevant considerations include, for example, the physicochemical properties of the antigen, such as the isoelectric point, molecular weight, glycosylation or other post-translational modification, and overall amino acid composition. These properties along with any known behavior of the drug in different solutions (*e.g.*, different buffers, cofactors, etc.) as well as its *in vivo* behavior can help guide the choice of formulation components. One parameter that impacts all the major degradation pathways is the solution pH. Thus, the initial formulations also assess the pH dependence of the degradation reactions and the mechanism for degradation, which can often be determined from the pH dependence to determine the stability of the protein in each solution. Rapid screening methods usually involve the use of accelerated stability at elevated temperatures (e.g., 40° C) using techniques known in the art.

In general the antigen compositions useful in embodiments described herein can be suitable for parenteral injection, in very small quantities. As such a composition should be free of contamination and have a pH compatible with the lymphatic system. However, because very small quantities of the antigenic composition will be delivered it need not be the same pH as blood or lymph, and it need not be aqueous-based. The preferable pH range that is compatible is from about 6.7 - 7.3 and can be prepared using water for injection to meet USP specifications (see Remington: The Science and Practice of Pharmacy, Nineteenth Edition; Chapters 86-88). For antigens that are less soluble, a suitable cosolvent or surfactant may be used, such as dimethyl sulfoxide (DMSO) or PLURONIC brand surfactants. Generally, a standard saline solution that is buffered with a physiologically acceptable weak acid and its base conjugate, *e.g.*, a phosphate or citrate buffering system, will be the basis of the antigen composition. In some cases, a small amount of an antioxidant may be useful to stabilize the composition and prevent oxidation. Factors to consider in preparing the antigen compositions may be found in the 1994 American Chemical Society book entitled "Formulation and Delivery of Proteins and Peptides" (Acs Symposium Series, No. 567) by Jeffery L. Cleland and Robert Langer (Editor)).

For nucleic acid encoded antigens similar considerations can apply, although the variety of physico-chemical properties encountered with polypeptides is absent, so that acceptable formulations will have nearly universal applicability. As seen in Examples 6-10, plasmid DNA in standard phosphate buffered saline (PBS) is an acceptable and effective formulation. In some embodiments of the invention, DNA is administered continuously or intermittently at short intervals, from a reservoir worn on, or implanted in, the patient's body. It is preferable that the DNA be maintained in a soluble, stable form at or near body temperature over a period of time measured minimally in days. In such applications where the formulated nucleic acid will be delivered from a reservoir over a period of several days or longer, the stability of the nucleic acid at room or body temperature for that period of time, as well as its continued sterility, take on increased importance. The addition of bacteriostatic agents (*e.g.*, benzyl or ethyl alcohol) and chelating agents (e.g. EDTA) is useful toward these ends. Formulations containing about 0.5-2 % ethyl alcohol, 0.25-0.5mM EDTA generally perform well. Such formulations are also appropriate for bolus injections.

Generally the amount of the antigen in the antigen composition will vary from patient to patient and from antigen to antigen, depending on such factors as the activity of the antigen in inducing a response and the flow rate of the lymph through the patient's system. In general the antigen composition may be delivered at a rate of from about 1 to about 500 microliters/hour or about 24 to about 12000 microliters/day. The concentration of the antigen is such that about 0.1 micrograms to about 10,000 micrograms of the antigen will be delivered during 24 hours. The flow rate is based on the knowledge that each minute approximately about 100 to about 1000 microliters of lymph fluid flows through an adult inguinal lymph node. The objective is to maximize local concentration of vaccine formulation in the lymph system. A certain amount of empirical investigation on patients will be necessary to determine the most efficacious level of infusion for a given vaccine preparation in humans.

To introduce the antigen composition into the lymphatic system of the patient the composition is preferably directed to a lymph vessel, lymph node, the spleen, or other appropriate portion of the lymph system. Preferably, the composition is directed to a lymph node such as an inguinal or axillary node by inserting a catheter or needle to the node and maintaining the catheter or needle throughout the delivery. Suitable needles or catheters are available made of metal or plastic (e.g., polyurethane, polyvinyl chloride [PVC], TEFLON, polyethylene, and the like). In inserting the catheter or needle into the inguinal node for example, the inguinal node is punctured under ultrasonographic control using a Vialon™ Insyte-W™ cannula and catheter of 24G3/4 (Becton Dickinson, USA) which is fixed using Tegaderm™ transparent dressing (Tegaderm™ 1624, 3M, St. Paul, MN 55144, USA). This procedure is generally done by an experienced radiologist. The location of the catheter tip inside the inguinal lymph node is confirmed by injection of a minimal volume of saline, which immediately and visibly increases the size of the lymph node. The latter procedure allows confirmation that the tip is inside the node. This procedure can be performed to ensure that the tip does not slip out of the lymph node and can be repeated on various days after implantation of the catheter. In the event that the tip does slip out of location inside the lymph node, a new catheter can be implanted.

### Formulation and Treatment protocol

There are several approaches to utilizing the combination of TuAAs with DNA vaccines. A first approach is to include all the antigens or epitopes from all the antigens in a given combination into a single DNA expression vector. This approach has the advantages of simplicity for manufacturing and administration to patients. However, in some instances, epitope competition can limit the usefulness of this approach. That is, it is possible that only the most immunogenic epitope will elicit an immune response when a vaccine with several epitopes representing all TuAAs in the combination is given to patients. It is also more difficult to design and construct a DNA vaccine in which all epitopes are expressed at high efficiencies. Nevertheless, because the procedure for treating patients is simple and uniform within each type of cancer, the cost is likely to be lower than for the other approaches described below.

An alternate approach is to include only one antigen or epitopes of one antigen in a DNA expression vector. This approach has the advantages of simplicity in designing and constructing the DNA vector, flexibility, and customized administration to patients. If a large number of individual TuAA vaccines are available, then one can customize treatment for each individual patient based on the TuAA expression profile of his or her tumor. For example, if the standard combination for treating a given type of cancer is TuAA A, B, and C (where A, B, and C designate different tumor associated antigens), but a patient's tumor expresses TuAA A, C, and Z (but not B), then the patient can be treated with separate vaccines for each of A, C, and Z. It is expected that this flexibility and customizability will improve the success rate of immunotherapy because antigen redundancy can be achieved for each patient. However, the procedure of treating the patient may be more complex. For example, delivery using this approach may include a sequential administration scheme (one antigen at a time), or injection into multiple, anatomically separate sites of the patient at about the same time.

Still another approach is to combine epitopes from multiple TuAAs that have similar immunogenicity into a DNA expression vector (more than one vector may be used for some combinations). This approach can have some of the advantages of the above two approaches but also can suffer from the disadvantages of the previous two.

A profile of the antigen expression of a particular tumor can be used to determine which antigen or combination of antigens to use.

Patients that can benefit from such methods of immunization can be recruited using methods to define their MHC protein expression profile and general level of immune responsiveness. In addition, their level of immunity can be monitored using standard techniques in conjunction with access to peripheral blood. Finally, treatment protocols can be adjusted based on the responsiveness to induction or amplification phases and variation in antigen expression. For example, repeated entrainment doses preferably can be administered until a detectable response is obtained, and then administering the amplifying peptide dose(s), rather than amplifying after some set number of entrainment doses. Similarly, scheduled amplifying or maintenance doses of peptide can be discontinued if their effectiveness wanes, antigen-specific regulatory T cell numbers rise, or some other evidence of tolerization is observed, and further entrainment can be administered before resuming amplification with the peptide.

Many variations and alternative elements of the invention have been disclosed. Still further variations and alternate elements will be apparent to one of skill in the art. Various embodiments of the invention can specifically include or exclude any of these variation or elements.

The following examples are for illustrative purposes only and are not intended to limit the scope of the embodiments in any way.

### Examples

### TuAA analysis and selection of combinations

The presence of TuAAs was measured by Real-Time PCR (RT-PCR). Briefly, total RNA was isolated from tumor specimens by standard methods and cDNA was made with standard reverse transcription procedures. Complementary DNA (cDNA) was amplified with specially designed, gene specific, primers that anneal only to cDNA but not genomic DNA. TuAA expression patterns of 12 ovarian and 7 colorectal tumor specimens were analyzed by RT-PCR. The results are summarized in the Table 4 below.

**Table 4**

| | Total # | PRAME | NY-ESO-1 | SSX-2 | PSMA | MAGE1 | MAGE3 |
|---|---|---|---|---|---|---|---|
| Ovarian | 12 | 12 | 5 | 6 | 6 | 4 | 3 |
| Colorectal | 7 | 5 | 1 | 2 | 5 | 0 | 1 |

### Example 1

### Ovarian Cancer

In the case of ovarian cancer, all samples analyzed were positive for PRAME. Thus the inclusion of PRAME in the combination improves coverage of the cases with ovarian cancer.

In order to achieve antigen redundancy and improve coverage in a large population, combinations of other antigens were considered in addition to PRAME. SSX-2 as well as PSMA were present in 6 of the 12 cases individually, but the combination of SSX-2 and PSMA provided coverage in 9 of 12 cases. Although NY-ESO-1 and SSX-2 were only present in 5 and 6 of the 12 cases, respectively, either NYESO-1 or SSX-2 was detected in 7 of the 12 cases.

Thus, the combination of PRAME, SSX-2, and PSMA or PRAME, NY-ESO-1, and SSX-2 provided preferable coverage and redundancy compared to the combination of PRAME and PSMA or the combination of PRAME and SSX-2. The combination of PRAME, SSX-2, and PSMA provided excellent coverage of cases and good antigen redundancy because the majority of ovarian tumor samples analyzed had at least two of the four TuAA in the combination present. The combination of PRAME, SSX-2, PSMA, and NY-ESO-1 provided more prefered antigen redundancy, and thus, lower possibility of tumor escape.

### Example 2

### Colorectal cancer

In the case of colorectal cancer, PRAME and PSMA were each detected in 5 of the 7 samples analyzed. In 6 of the 7 cases, either PRAME or PSMA was detected. Although SSX-2 was only detected in 2 of 7 cases, both SSX-2-PRAME and SSX2-PSMA combinations increased coverage to 6 of 7. Similarly, although NYESO-1 was detected in only 1 of 7 cases, the combination of NY-ESO-1-PRAME as well as the NYESO-1-PSMA combination increased coverage to 6 of 7. The addition of SSX-2 or NYESO-1 to the PRAME and PSMA combination improved coverage to 7 of 7. Thus, the combination of PRAME, PSMA, and NYESO-1, or the combination of PRAME, PSMA, and SSX-2 provided good coverage of cases and redundancy of antigens for a majority of patients. The combination of PRAME, PSMA, NY-ESO-1, and SSX-2 provided further redundancy.

### Comparative Example 3

### Pancreatic Cancer

In pancreatic cancer specimens, the presence of NYESO-1 and SSX-2 was detected in 40% and 20% of the specimens, respectively. PSMA and over-expression of HER2-/neu were reported to be present in 100% and 21% of pancreatic tumors, respectively (Chang SS et al, Cancer Res 1999, 59:3192; Safran H et al, Am J Clin Oncol. 2001, 24:496). Although over-expression of HER2/neu may render the cancer tissue a preferred target, thus providing some specificity for immunotherapy, low level expression of HER2/neu in normal tissues remains a concern. Thus, the combination of NYESO-1, SSX-2, and PSMA provides excellent coverage and some redundancy for treating pancreatic cancer.

### Comparative Example 4

### Non-small cell lung cancer

For non-small cell lung cancer, the reported presence of NYESO-1, SSX-2, MAGE-3, BAGE, over-expression of Her2/neu, and PSMA was 21, 15, 60, 6, 16, and 100%, respectively (Scanlan MJ et al, Cancer lett 2000, 150:155; Chang SS et al, Cancer Res 1999, 59:3192; Selvaggi G et al, Cancer 2002, 94:2669). Thus, the combination of NYESO-1, SSX-2, MAGE-3, and PSMA provides coverage and antigen redundancy for the immunotherapy of non-small cell lung cancer.

### Comparative Example 5

### Renal cell carcinoma

For renal cell carcinoma, SSX-2, PSMA and PRAME were detected with frequencies of 5, 100 and 40%, respectively (Sahin, U et al, Clin Cancer Res. 2000, 6:3916; Chang SS et al, Urology 2001, 57:801; Neumann E et al, Cancer Res. 1998, 58:4090). Thus, the combination of PSMA and PRAME provides excellent coverage and redundancy for renal cell carcinoma. Adding SSX-2 to the combination of PSMA and PRAME improves redundancy.

### Comparative Example 6

### Melanoma

For melanoma, Melan A, Tyrosinase, NYESO-1, and SSX-2 were reported to be present in 92, 92, 41, and 35% of tumor specimens, respectively (Fetsch PA, et al, Cancer 1999, 87:37; Fetsch PA, et al, Cancer 2000, 90:252; Schultz-Thater E et al, Br J Cancer 2000, 83:204; Sahin, U et al, Clin Cancer Res. 2000, 6:3916). Therefore, the combination of Melan A, Tyrosinase, NYESO-1, and SSX-2 provides excellent coverage and antigen redundancy for the immunotherapy of melanoma. Significant redundancy is achieved using tyrosinase and melan-A together, or by combining NY-ESO-1 and SSX-2 with either of tyrosinase or melan-A.

### Comparative Example 7

### Schedule of immunization with plasmids expressing epitopes from two antigens

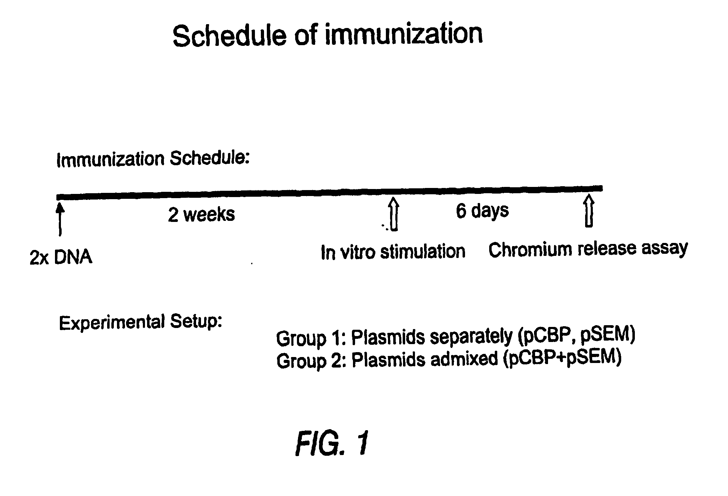

Two groups of HHD mice (n=4) were immunized via intra lymph node injection with either pSEM expressing Melan-A ₂₆₋₃₅A27L (ELA) and pCBP expressing SSX-2₄₁₋₄₉ as a mixture; or with pSEM in the left inguinal lymph node and pCBP in the right inguinal lymph node, twice, at day 0 and 4 as shown in Figure 1. The amount of the plasmid was 25µg/plasmid/dose. Two weeks later, the animals were sacrificed, and cytotoxicity was measured against T2 cells pulsed or not with peptide.

### Comparative Example 8

### Co-adminishation of different vectors carrying distinct antigens

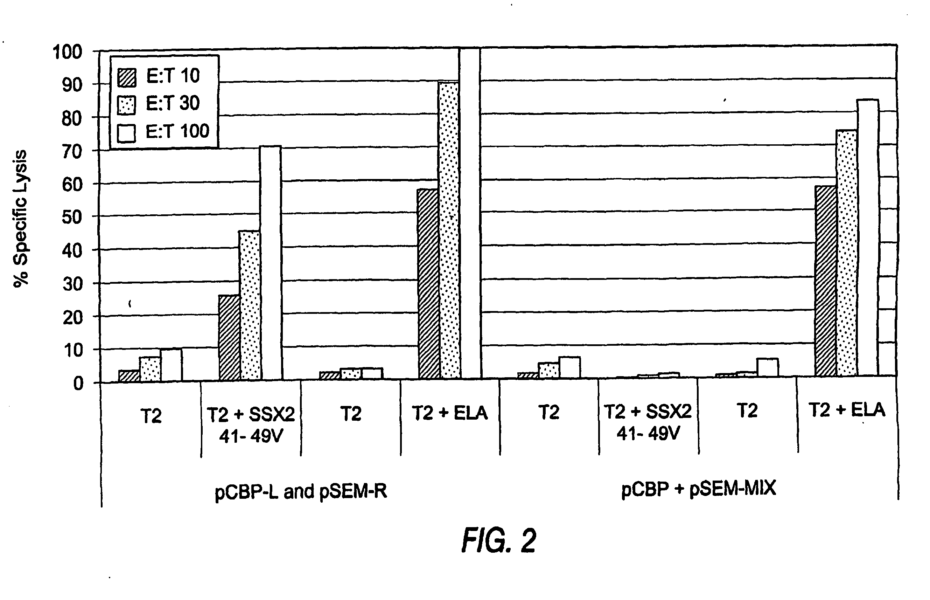

The animals immunized as described in Example 7, were sacrificed and splenocytes from each group pooled and stimulated with the two peptides (ELA or SSX-2₄₁₋₄₉) in parallel. The cytotoxicity was measured by incubation with Cr⁵¹-tagged, peptide loaded T2 target cells. Data in Figure 2 show mean of specific cytotoxicity (n=4/group) against various target cells.

The results show that use of plasmid mixture interferes with the response elicited by pCBP plasmid; however, segregating the two plasmids relative to site of administration rescues the activity of pCBP. Thus, the co-administration of different vectors carrying distinct antigens can result in establishment of a hierarchy with regard to immunogenicity. Vector segregation can rescue the immunogenicity of the less dominant component, resulting in a multivalent response.

### Comparative Example 9

### Rescue of Multivalent Response by Addition of Peptide Boost Steps

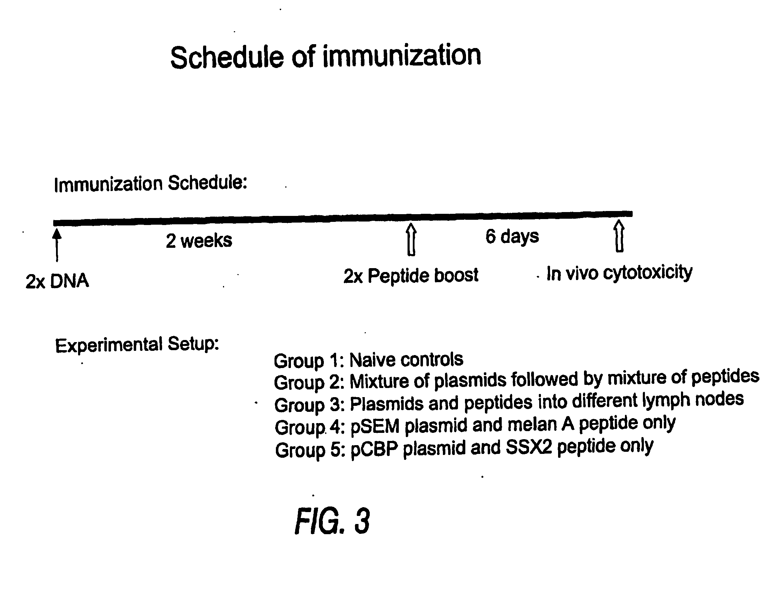

Four groups of HHD mice (n=6) were immunized via intra lymph node injection with either pSEM and pCBP as a mixture; or with pSEM in the left inguinal lymph node and pCBP in the right inguinal lymph node, twice, at day 0 and 4 as shown in Figure 3. As a control, mice were immunized with either pSEM or pCBP plasmid. The amount of the plasmid was 25µg/plasmid/dose. Two weeks later, the animals were boosted with melan A and/or SSX-2 peptides, mirroring the plasmid immunization dose and combination Two weeks later, the animals were challenged with splenocytes stained with CFSE and loaded or not with Melan A or SSX-2 peptide, for evaluation of *in vivo* cytotoxicity.

### Comparative Example 10

### Peptide amplification rescues the immunogenicity of the less dominant epitope

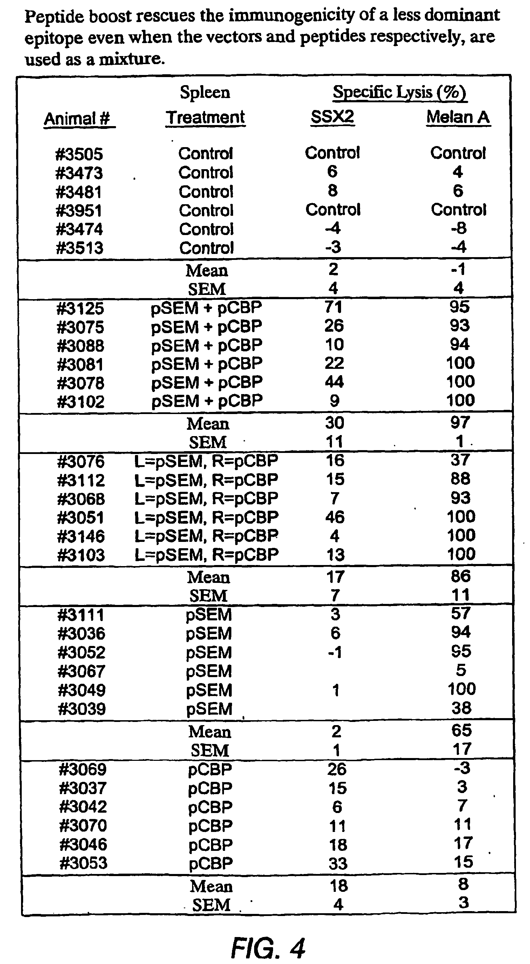

Mice were immunized as described in Example 9 and challenged with HHD littermate splenocytes coated with ELA or SSX-2 peptide, employing a triple peak CFSE *in vivo* cytotoxicity assay that allows the assessment of the specific lysis of two antigen targets simultaneously. Equal numbers of control-CFSE^{lo}, SSX-2₄₁₋₄₉-CFSE^{med}, and ELA-CFSE^{hi} cells were intravenously infused into immunized mice and 18 hours later the mice were sacrificed and target cell elimination was measured in the spleen (Figure 4) by CFSE fluorescence using a flow cytometry. Figure 4 shows the percent specific lysis of the SSX2 and Melan-A antigen targets from individual mice, as well as the mean and SEM for each group.

The results show that immunizing the animals with a mixture of the two vaccines comprising plasmids followed by peptides generated immunity to both antigens and resulted in the highest immune response, representing an average SSX-2 percent specific lysis in the spleen of 30+/-11, and an average Melan-A percent specific lysis of 97+/-1.

### Example 11

### Clinical practice for entrain-and-amplify immunization

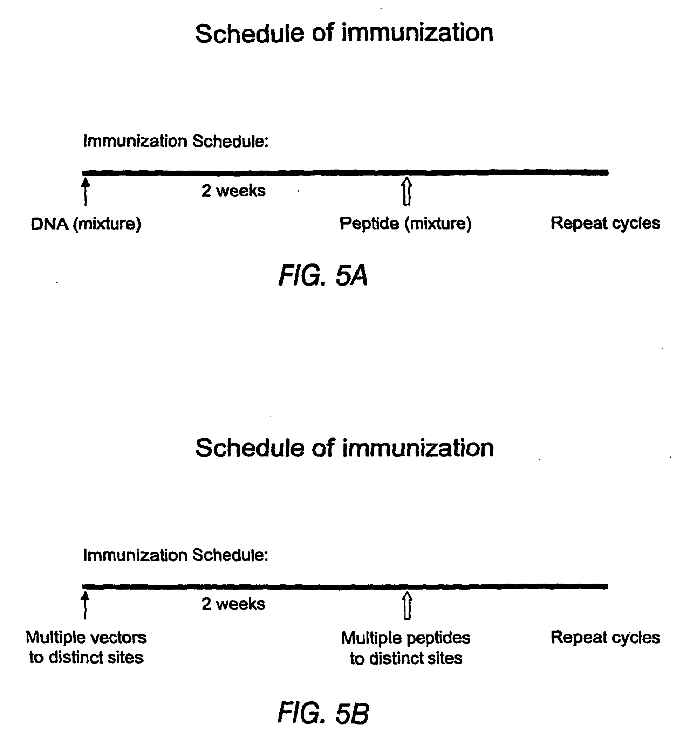

The data in figures 2 and 4 suggest two scenarios for achieving a strong multivalent response in the clinic, shown in Figure 5. In the first scenario (A), use of peptides for boosting restores multivalent immune responses even if plasmids and peptides are used as mixtures. In the second scenario (B), segregation of plasmid and peptide components respectively, allows induction of multivalent immune responses.

### Example 12

### Antigen sequences

Below are sequences of the antigens listed in Table 1.

| **SEQ. ID NO. 1** | | | | |
|---|---|---|---|---|
| SIZE: 529 aa | | | | |
| DEFINITION Tyrosinase precursor | | | | |
| ACCESSION NO.: P14679 | | | | |
| | | | | |
| mllavlycll | wsfqtsaghf | pracvssknl | mekeccppws | gdrspcgqls |
| grgscqnill | snaplgpqfp | ftgvddresw | psvfynrtcq | csgnfmgfnc |
| gnckfgfwgp | ncterrllvr | rnifdlsape | kdkffayltl | akhtissdyv |
| ipigtygqmk | ngstpmfndi | niydlfvwmh | yyvsmdallg | gseiwrdidf |
| aheapaflpw | hrlfllrweq | eiqkltgden | ftipywdwrd | aekcdictde |
| ymggqhptnp | nllspasffs | swqivcsrle | eynshqslcn | gtpegplrrn |
| pgnhdksrtp | rlpssadvef | clsltqyesg | smdkaanfsf | rntlegfasp |
| ltgiadasqs | smhnalhiym | ngtmsqvqgs | andpifllhh | afvdsifeqw |
| lrrhrplqev | ypeanapigh | nresymvpfi | plyrngdffi | sskdlgydys |
| ylqdsdpdsf | qdyiksyleq | asriwswllg | aamvgavlta | llaglvsllc |
| rhkrkqlpee kqpllmeked yhslyqshl | | | | |
| | | | | |

| **SEQ. ID NO. 2** | | | | |
|---|---|---|---|---|
| SIZE: 118 aa | | | | |
| DEFINITION Melanoma antigen recognized by T-cells 1 (MART-1) (Melan-A protein) | | | | |
| ACCESSION NO.: Q16655 | | | | |
| | | | | |
| mpredahfiy | gypkkghghs | yttaeeaagi | giltvilgvl | lligcwycrr |
| rngyralmdk | slhvgtqcal | trrcpqegfd | hrdskvslqe | kncepvvpna |
| ppayeklsae qspppysp | | | | |
| | | | | |

| **SEQ. ID NO. 3** | | | | |
|---|---|---|---|---|
| SIZE: 223 aa | | | | |
| DEFINITION: synovial sarcoma, X breakpoint 2 isoform a; sarcoma, synovial, X-chromosome-related 2; SSX2 protein [Homo sapiens]. | | | | |
| ACCESSION NO.: NP_003138 | | | | |
| | | | | |
| mngddafarr | ptvgaqipek | iqkafddiak | yfskeewekm | kasekifyvy |
| mkrkyeamtk | lgfkatlppf | mcnkraedfq | gndldndpnr | gnqverpqmt |
| fgrlqgispk | impkkpaeeg | ndseevpeas | gpqndgkelc | ppgkpttsek |
| ihersgnrea | qekeerrgta | hrwssqnthn | igrfslstsm | gavhgtpkti |
| thnrdpkggn mpgptdcvre nsw | | | | |

| **SEQ. ID NO. 4** | | | | |
|---|---|---|---|---|
| SIZE: 750 aa | | | | |
| DEFINITION folate hydrolase (prostate-specific membrane antigen) [Homo sapiens]. | | | | |
| ACCESSION NO.: NP_004467 | | | | |
| | | | | |
| mwnllhetds | avatarrprw | lcagalvlag | gffllgflfg | wfikssneat |
| nitpkhnmka | fldelkaeni | kkflynftqi | phlagteqnf | qlakqiqsqw |
| kefgldsvel | ahydvllsyp | nkthpnyisi | inedgneifn | tslfeppppg |
| yenvsdivpp | fsafspqgmp | egdlvyvnya | rtedffkler | dmkincsgki |
| viarygkvfr | gnkvknaqla | gakgvilysd | padyfapgvk | sypdgwnlpg |
| ggvqrgniln | lngagdpltp | gypaneyayr | rgiaeavglp | sipvhpigyy |
| daqkllekmg | gsappdsswr | gslkvpynvg | pgftgnfstq | kvkmhihstn |
| evtriynvig | tlrgavepdr | yvilgghrds | wvfggidpqs | gaavvheivr |
| sfgtlkkegw | rprrtilfas | wdaeefgllg | stewaeensr | llqergvayi |
| nadssiegny | tlrvdctplm | yslvhnltke | lkspdegfeg | kslyeswtkk |
| spspefsgmp | risklgsgnd | fevffqrlgi | asgrarytkn | wetnkfsgyp |
| lyhsvyetye | lvekfydpmf | kyhltvaqvr | ggmvfelans | ivlpfdcrdy |
| avvlrkyadk | iysismkhpq | emktysvsfd | slfsavknft | eiaskfserl |
| qdfdksnpiv | lrmmndqlmf | lerafidplg | lpdrpfyrhv | iyapsshnky |
| agesfpgiyd alfdieskvd pskawgevkr qiyvaaftvq aaaetlseva | | | | |
| | | | | |

| **SEQ. ID NO. 5** | | | | |
|---|---|---|---|---|
| SIZE: 309 aa | | | | |
| DEFINITION Melanoma-associated antigen 1 (MAGE-1 antigen) (Antigen MZ2-E). | | | | |
| ACCESSION NO.: P43355 | | | | |
| | | | | |
| msleqrslhc | kpeealeaqq | ealglvcvqa | atssssplvl | gtleevptag |
| stdppqspqg | asafpttinf | trqrqpsegs | ssreeegpst | scileslfra |
| vitkkvadlv | gflllkyrar | epvtkaemle | sviknykhcf | peifgkases |
| lqlvfgidvk | eadptghsyv | lvtclglsyd | gllgdnqimp | ktgfliivlv |
| miamegghap | eeeiweelsv | mevydgrehs | aygeprkllt | qdlvqekyle |
| yrqvpdsdpa ryeflwgpra laetsyvkvl eyvikvsarv rfffpslrea | | | | |
| alreeeegv | | | | |
| | | | | |

| **SEQ. ID NO. 6** | | | | |
|---|---|---|---|---|
| SIZE: 314 aa | | | | |
| DEFINITION Melanoma-associated antigen 3 (MAGE-3 antigen) (Antigen MZ2-D). | | | | |
| ACCESSION NO.: P43357 | | | | |
| | | | | |
| mpleqrsqhc | kpeeglearg | ealglvgaqa | pateeqeaas | ssstlvevtl |
| gevpaaespd | ppqspqgass | lpttmnyplw | sqsyedssnq | eeegpstfpd |
| lesefqaals | rkvaelvhfl | llkyrarepv | tkaemlgsw | gnwqyffpvi |
| fskassslql | vfgielmevd | pighlyifat | clglsydgll | gdnqimpkag |
| lliivlaiia | regdcapeek | iweelsvlev | fegredsilg | dpkklltqhf |
| vqenyleyrq | vpgsdpacye | flwgpralve | tsyvkvlhhm | vkisggphis |
| ypplhewvlr egee | | | | |

| **SEQ. ID NO. 7** | | | | |
|---|---|---|---|---|
| SIZE: 180 aa | | | | |
| DEFINITION Cancer/testis antigen 1 (Autoimmunogenic cancer/testis antigen NY-ESO-1). | | | | |
| ACCESSION NO.: P78358 | | | | |
| | | | | |
| mqaegrgtgg | stgdadgpgg | pgipdgpggn | aggpgeagat | ggrgprgaga |
| arasgpggga | prgphggaas | glngccrcga | rgpesrllef | ylampfatpm |
| eaelarrsla | qdapplpvpg | vllkeftvsg | niltirltaa | dhrqlqlsis |
| sclqqlsllm witqcflpvf laqppsgqrr | | | | |
| | | | | |

| **SEQ. ID NO. 8** | | | | |
|---|---|---|---|---|
| SIZE: 509 aa | | | | |
| DEFINITION preferentially expressed antigen in melanoma; melanoma antigen preferentially expressed in tumors; Opa-interacting protein OIP4; preferentially expressed antigen of melanoma [Homo sapiens] . | | | | |
| ACCESSION NO.: NP_006106 | | | | |
| | | | | |
| merrrlwgsi | qsryismsvw | tsprrlvela | gqsllkdeal | aiaalellpr |
| elfpplfmaa | fdgrhsqtlk | amvqawpftc | lplgvlmkgq | hlhletfkav |
| ldgldvllaq | evrprrwklq | vldlrknshq | dfwtvwsgnr | aslysfpepe |
| aaqpmtkkrk | vdglsteaeq | pfipvevlvd | lflkegacde | lfsyliekvk |
| rkknvlrlcc | kklkifampm | qdikmilkmv | qldsiedlev | tctwklptla |
| kfspylgqmi | nlrrlllshi | hassyispek | eeqyiaqfts | qflslqclqa |
| lyvdslfflr | grldqllrhv | mnpletlsit | ncrlsegdvm | hlsqspsvsq |
| lsvlslsgvm | ltdvspeplq | allerasatl | qdlvfdecgi | tddqllallp |
| slshcsqltt | lsfygnsisi | salqsllqhl | iglsnlthvl | ypvplesyed |
| ihgtlhlerl aylharlrel lcelgrpsmv wlsanpcphc gdrtfydpep | | | | |
| ilcpcfmpn | | | | |
| | | | | |

| **SEQ. ID NO. 9** | | | | |
|---|---|---|---|---|
| SIZE: 1255 aa3 | | | | |
| DEFINITION Receptor protein-tyrosine kinase erbB-2 precursor (p185erbB2) (NEU proto-oncogene) (C-erbB-2) (Tyrosine kinase-type cell surface receptor HER2) (MLN 19). | | | | |
| ACCESSION NO.: P04626 | | | | |
| | | | | |
| melaalcrwg | lllallppga | astqvctgtd | mklrlpaspe | thldmlrhly |
| qgcqvvqgnl | eltylptnas | lsflqdiqev | qgyvliahnq | vrqvplqrlr |
| ivrgtqlfed | nyalavldng | dplnnttpvt | gaspgglrel | qlrslteilk |
| ggvliqrnpq | lcyqdtilwk | difhknnqla | ltlidtnrsr | achpcspmck |
| gsrcwgesse | dcqsltrtvc | aggcarckgp | lptdccheqc | aagctgpkhs |
| dclaclhfnh | sgicelhcpa | lvtyntdtfe | smpnpegryt | fgascvtacp |
| ynylstdvgs | ctlvcplhnq | evtaedgtqr | cekcskpcar | vcyglgmehl |
| revravtsan | iqefagckki | fgslaflpes | fdgdpasnta | plqpeqlqvf |
| etleeitgyl | yisawpdslp | dlsvfqnlqv | irgrilhnga | ysltlqglgi |
| swlglrslre | lgsglalihh | nthlcfvhtv | pwdqlfrnph | qallhtanrp |
| edecvgegla | chqlcarghc | wgpgptqcvn | csqflrgqec | veecrvlqgl |
| preyvnarhc | lpchpecqpq | ngsvtcfgpe | adqcvacahy | kdppfcvarc |
| psgvkpdlsy | mpiwkfpdee | gacqpcpinc | thscvdlddk | gcpaeqrasp |
| ltsiisawg | illwvlgw | fgilikrrqq | kirkytmrrl | lqetelvepl |
| tpsgampnqa | qmrilketel | rkvkvlgsga | fgtvykgiwi | pdgenvkipv |
| aikvlrents | pkankeilde | ayvmagvgsp | yvsrllgicl | tstvqlvtql |
| mpygclldhv | renrgrlgsq | dllnwcmqia | kgmsyledvr | lvhrdlaarn |
| vlvkspnhvk | itdfglarll | dideteyhad | ggkvpikwma | lesilrrrft |
| hqsdvwsygv | tvwelmtfga | kpydgipare | ipdllekger | lpqppictid |
| vymimvkcwm | idsecrprfr | elvsefsrma | rdpqrfwiq | nedlgpaspl |
| dstfyrslle | dddmgdlvda | eeylvpqqgf | fcpdpapgag | gmvhhrhrss |
| strsgggdlt | lglepseeea | prsplapseg | agsdvfdgdl | gmgaakglqs |
| lpthdpsplq | rysedptvpl | psetdgyvap | ltcspqpeyv | nqpdvrpqpp |
| spregplpaa | rpagatlerp | ktlspgkngv | vkdvfafgga | venpeyltpq |
| ggaapqphpp pafspafdnl yywdqdpper gappstfkgt ptaenpeylg | | | | |
| ldvpv | | | | |
| | | | | |

| **SEQ. ID NO. 10** | | | | |
|---|---|---|---|---|
| SIZE: 2384 bp | | | | |
| DEFINITION Homo sapiens tyrosinase (oculocutaneous albinism IA) (TYR), mRNA. | | | | |
| ACCESSION NO.: NM_000372 | | | | |
| | | | | |
| tattgagttc | ttcaaacatt | gtagcctctt | tatggtctct | gagaaataac |
| taccttaaac | ccataatctt | taatacttcc | taaactttct | taataagaga |
| agctctattc | ctgacactac | ctctcatttg | caaggtcaaa | tcatcattag |
| ttttgtagtc | tattaactgg | gtttgcttag | gtcaggcatt | attattacta |
| accttattgt | taatattcta | accataagaa | ttaaactatt | aatggtgaat |
| agagtttttc | actttaacat | aggcctatcc | cactggtggg | atacgagcca |
| attcgaaaga | aaagtcagtc | atgtgctttt | cagaggatga | aagcttaaga |
| taaagactaa | aagtgtttga | tgctggaggt | gggagtggta | ttatataggt |
| ctcagccaag | acatgtgata | atcactgtag | tagtagctgg | aaagagaaat |
| ctgtgactcc | aattagccag | ttcctgcaga | ccttgtgagg | actagaggaa |
| gaatgctcct | ggctgttttg | tactgcctgc | tgtggagttt | ccagacctcc |
| gctggccatt | tccctagagc | ctgtgtctcc | tctaagaacc | tgatggagaa |
| ggaatgctgt | ccaccgtgga | gcggggacag | gagtccctgt | ggccagcttt |
| caggcagagg | ttcctgtcag | aatatccttc | tgtccaatgc | accacttggg |
| cctcaatttc | ccttcacagg | ggtggatgac | cgggagtcgt | ggccttccgt |
| cttttataat | aggacctgcc | agtgctctgg | caacttcatg | ggattcaact |
| gtggaaactg | caagtttggc | ttttggggac | caaactgcac | agagagacga |
| ctcttggtga | gaagaaacat | cttcgatttg | agtgccccag | agaaggacaa |
| attttttgcc | tacctcactt | tagcaaagca | taccatcagc | tcagactatg |
| tcatccccat | agggacctat | ggccaaatga | aaaatggatc | aacacccatg |
| tttaacgaca | tcaatattta | tgacctcttt | gtctggatgc | attattatgt |
| gtcaatggat | gcactgcttg | ggggatctga | aatctggaga | gacattgatt |
| ttgcccatga | agcaccagct | tttctgcctt | ggcatagact | cttcttgttg |
| cggtgggaac | aagaaatcca | gaagctgaca | ggagatgaaa | acttcactat |
| tccatattgg | gactggcggg | atgcagaaaa | gtgtgacatt | tgcacagatg |
| agtacatggg | aggtcagcac | cccacaaatc | ctaacttact | cagcccagca |
| tcattcttct | cctcttggca | gattgtctgt | agccgattgg | aggagtacaa |
| cagccatcag | tctttatgca | atggaacgcc | cgagggacct | ttacggcgta |
| atcctggaaa | ccatgacaaa | tccagaaccc | caaggctccc | ctcttcagct |
| gatgtagaat | tttgcctgag | tttgacccaa | tatgaatctg | gttccatgga |
| taaagctgcc | aatttcagct | ttagaaatac | actggaagga | tttgctagtc |
| cacttactgg | gatagcggat | gcctctcaaa | gcagcatgca | caatgccttg |
| cacatctata | tgaatggaac | aatgtcccag | gtacagggat | ctgccaacga |
| tcctatcttc | cttcttcacc | atgcatttgt | tgacagtatt | tttgagcagt |
| ggctccgaag | gcaccgtcct | cttcaagaag | tttatccaga | agccaatgca |
| cccattggac | ataaccggga | atcctacatg | gttcctttta | taccactgta |
| cagaaatggt | gatttcttta | tttcatccaa | agatctgggc | tatgactata |
| gctatctaca | agattcagac | ccagactctt | ttcaagacta | cattaagtcc |
| tatttggaac | aagcgagtcg | gatctggtca | tggctccttg | gggcggcgat |
| ggtaggggcc | gtcctcactg | ccctgctggc | agggcttgtg | agcttgctgt |
| gtcgtcacaa | gagaaagcag | cttcctgaag | aaaagcagcc | actcctcatg |
| gagaaagagg | attaccacag | cttgtatcag | agccatttat | aaaaggctta |
| ggcaatagag | tagggccaaa | aagcctgacc | tcactctaac | tcaaagtaat |
| gtccaggttc | ccagagaata | tctgctggta | tttttctgta | aagaccattt |
| gcaaaattgt | aacctaatac | aaagtgtagc | cttcttccaa | ctcaggtaga |
| acacacctgt | ctttgtcttg | ctgttttcac | tcagcccttt | taacattttc |
| ccctaagccc | atatgtctaa | ggaaaggatg | ctatttggta | atgaggaact |
| gttatttgta tgtgaattaa agtgctctta tttt | | | | |
| | | | | |

| **SEQ . ID NO. 11** | | | | |
|---|---|---|---|---|
| SIZE: 1524 bp | | | | |
| DEFINITION Human melanoma antigen recognized by T-cells (MART-1) mRNA. | | | | |
| ACCESSION NO.: U06452 | | | | |
| | | | | |
| agcagacaga | ggactctcat | taaggaaggt | gtcctgtgcc | ctgaccctac |
| aagatgccaa | gagaagatgc | tcacttcatc | tatggttacc | ccaagaaggg |
| gcacggccac | tcttacacca | cggctgaaga | ggccgctggg | atcggcatcc |
| tgacagtgat | cctgggagtc | ttactgctca | tcggctgttg | gtattgtaga |
| agacgaaatg | gatacagagc | cttgatggat | aaaagtcttc | atgttggcac |
| tcaatgtgcc | ttaacaagaa | gatgcccaca | agaagggttt | gatcatcggg |
| acagcaaagt | gtctcttcaa | gagaaaaact | gtgaacctgt | ggttcccaat |
| gctccacctg | cttatgagaa | actctctgca | gaacagtcac | caccacctta |
| ttcaccttaa | gagccagcga | gacacctgag | acatgctgaa | attatttctc |
| tcacactttt | gcttgaattt | aatacagaca | tctaatgttc | tcctttggaa |
| tggtgtagga | aaaatgcaag | ccatctctaa | taataagtca | gtgttaaaat |
| tttagtaggt | ccgctagcag | tactaatcat | gtgaggaaat | gatgagaaat |
| attaaattgg | gaaaactcca | tcaataaatg | ttgcaatgca | tgatactatc |
| tgtgccagag | gtaatgttag | taaatccatg | gtgttatttt | ctgagagaca |
| gaattcaagt | gggtattctg | gggccatcca | atttctcttt | acptgaaatt |
| tggctaataa | caaactagtc | aggttttcga | accttgaccg | acatgaactg |
| tacacagaat | tgttccagta | ctatggagtg | ctcacaaagg | atacttttac |
| aggttaagac | aaagggttga | ctggcctatt | tatctgatca | agaacatgtc |
| agcaatgtct | ctttgtgctc | taaaattcta | ttatactaca | ataatatatt |
| gtaaagatcc | tatagctctt | tttttttgag | atggagtttc | gcttttgttg |
| cccaggctgg | agtgcaatgg | cgcgatcttg | gctcaccata | acctccgcct |
| cccaggttca | agcaattctc | ctgccttagc | ctcctgagta | gctgggatta |
| caggcgtgcg | ccactatgcc | tgactaattt | tgtagtttta | gtagagacgg |
| ggtttctcca | tgttggtcag | gctggtctca | aactcctgac | ctcaggtgat |
| ctgcccgcct | cagcctccca | aagtgctgga | attacaggcg | tgagccacca |
| cgcctggctg | gatcctatat | cttaggtaag | acatataacg | cagtctaatt |
| acatttcact | tcaaggctca | atgctattct | aactaatgac | aagtattttc |
| tactaaacca | gaaattggta | gaaggattta | aataagtaaa | agctactatg |
| tactgcctta | gtgctgatgc | ctgtgtactg | ccttaaatgt | acctatggca |
| atttagctct | cttgggttcc | caaatccctc | tcacaagaat | gtgcagaaga |
| aatcataaag gatcagagat tctg | | | | |

| **SEQ. ID NO. 12** | | | | |
|---|---|---|---|---|
| SIZE: 1466 bp | | | | |
| DEFINITION Homo sapiens synovial sarcoma, X breakpoint 2 (SSX2), transcript variant 1, mRNA. | | | | |
| ACCESSION NO.: NM_003147 | | | | |
| | | | | |
| gcatgctctg | actttctctc | tctttcgatt | cttccatact | cagagtacgc |
| acggtctgat | tttctctttg | gattcttcca | aaatcagagt | cagactgctc |
| ccggtgccat | gaacggagac | gacgcctttg | caaggagacc | cacggttggt |
| gctcaaatac | cagagaagat | ccaaaaggcc | ttcgatgata | ttgccaaata |
| cttctctaag | gaagagtggg | aaaagatgaa | agcctcggag | aaaatcttct |
| atgtgtatat | gaagagaaag | tatgaggcta | tgactaaact | aggtttcaag |
| gccaccctcc | cacctttcat | gtgtaataaa | cgggccgaag | acttccaggg |
| gaatgatttg | gataatgacc | ctaaccgtgg | gaatcaggtt | gaacgtcctc |
| agatgacttt | cggcaggctc | cagggaatct | ccccgaagat | catgcccaag |
| aagccagcag | aggaaggaaa | tgattcggag | gaagtgccag | aagcatctgg |
| cccacaaaat | gatgggaaag | agctgtgccc | cccgggaaaa | ccaactacct |
| ctgagaagat | tcacgagaga | tctggaaata | gggaggccca | agaaaaggaa |
| gagagacgcg | gaacagctca | tcggtggagc | agtcagaaca | cacacaacat |
| tggtcgattc | agtttgtcaa | cttctatggg | tgcagttcat | ggtaccccca |
| aaacaattac | acacaacagg | gacccaaaag | gggggaacat | gcctggaccc |
| acagactgcg | tgagagaaaa | cagctggtga | tttatgaaga | gatcagcgac |
| cctgaggaag | atgacgagta | actcccctca | gggatacgac | acatgcccat |
| gatgagaagc | agaacgtggt | gacctttcac | gaacatgggc | atggctgcgg |
| acccctcgtc | atcaggtgca | tagcaagtga | aagcaagtgt | tcacaacagt |
| gaaaagttga | gcgtcatttt | tcttagtgtg | ccaagagttc | gatgttagcg |
| tttacgttgt | aetttcttac | actgtgtcat | tctgttagat | actaacattt |
| tcattgatga | gcaagacata | cttaatgcat | attttggttt | gtgtatccat |
| gcacctacct | tagaaaacaa | gtattgtcgg | ttacctctgc | atggaacagc |
| attaccctcc | tctctcccca | gatgtgacta | ctgagggcag | ttctgagtgt |
| ttaatttcag | attttttcct | ctgcatttac | acacacacgc | acacaaacca |
| caccacacac | acacacacac | acacacacac | acacacacac | acacaccaag |
| taccagtata | agcatctgcc | atctgctttt | cccattgcca | tgcgtcctgg |
| tcaagctccc | ctcactctgt | ttcctggtca | gcatgtactc | ccctcatccg |
| attcccctgt | agcagtcact | gacagttaat | aaacctttgc | aaacgttcaa |
| aaaaaaaaaa aaaaaa | | | | |
| | | | | |

| **SEQ. ID NO. 13** | | | | |
|---|---|---|---|---|
| SIZE: 2653 bp | | | | |
| DEFINITION Homo sapiens folate hydrolase (prostate-specific membrane antigen) 1 (FOLH1), mRNA. | | | | |
| ACCESSION NO.: NM_004476 | | | | |
| | | | | |
| ctcaaaaggg | gccggatttc | cttctcctgg | aggcagatgt | tgcctctctc |
| tctcgctcgg | attggttcag | tgcactctag | aaacactgct | gtggtggaga |
| aactggaccc | caggtctgga | gcgaattcca | gcctgcaggg | ctgataagcg |
| aggcattagt | gagattgaga | gagactttac | cccgccgtgg | tggttggagg |
| gcgcgcagta | gagcagcagc | acaggcgcgg | gtcccgggag | gccggctctg |
| ctcgcgccga | gatgtggaat | ctccttcacg | aaaccgactc | ggctgtggcc |
| accgcgcgcc | gcccgcgctg | gctgtgcgct | ggggcgctgg | tgctggcggg |
| tggcttcttt | ctcctcggct | tcctcttcgg | gtggtttata | aaatcctcca |
| atgaagctac | taacattact | ccaaagcata | atatgaaagc | atttttggat |
| gaattgaaag | ctgagaacat | caagaagttc | ttatataatt | ttacacagat |
| accacattta | gcaggaacag | aacaaaactt | tcagcttgca | aagcaaattc |
| aatcccagtg | gaaagaattt | ggcctggatt | ctgttgagct | agcacattat |
| gatgtcctgt | tgtcctaccc | aaataagact | catcccaact | acatctcaat |
| aattaatgaa | gatggaaatg | agattttcaa | cacatcatta | tttgaaccac |
| ctcctccagg | atatgaaaat | gtttcggata | ttgtaccacc | tttcagtgct |
| ttctctcctc | aaggaatgcc | agagggcgat | ctagtgtatg | ttaactatgc |
| acgaactgaa | gacttcttta | aattggaacg | ggacatgaaa | atcaattgct |
| ctgggaaaat | tgtaattgcc | agatatggga | aagttttcag | aggaaataag |
| gttaaaaatg | cccagctggc | aggggccaaa | ggagtcattc | tctactccga |
| ccctgctgac | tactttgctc | ctggggtgaa | gtcctatcca | gatggttgga |
| atcttcctgg | aggtggtgtc | cagcgtggaa | atatcctaaa | tctgaatggt |
| gcaggagacc | ctctcacacc | aggttaccca | gcaaatgaat | atgcttatag |
| gcgtggaatt | gcagaggctg | ttggtcttcc | aagtattcct | gttcatccaa |
| ttggatacta | tgatgcacag | aagctcctag | aaaaaatggg | tggctcagca |
| ccaccagata | gcagctggag | aggaagtctc | aaagtgccct | acaatgttgg |
| acctggcttt | actggaaact | tttctacaca | aaaagtcaag | atgcacatcc |
| actctaccaa | tgaagtgaca | agaatttaca | atgtgatagg | tactctcaga |
| ggagcagtgg | aaccagacag | atatgtcatt | ctgggaggtc | accgggactc |
| atgggtgttt | ggtggtattg | accctcagag | tggagcagct | gttgttcatg |
| aaattgtgag | gagctttgga | acactgaaaa | aggaagggtg | gagacctaga |
| agaacaattt | tgtttgcaag | ctgggatgca | gaagaatttg | gtcttcttgg |
| ttctactgag | tgggcagagg | agaattcaag | actccttcaa | gagcgtggcg |
| tggcttatat | taatgctgac | tcatctatag | aaggaaacta | cactctgaga |
| gttgattgta | caccgctgat | gtacagcttg | gtacacaacc | taacaaaaga |
| gctgaaaagc | cctgatgaag | gctttgaagg | caaatctctt | tatgaaagtt |
| ggactaaaaa | aagtccttcc | ccagagttca | gtggcatgcc | caggataagc |
| aaattgggat | ctggaaatga | ttttgaggtg | ttcttccaac | gacttggaat |
| tgcttcaggc | agagcacggt | atactaaaaa | ttgggaaaca | aacaaattca |
| gcggctatcc | actgtatcac | agtgtctatg | aaacatatga | gttggtggaa |
| aagttttatg | atccaatgtt | taaatatcac | ctcactgtgg | cccaggttcg |
| aggagggatg | gtgtttgagc | tagccaattc | catagtgctc | ccttttgatt |
| gtcgagatta | tgctgtagtt | ttaagaaagt | atgctgacaa | aatctacagt |
| atttctatga | aacatccaca | ggaaatgaag | acatacagtg | tatcatttga |
| ttcacttttt | tctgcagtaa | agaattttac | agaaattgct | tccaagttca |
| gtgagagact | ccaggacttt | gacaaaagca | acccaatagt | attaagaatg |
| atgaatgatc | aactcatgtt | tctggaaaga | gcatttattg | atccattagg |
| gttaccagac | aggccttttt | ataggcatgt | catctatgct | ccaagcagcc |
| acaacaagta | tgcaggggag | tcattcccag | gaatttatga | tgctctgttt |
| gatattgaaa | gcaaagtgga | cccttccaag | gcctggggag | aagtgaagag |
| acagatttat | gttgcagcct | tcacagtgca | ggcagctgca | gagactttga |
| gtgaagtagc | ctaagaggat | tctttagaga | atccgtattg | aatttgtgtg |
| gtatgtcact | cagaaagaat | cgtaatgggt | atattgataa | attttaaaat |
| tggtatattt gaaataaagt tgaatattat atataaaaaa aaaaaaaaaa aaa | | | | |
| | | | | |

| **SEQ. ID NO. 14** | | | | |
|---|---|---|---|---|
| SIZE: 2420 bp | | | | |
| DEFINITION Human antigen (MAGE-1) gene, complete cds. | | | | |
| ACCESSION NO.: M77481 | | | | |
| | | | | |
| ggatccaggc | cctgccagga | aaaatataag | ggccctgcgt | gagaacagag |
| ggggtcatcc | actgcatgag | agtggggatg | tcacagagtc | cagcccaccc |
| tcctggtagc | actgagaagc | cagggctgtg | cttgcggtct | gcaccctgag |
| ggcccgtgga | ttcctcttcc | tggagctcca | ggaaccaggc | agtgaggcct |
| tggtctgaga | cagtatcctc | aggtcacaga | gcagaggatg | cacagggtgt |
| gccagcagtg | aatgtttgcc | ctgaatgcac | accaagggcc | ccacctgcca |
| caggacacat | aggactccac | agagtctggc | ctcacctccc | tactgtcagt |
| cctgtagaat | cgacctctgc | tggccggctg | taccctgagt | accctctcac |
| ttcctccttc | aggttttcag | gggacaggcc | aacccagagg | acaggattcc |
| ctggaggcca | cagaggagca | ccaaggagaa | gatctgtaag | taggcctttg |
| ttagagtctc | caaggttcag | ttctcagctg | aggcctctca | cacactccct |
| ctctccccag | gcctgtgggt | cttcattgcc | cagctcctgc | ccacactcct |
| gcctgctgcc | ctgacgagag | tcatcatgtc | tcttgagcag | aggagtctgc |
| actgcaagcc | tgaggaagcc | cttgaggccc | aacaagaggc | cctgggcctg |
| gtgtgtgtgc | aggctgccac | ctcctcctcc | tctcctctgg | tcctgggcac |
| cctggaggag | gtgcccactg | ctgggtcaac | agatcctccc | cagagtcctc |
| agggagcctc | cgcctttccc | actaccatca | acttcactcg | acagaggcaa |
| cccagtgagg | gttccagcag | ccgtgaagag | gaggggccaa | gcacctcttg |
| tatcctggag | tccttgttcc | gagcagtaat | cactaagaag | gtggctgatt |
| tggttggttt | tctgctcctc | aaatatcgag | ccagggagcc | agtcacaaag |
| gcagaaatgc | tggagagtgt | catcaaaaat | tacaagcact | gttttcctga |
| gatcttcggc | aaagcctctg | agtccttgca | gctggtcttt | ggcattgacg |
| tgaaggaagc | agaccccacc | ggccactcct | atgtccttgt | cacctgccta |
| ggtctctcct | atgatggcct | gctgggtgat | aatcagatca | tgcccaagac |
| aggcttcctg | ataattgtcc | tggtcatgat | tgcaatggag | ggcggccatg |
| ctcctgagga | ggaaatctgg | gaggagctga | gtgtgatgga | ggtgtatgat |
| gggagggagc | acagtgccta | tggggagccc | aggaagctgc | tcacccaaga |
| tttggtgcag | gaaaagtacc | tggagtaccg | gcaggtgccg | gacagtgatc |
| ccgcacgcta | tgagttcctg | tggggtccaa | gggccctcgc | tgaaaccagc |
| tatgtgaaag | tccttgagta | tgtgatcaag | gtcagtgcaa | gagttcgctt |
| tttcttccca | tccctgcgtg | aagcagcttt | gagagaggag | gaagagggag |
| tctgagcatg | agttgcagcc | aaggccagtg | ggagggggac | tgggccagtg |
| caccttccag | ggccgcgtcc | agcagcttcc | cctgcctcgt | gtgacatgag |
| gcccattctt | cactctgaag | agagcggtca | gtgttctcag | tagtaggttt |
| ctgttctatt | gggtgacttg | gagatttatc | tttgttctct | tttggaattg |
| ttcaaatgtt | tttttttaag | ggatggttga | atgaacttca | gcatccaagt |
| ttatgaatga | cagcagtcac | acagttctgt | gtatatagtt | taagggtaag |
| agtcttgtgt | tttattcaga | ttgggaaatc | cattctattt | tgtgaattgg |
| gataataaca | gcagtggaat | aagtacttag | aaatgtgaaa | aatgagcagt |
| aaaatagatg | agataaagaa | ctaaagaaat | taagagatag | tcaattcttg |
| ccttatacct | cagtctattc | tgtaaaattt | ttaaagatat | atgcatacct |
| ggatttcctt | ggcttctttg | agaatgtaag | agaaattaaa | tctgaataaa |
| gaattcttcc | tgttcactgg | ctcttttctt | ctccatgcac | tgagcatctg |
| ctttttggaa | ggccctgggt | tagtagtgga | gatgctaagg | taagccagac |
| tcatacccac | ccatagggtc | gtagagtcta | ggagctgcag | tcacgtaatc |
| gaggtggcaa | gatgtcctct | aaagatgtag | ggaaaagtga | gagaggggtg |
| agggtgtggg | gctccgggtg | agagtggtgg | agtgtcaatg | ccctgagctg |
| gggcattttg | ggctttggga | aactgcagtt | ccttctgggg | gagctgattg |
| taatgatctt gggtggatcc | | | | |
| | | | | |

| **SEQ. ID NO. 15** | | | | |
|---|---|---|---|---|
| SIZE: 4204 bp DNA linear PRI 07-APR-1994 | | | | |
| DEFINITION Human MAGE-3 antigen (MAGE-3) gene, complete cds. | | | | |
| ACCESSION NO.: U03735 | | | | |
| | | | | |
| acgcaggcag | tgatgtcacc | cagaccacac | cccttccccc | aatgccactt |
| cagggggtac | tcagagtcag | agacttggtc | tgaggggagc | agaagcaatc |
| tgcagaggat | ggcggtccag | gctcagccag | gcatcaactt | caggaccctg |
| agggatgacc | gaaggccccg | cccacccacc | cccaactccc | ccgaccccac |
| caggatctac | agcctcagga | cccccgtccc | aatccttacc | ccttgcccca |
| tcaccatctt | catgcttacc | tccaccccca | tccgatcccc | atccaggcag |
| aatccagttc | cacccctgcc | cggaacccag | ggtagtaccg | ttgccaggat |
| gtgacgccac | tgacttgcgc | attggaggtc | agaagaccgc | gagattctcg |
| ccctgagcaa | cgagcgacgg | cctgacgtcg | gcggagggaa | gccggcccag |
| gctcggtgag | gaggcaaggt | aagacgctga | gggaggactg | aggcgggcct |
| cacctcagac | agagggcctc | aaataatcca | gtgctgcctc | tgctgccggg |
| cctgggccac | cccgcagggg | aagacttcca | ggctgggtcg | ccactacctc |
| accccgccga | cccccgccgc | tttagccacg | gggaactctg | gggacagagc |
| ttaatgtggc | cagggcaggg | ctggttagaa | gaggtcaggg | cccacgctgt |
| ggcaggaatc | aaggtcagga | ccccgagagg | gaactgaggg | cagcctaacc |
| accaccctca | ccaccattcc | cgtcccccaa | cacccaaccc | cacccccatc |
| ccccattccc | atccccaccc | ccacccctat | cctggcagaa | tccgggcttt |
| gcccctggta | tcaagtcacg | gaagctccgg | gaatggcggc | caggcacgtg |
| agtcctgagg | ttcacatcta | cggctaaggg | agggaagggg | ttcggtatcg |
| cgagtatggc | cgttgggagg | cagcgaaagg | gcccaggcct | cctggaagac |
| agtggagtcc | tgaggggacc | cagcatgcca | ggacaggggg | cccactgtac |
| ccctgtctca | aaccgaggca | ccttttcatt | cggctacggg | aatcctaggg |
| atgcagaccc | acttcagcag | ggggttgggg | cccagccctg | cgaggagtca |
| tggggaggaa | gaagagggag | gactgagggg | accttggagt | ccagatcagt |
| ggcaaccttg | ggctggggga | tgctgggcac | agtggccaaa | tgtgctctgt |
| gctcattgcg | ccttcagggt | gaccagagag | ttgagggctg | tggtctgaag |
| agtgggactt | caggtcagca | gagggaggaa | tcccaggatc | tgcagggccc |
| aaggtgtacc | cccaaggggc | ccctatgtgg | tggacagatg | cagtggtcct |
| aggatctgcc | aagcatccag | gtgaagagac | tgagggagga | ttgagggtac |
| ccctgggaca | gaatgcggac | tgggggcccc | ataaaaatct | gccctgctcc |
| tgctgttacc | tcagagagcc | tgggcagggc | tgtcagctga | ggtccctcca |
| ttatcctagg | atcactgatg | tcagggaagg | ggaagccttg | gtctgagggg |
| gctgcactca | gggcagtaga | gggaggctct | cagaccctac | taggagtgga |
| ggtgaggacc | aagcagtctc | ctcacccagg | gtacatggac | ttcaataaat |
| ttggacatct | ctcgttgtcc | tttccgggag | gacctgggaa | tgtatggcca |
| gatgtgggtc | ccctcatgtt | tttctgtacc | atatcaggta | tgtgagttct |
| tgacatgaga | gattctcagg | ccagcagaag | ggagggatta | ggccctataa |
| ggagaaaggt | gagggccctg | agtgagcaca | gaggggatcc | tccaccccag |
| tagagtgggg | acctcacaga | gtctggccaa | ccctcctgac | agttctggga |
| atccgtggct | gcgtttgctg | tctgcacatt | gggggcccgt | ggattcctct |
| cccaggaatc | aggagctcca | ggaacaaggc | agtgaggact | tggtctgagg |
| cagtgtcctc | aggtcacaga | gtagaggggg | ctcagatagt | gccaacggtg |
| aaggtttgcc | ttggattcaa | accaagggcc | ccacctgccc | cagaacacat |
| ggactccaga | gcgcctggcc | tcaccctcaa | tactttcagt | cctgcagcct |
| cagcatgcgc | tggccggatg | taccctgagg | tgccctctca | cttcctcctt |
| caggttctga | ggggacaggc | tgacctggag | gaccagaggc | ccccggagga |
| gcactgaagg | agaagatctg | taagtaagcc | tttgttagag | cctccaaggt |
| tccattcagt | actcagctga | ggtctctcac | atgctccctc | tctccccagg |
| ccagtgggtc | tccattgccc | agctcctgcc | cacactcccg | cctgttgccc |
| tgaccagagt | catcatgcct | cttgagcaga | ggagtcagca | ctgcaagcct |
| gaagaaggcc | ttgaggcccg | aggagaggcc | ctgggcctgg | tgggtgcgca |
| ggctcctgct | actgaggagc | aggaggctgc | ctcctcctct | tctactctag |
| ttgaagtcac | cctgggggag | gtgcctgctg | ccgagtcacc | agatcctccc |
| cagagtcctc | agggagcctc | cagcctcccc | actaccatga | actaccctct |
| ctggagccaa | tcctatgagg | actccagcaa | ccaagaagag | gaggggccaa |
| gcaccttccc | tgacctggag | tccgagttcc | aagcagcact | cagtaggaag |
| gtggccgagt | tggttcattt | tctgctcctc | aagtatcgag | ccagggagcc |
| ggtcacaaag | gcagaaatgc | tggggagtgt | cgtcggaaat | tggcagtatt |
| tctttcctgt | gatcttcagc | aaagcttcca | gttccttgca | gctggtcttt |
| ggcatcgagc | tgatggaagt | ggaccccatc | ggccacttgt | acatctttgc |
| cacctgcctg | ggcctctcct | acgatggcct | gctgggtgac | aatcagatca |
| tgcccaaggc | aggcctcctg | ataatcgtcc | tggccataat | cgcaagagag |
| ggcgactgtg | cccctgagga | gaaaatctgg | gaggagctga | gtgtgttaga |
| ggtgtttgag | gggagggaag | acagtatctt | gggggatccc | aagaagctgc |
| tcacccaaca | tttcgtgcag | gaaaactacc | tggagtaccg | gcaggtcccc |
| ggcagtgatc | ctgcatgtta | tgaattcctg | tggggtccaa | gggccctcgt |
| tgaaaccagc | tatgtgaaag | tcctgcacca | tatggtaaag | atcagtggag |
| gacctcacat | ttcctaccca | cccctgcatg | agtgggtttt | gagagagggg |
| gaagagtgag | tctgagcacg | agttgcagcc | agggccagtg | ggagggggtc |
| tgggccagtg | caccttccgg | ggccgcatcc | cttagtttcc | actgcctcct |
| gtgacgtgag | gcccattctt | cactctttga | agcgagcagt | cagcattctt |
| agtagtgggt | ttctgttctg | ttggatgact | ttgagattat | tctttgtttc |
| ctgttggagt | tgttcaaatg | ttccttttaa | cggatggttg | aatgagcgtc |
| agcatccagg | tttatgaatg | acagtagtca | cacatagtgc | tgtttatata |
| gtttaggagt | aagagtcttg | ttttttactc | aaattgggaa | atccattcca |
| ttttgtgaat | tgtgacataa | taatagcagt | ggtaaaagta | tttgcttaaa |
| attgtgagcg | aattagcaat | aacatacatg | agataactca | agaaatcaaa |
| agatagttga | ttcttgcctt | gtacctcaat | ctattctgta | aaattaaaca |
| aatatgcaaa | ccaggatttc | cttgacttct | ttgagaatgc | aagcgaaatt |
| aaatctgaat | aaataattct | tcctcttcac | tggctcgttt | cttttccgtt |
| cactcagcat | ctgctctgtg | ggaggccctg | ggttagtagt | ggggatgcta |
| aggtaagcca | gactcacgcc | tacccatagg | gctgtagagc | ctaggacctg |
| cagtcatata | attaaggtgg | tgagaagtcc | tgtaagatgt | agaggaaatg |
| taagagaggg gtgagggtgt ggcgctccgg gtgagagtag tggagtgtca gtgc | | | | |
| | | | | |

| **SEQ. ID NO. 16** | | | | |
|---|---|---|---|---|
| SIZE: 752 bp | | | | |
| DEFINITION Human autoimmunogenic cancer/testis antigen NY-ESO-1 mRNA, complete cds. | | | | |
| ACCESSION NO.: U87459 | | | | |
| | | | | |
| atcctcgtgg | gccctgacct | tctctctgag | agccgggcag | aggctccgga |
| gccatgcagg | ccgaaggccg | gggcacaggg | ggttcgacgg | gcgatgctga |
| tggcccagga | ggccctggca | ttcctgatgg | cccagggggc | aatgctggcg |
| gcccaggaga | ggcgggtgcc | acgggcggca | gaggtccccg | gggcacaggg |
| gcagcaaggg | cctcggggcc | gggaggaggc | gccccgcggg | gtccgcatgg |
| cggcgcggct | tcagggctga | atggatgctg | cagatgcggg | gccagggggc |
| cggagagccg | cctgcttgag | ttctacctcg | ccatgccttt | cgcgacaccc |
| atggaagcag | agctggcccg | caggagcctg | gcccaggatg | ccccaccgct |
| tcccgtgcca | ggggtgcttc | tgaaggagtt | cactgtgtcc | ggcaacatac |
| tgactatccg | actgactgct | gcagaccacc | gccaactgca | gctctccatc |
| agctcctgtc | tccagcagct | ttccctgttg | atgtggatca | cgcagtgctt |
| tctgcccgtg | tttttggctc | agcctccctc | agggcagagg | cgctaagccc |
| agcctggcgc | cccttcctag | gtcatgcctc | ctcccctagg | gaatggtccc |
| agcacgagtg | gccagttcat | tgtgggggcc | tgattgtttg | tcgctggagg |
| aggacggctt acatgtttgt ttctgtagaa aataaaactg agctacgaaa aa | | | | |
| | | | | |

| **SEQ. ID NO. 17** | | | | |
|---|---|---|---|---|
| SIZE: 2148 bp | | | | |
| DEFINITION Homo sapiens preferentially expressed antigen in melanoma (PRAME), mRNA. | | | | |
| ACCESSION NO.: NM_006115 | | | | |
| | | | | |
| gcttcagggt | acagctcccc | cgcagccaga | agccgggcct | gcagcgcctc |
| agcaccgctc | cgggacaccc | cacccgcttc | ccaggcgtga | cctgtcaaca |
| gcaacttcgc | ggtgtggtga | actctctgag | gaaaaaccat | tttgattatt |
| actctcagac | gtgcgtggca | acaagtgact | gagacctaga | aatccaagcg |
| ttggaggtcc | tgaggccagc | ctaagtcgct | tcaaaatgga | acgaaggcgt |
| ttgtggggtt | ccattcagag | ccgatacatc | agcatgagtg | tgtggacaag |
| cccacggaga | cttgtggagc | tggcagggca | gagcctgctg | aaggatgagg |
| ccctggccat | tgccgccctg | gagttgctgc | ccagggagct | cttcccgcca |
| ctcttcatgg | cagcctttga | cgggagacac | agccagaccc | tgaaggcaat |
| ggtgcaggcc | tggcccttca | cctgcctccc | tctgggagtg | ctgatgaagg |
| gacaacatct | tcacctggag | accttcaaag | ctgtgcttga | tggacttgat |
| gtgctccttg | cccaggaggt | tcgccccagg | aggtggaaac | ttcaagtgct |
| ggatttacgg | aagaactctc | atcaggactt | ctggactgta | tggtctggaa |
| acagggccag | tctgtactca | tttccagagc | cagaagcagc | tcagcccatg |
| acaaagaagc | gaaaagtaga | tggtttgagc | acagaggcag | agcagccctt |
| cattccagta | gaggtgctcg | tagacctgtt | cctcaaggaa | ggtgcctgtg |
| atgaattgtt | ctcctacctc | attgagaaag | tgaagcgaaa | gaaaaatgta |
| ctacgcctgt | gctgtaagaa | gctgaagatt | tttgcaatgc | ccatgcagga |
| tatcaagatg | atcctgaaaa | tggtgcagct | ggactctatt | gaagatttgg |
| aagtgacttg | tacctggaag | ctacccacct | tggcgaaatt | ttctccttac |
| ctgggccaga | tgattaatct | gcgtagactc | ctcctctccc | acatccatgc |
| atcttcctac | atttccccgg | agaaggaaga | gcagtatatc | gcccagttca |
| cctctcagtt | cctcagtctg | cagtgcctgc | aggctctcta | tgtggactct |
| ttatttttcc | ttagaggccg | cctggatcag | ttgctcaggc | acgtgatgaa |
| ccccttggaa | accctctcaa | taactaactg | ccggctttcg | gaaggggatg |
| tgatgcatct | gtcccagagt | cccagcgtca | gtcagctaag | tgtcctgagt |
| ctaagtgggg | tcatgctgac | cgatgtaagt | cccgagcccc | tccaagctct |
| gctggagaga | gcctctgcca | ccctccagga | cctggtcttt | gatgagtgtg |
| ggatcacgga | tgatcagctc | cttgccctcc | tgccttccct | gagccactgc |
| tcccagctta | caaccttaag | cttctacggg | aattccatct | ccatatctgc |
| cttgcagagt | ctcctgcagc | acctcatcgg | gctgagcaat | ctgacccacg |
| tgctgtatcc | tgtccccctg | gagagttatg | aggacatcca | tggtaccctc |
| cacctggaga | ggcttgccta | tctgcatgcc | aggctcaggg | agttgctgtg |
| tgagttgggg | cggcccagca | tggtctggct | tagtgccaac | ccctgtcctc |
| actgtgggga | cagaaccttc | tatgacccgg | agcccatcct | gtgcccctgt |
| ttcatgccta | actagctggg | tgcacatatc | aaatgcttca | ttctgcatac |
| ttggacacta | aagccaggat | gtgcatgcat | cttgaagcaa | caaagcagcc |
| acagtttcag | acaaatgttc | agtgtgagtg | aggaaaacat | gttcagtgag |
| gaaaaaacat | tcagacaaat | gttcagtgag | gaaaaaaagg | ggaagttggg |
| gataggcaga | tgttgacttg | aggagttaat | gtgatctttg | gggagataca |
| tcttatagag | ttagaaatag | aatctgaatt | tctaaaggga | gattctggct |
| tgggaagtac | atgtaggagt | taatccctgt | gtagactgtt | gtaaagaaac |
| tgttgaaaat aaagagaagc aatgtgaagc aaaaaaaaaa aaaaaaaa | | | | |
| | | | | |

| **SEQ. ID NO. 18** | | | | |
|---|---|---|---|---|
| SIZE: 4530 bp | | | | |
| DEFINITION Human tyrosine kinase-type receptor (HER2) mRNA, complete cds. | | | | |
| ACCESSION NO.: M11730 | | | | |
| | | | | |
| aattctcgag | ctcgtcgacc | ggtcgacgag | ctcgagggtc | gacgagctcg |
| agggcgcgcg | cccggccccc | acccctcgca | gcaccccgcg | ccccgcgccc |
| tcccagccgg | gtccagccgg | agccatgggg | ccggagccgc | agtgagcacc |
| atggagctgg | cggccttgtg | ccgctggggg | ctcctcctcg | ccctcttgcc |
| ccccggagcc | gcgagcaccc | aagtgtgcac | cggcacagac | atgaagctgc |
| ggctccctgc | cagtcccgag | acccacctgg | acatgctccg | ccacctctac |
| cagggctgcc | aggtggtgca | gggaaacctg | gaactcacct | acctgcccac |
| caatgccagc | ctgtccttcc | tgcaggatat | ccaggaggtg | cagggctacg |
| tgctcatcgc | tcacaaccaa | gtgaggcagg | tcccactgca | gaggctgcgg |
| attgtgcgag | gcacccagct | ctttgaggac | aactatgccc | tggccgtgct |
| agacaatgga | gacccgctga | acaataccac | ccctgtcaca | ggggcctccc |
| caggaggcct | gcgggagctg | cagcttcgaa | gcctcacaga | gatcttgaaa |
| ggaggggtct | tgatccagcg | gaacccccag | ctctgctacc | aggacacgat |
| tttgtggaag | gacatcttcc | acaagaacaa | ccagctggct | ctcacactga |
| tagacaccaa | ccgctctcgg | gcctgccacc | cctgttctcc | gatgtgtaag |
| ggctcccgct | gctggggaga | gagttctgag | gattgtcaga | gcctgacgcg |
| cactgtctgt | gccggtggct | gtgcccgctg | caaggggcca | ctgcccactg |
| actgctgcca | tgagcagtgt | gctgccggct | gcacgggccc | caagcactct |
| gactgcctgg | cctgcctcca | cttcaaccac | agtggcatct | gtgagctgca |
| ctgcccagcc | ctggtcacct | acaacacaga | cacgtttgag | tccatgccca |
| atcccgaggg | ccggtataca | ttcggcgcca | gctgtgtgac | tgcctgtccc |
| tacaactacc | tttctacgga | cgtgggatcc | tgcaccctcg | tctgccccct |
| gcacaaccaa | gaggtgacag | cagaggatgg | aacacagcgg | tgtgagaagt |
| gcagcaagcc | ctgtgcccga | gtgtgctatg | gtctgggcat | ggagcacttg |
| cgagaggtga | gggcagttac | cagtgccaat | atccaggagt | ttgctggctg |
| caagaagatc | tttgggagcc | tggcatttct | gccggagagc | tttgatgggg |
| acccagcctc | caacactgcc | ccgctccagc | cagagcagct | ccaagtgttt |
| gagactctgg | aagagatcac | aggttaccta | tacatctcag | catggccgga |
| cagcctgcct | gacctcagcg | tcttccagaa | cctgcaagta | atccggggac |
| gaattctgca | caatggcgcc | tactcgctga | ccctgcaagg | gctgggcatc |
| agctggctgg | ggctgcgctc | actgagggaa | ctgggcagtg | gactggccct |
| catccaccat | aacacccacc | tctgcttcgt | gcacacggtg | ccctgggacc |
| agctctttcg | gaacccgcac | caagctctgc | tccacactgc | caaccggcca |
| gaggacgagt | gtgtgggcga | gggcctggcc | tgccaccagc | tgtgcgcccg |
| agggcactgc | tggggtccag | ggcccaccca | gtgtgtcaac | tgcagccagt |
| tccttcgggg | ccaggagtgc | gtggaggaat | gccgagtact | gcaggggctc |
| cccagggagt | atgtgaatgc | caggcactgt | ttgccgtgcc | accctgagtg |
| tcagccccag | aatggctcag | tgacctgttt | tggaccggag | gctgaccagt |
| gtgtggcctg | tgcccactat | aaggaccctc | ccttctgcgt | ggcccgctgc |
| cccagcggtg | tgaaacctga | cctctcctac | atgcccatct | ggaagtttcc |
| agatgaggag | ggcgcatgcc | agccttgccc | catcaactgc | acccactcct |
| gtgtggacct | ggatgacaag | ggctgccccg | ccgagcagag | agccagccct |
| ctgacgtcca | tcgtctctgc | ggtggttggc | attctgctgg | tcgtggtctt |
| gggggtggtc | tttgggatcc | tcatcaagcg | acggcagcag | aagatccgga |
| agtacacgat | gcggagactg | ctgcaggaaa | cggagctggt | ggagccgctg |
| acacctagcg | gagcgatgcc | caaccaggcg | cagatgcgga | tcctgaaaga |
| gacggagctg | aggaaggtga | aggtgcttgg | atctggcgct | tttggcacag |
| tctacaaggg | catctggatc | cctgatgggg | agaatgtgaa | aattccagtg |
| gccatcaaag | tgttgaggga | aaacacatcc | cccaaagcca | acaaagaaat |
| cttagacgaa | gcatacgtga | tggctggtgt | gggctcccca | tatgtctccc |
| gccttctggg | catctgcctg | acatccacgg | tgcagctggt | gacacagctt |
| atgccctatg | gctgcctctt | agaccatgtc | cgggaaaacc | gcggacgcct |
| gggctcccag | gacctgctga | actggtgtat | gcagattgcc | aaggggatga |
| gctacctgga | ggatgtgcgg | ctcgtacaca | gggacttggc | cgctcggaac |
| gtgctggtca | agagtcccaa | ccatgtcaaa | attacagact | tcgggctggc |
| tcggctgctg | gacattgacg | agacagagta | ccatgcagat | gggggcaagg |
| tgcccatcaa | gtggatggcg | ctggagtcca | ttctccgccg | gcggttcacc |
| caccagagtg | atgtgtggag | ttatggtgtg | actgtgtggg | agctgatgac |
| ttttggggcc | aaaccttacg | atgggatccc | agcccgggag | atccctgacc |
| tgctggaaaa | gggggagcgg | ctgccccagc | cccccatctg | caccattgat |
| gtctacatga | tcatggtcaa | atgttggatg | attgactctg | aatgtcggcc |
| aagattccgg | gagttggtgt | ctgaattctc | ccgcatggcc | agggaccccc |
| agcgctttgt | ggtcatccag | aatgaggact | tgggcccagc | cagtcccttg |
| gacagcacct | tctaccgctc | actgctggag | gacgatgaca | tgggggacct |
| ggtggatgct | gaggagtatc | tggtacccca | gcagggcttc | ttctgtccag |
| accctgcccc | gggcgctggg | ggcatggtcc | accacaggca | ccgcagctca |
| tctaccagga | gtggcggtgg | ggacctgaca | ctagggctgg | agccctctga |
| agaggaggcc | cccaggtctc | cactggcacc | ctccgaaggg | gctggctccg |
| atgtatttga | tggtgacctg | ggaatggggg | cagccaaggg | gctgcaaagc |
| ctccccacac | atgaccccag | ccctctacag | cggtacagtg | aggaccccac |
| agtacccctg | ccctctgaga | ctgatggcta | cgttgccccc | ctgacctgca |
| gcccccagcc | tgaatatgtg | aaccagccag | atgttcggcc | ccagccccct |
| tcgccccgag | agggccctct | gcctgctgcc | cgacctgctg | gtgccactct |
| ggaaagggcc | aagactctct | ccccagggaa | gaatggggtc | gtcaaagacg |
| tttttgcctt | tgggggtgcc | gtggagaacc | ccgagtactt | gacaccccag |
| ggaggagctg | cccctcagcc | ccaccctcct | cctgccttca | gcccagcctt |
| cgacaacctc | tattactggg | accaggaccc | accagagcgg | ggggctccac |
| ccagcacctt | caaagggaca | cctacggcag | agaacccaga | gtacctgggt |
| ctggacgtgc | cagtgtgaac | cagaaggcca | agtccgcaga | agccctgatg |
| tgtcctcagg | gagcagggaa | ggcctgactt | ctgctggcat | caagaggtgg |
| gagggccctc | cgaccacttc | caggggaacc | tgccatgcca | ggaacctgtc |
| ctaaggaacc | ttccttcctg | cttgagttcc | cagatggctg | gaaggggtcc |
| agcctcgttg | gaagaggaac | agcactgggg | agtctttgtg | gattctgagg |
| ccctgcccaa | tgagactcta | gggtccagtg | gatgccacag | cccagcttgg |
| ccctttcctt | ccagatcctg | ggtactgaaa | gccttaggga | agctggcctg |
| agaggggaag | cggccctaag | ggagtgtcta | agaacaaaag | cgacccattc |
| agagactgtc | cctgaaacct | agtactgccc | cccatgagga | aggaacagca |
| atggtgtcag | tatccaggct | ttgtacagag | tgcttttctg | tttagttttt |
| actttttttg | ttttgttttt | ttaaagacga | aataaagacc | caggggagaa |
| tgggtgttgt | atggggaggc | aagtgtgggg | ggtccttctc | cacacccact |
| ttgtccattt gcaaatatat tttggaaaac | | | | |

## Claims

1. Use of an immunogenic composition which immunizes against PRAME and at least one other tumor-associated antigen for the manufacture of a medicament for the treatment of ovarian or colorectal cancer, wherein the at least one other tumor-associated antigen is selected from the group consisting of SSX-2, NY-ESO-1, and PSMA.

2. Use of Claim 1, wherein the at least one other tumor-associated antigen comprises NY-ESO-1 and SSX-2.

3. Use of Claim 1, wherein the at least one other tumor-associated antigen comprises NY-ESO-1, SSX-2, and PSMA.

4. Use of Claim 1, wherein a cytolytic T cell response is induced.

5. Use of Claim 1, wherein the immunogenic composition further comprises at least one antigen associated with tumor neovasculature.

6. Use of Claim 5, wherein the antigen associated with tumor neovasculature is selected from the group consisting of PSMA, VEGFR2, and Tie-2.

7. An immunogenic composition for use in the treatment of ovarian or colorectal cancer comprising, separately or in combination, the antigen PRAME and at least one other tumor-associated antigen, wherein each antigen is 1) a whole antigen, 2) a fragment of an antigen, 3) an epitope cluster derived from an antigen, 4) an epitope derived from an antigen, or 5) a nucleic acid encoding any of 1) to 4), wherein the at least one other tumor-associated antigen is selected from the group consisting of SSX-2, NY-ESO-1 and PSMA.

8. The composition of Claim 7, further comprising at least one antigen associated with tumor neovasculature.

9. The composition of Claim 8, wherein the antigen associated with tumor neovasculature is selected from the group consisting of PSMA, VEGFR2, and Tie-2.

## Patentansprüche

1. Verwendung einer immunogenen Zusammensetzung, welche gegen PRAME und mindestens ein anderes Tumor-assoziiertes Antigen immunisiert, für die Herstellung eines Medikaments zur Behandlung von Ovarial- oder Kolorektalkrebs, wobei das mindestens eine andere Tumor-assoziierte Antigen ausgewählt ist aus der Gruppe bestehend aus SSX-2, NY-ESO-1 und PSMA.

2. Verwendung nach Anspruch 1, wobei das mindestens eine andere Tumor-assoziierte Antigen NY-ESO-1 und SSX-2 umfasst.

3. Verwendung nach Anspruch 1, wobei das mindestens eine andere Tumor-assoziierte Antigen NY-ESO-1, SSX-2 und PSMA umfasst.

4. Verwendung nach Anspruch 1, wobei eine zytotoxische T-Zellantwort induziert wird.

5. Verwendung nach Anspruch 1, wobei die immunogene Zusammensetzung weiterhin mindestens ein Antigen umfasst, welches mit Tumor-Neovaskulatur assoziiert ist.

6. Verwendung nach Anspruch 5, wobei das Antigen, welches mit Tumor-Neovaskulatur assoziiert ist, ausgewählt ist aus der Gruppe bestehend aus PSMA, VEGFR2 und Tie-2.

7. Eine immunogene Zusammensetzung für die Verwendung zur Behandlung von Ovarial- oder Kolorektalkrebs umfassend, getrennt oder in Kombination, das Antigen PRAME und mindestens ein weiteres Tumor-assoziiertes Antigen, wobei jedes Antigen 1) ein ganzes Antigen, 2) ein Fragment von einem Antigen, 3) ein Epitop-Cluster abgeleitet von einem Antigen, 4) ein Epitop abgeleitet von einem Antigen oder 5) eine Nukleinsäure kodierend eines von 1) bis 4) ist, wobei das mindestens eine andere Tumor-assoziierte Antigen ausgewählt ist aus der Gruppe bestehend aus SSX-2, NY-ESO-1 und PSMA.

8. Die Zusammensetzung nach Anspruch 7, weiterhin umfassend mindestens ein Antigen, welches mit Tumor-Neovaskulatur assoziiert ist.

9. Die Zusammensetzung nach Anspruch 8, wobei das Antigen, welches mit Tumor-Neovaskulatur assoziiert ist, ausgewählt ist aus der Gruppe bestehend aus PSMA, VEGFR2 und Tie-2.

## Revendications

1. Utilisation d'une composition immunogénique qui immunise contre le PRAME et d'au moins un autre antigène tumoral en vue de la fabrication d'un médicament pour le traitement du cancer ovarien ou colorectal, où l'au moins un autre antigène tumoral est sélectionné dans le groupe constitué par SSX-2, NY-ESO-1 et PSMA.

2. Utilisation selon la revendication 1, où l'au moins un autre antigène tumoral comprend NY-ESO-1 et SSX-2.

3. Utilisation selon la revendication 1, où l'au moins un autre antigène tumoral comprend NY-ESO-1, SSX-2 et PSMA.

4. Utilisation selon la revendication 1, où une réaction de cellules T cytolytiques est induite.

5. Utilisation selon la revendication 1, où la composition immunogénique comprend en outre au moins un antigène associé à la néovasculature tumorale.

6. Utilisation selon la revendication 5, où l'antigène associé à la néovasculature tumorale est sélectionné dans le groupe constitué par PSMA, VEGFR2 et Tie-2.

7. Composition immunogénique destinée à être utilisée dans le traitement du cancer ovarien ou colorectal, comprenant, séparément ou en combinaison, l'antigène PRAME et au moins un autre antigène tumoral, où chaque antigène est 1) un antigène entier, 2) un fragment d'un antigène, 3) un agrégat d'épitopes dérivé d'un antigène, 4) un épitope dérivé d'un antigène, ou 5) un acide nucléique codant n'importe lequel des éléments 1) à 4), où l'au moins un autre antigène tumoral est sélectionné dans le groupe constitué par SSX-2, NY-ESO-1 et PSMA.

8. La composition selon la revendication 7, comprenant en outre au moins un antigène associé à la néovasculature tumorale.

9. La composition selon la revendication 8, où l'antigène associé à la néovasculature tumorale est sélectionné dans le groupe constitué par PSMA, VEGFR2 et Tie-2.
